# EUROPEAN PATENT APPLICATION

(11) **EP 4 800 090 A1**
(43) Date of publication of application: **02.09.2026**
(21) Application number: 24882423.7
(22) Date of filing: 23.10.2024
(51) Int. Cl.: C12M 1/00, C12M 1/36, C12N 1/00, C12N 5/00, C12N 7/00, G01N 35/08, G01N 37/00

(54) **DROPLET CONTROL DEVICE, DROPLET PRODUCTION METHOD, AND COMPOSITION**

(30) Priority: 23.10.2023 JP 2023181844
(71) Applicant: Japan Science and Technology Agency, Kawaguchi-shi, Saitama 332-0012 (JP)
(72) Inventor: MURAOKA, Takahiro, Fuchu-shi, Tokyo 183-8538 (JP); UCHIDA, Noriyuki, Fuchu-shi, Tokyo 183-8538 (JP); KONDO, Shiori, Fuchu-shi, Tokyo 183-8538 (JP)
(74) Representative: Vossius & Partner Patentanwälte Rechtsanwälte mbB
(86) International application number: PCT/JP2024/037807
(87) International publication number: WO 2025/089312

(57) **Abstract**

A droplet control device for controlling droplets produced from a polysaccharide and a polyether includes a light irradiation unit configured to irradiate with light and a monitoring unit configured to monitor a state of the droplets, in which an aqueous medium containing the droplets further contains a photoisomerizable compound, and the light irradiation unit is configured to irradiate the aqueous medium with light having a wavelength that causes the photoisomerizable compound to undergo photoisomerization.

## Description

### TECHNICAL FIELD

The present invention relates to a droplet control device, a droplet production method, and a composition. In addition, the present invention relates to a device for separating a target substance and a method for separating a target substance.

Priority is claimed on Japanese Patent Application No. 2023-181844, filed October 23, 2023, the content of which is incorporated herein by reference.

### BACKGROUND ART

As a technology for providing a closed space that maintains fluidity in water, liquid-liquid phase-separated droplets have been known. For example, by using droplets having a diameter of 10 nm, it is possible to construct an ultra-small reaction space of 0.5 yL (0.5 x 10⁻²⁴ L). By using a two-phase system of dextran and polyethylene glycol, it is possible to produce a large number of droplets at low cost and in an extremely simple manner. However, generally, droplets are unstable and droplets spontaneously fuse together within a few minutes, changing to macro-scale phase separation.

As a technology using the two-phase system of dextran and polyethylene glycol, a method of forming droplets on a femtoliter reactor array and performing a digital bioassay has been reported (Non-Patent Document 1).

### Citation List

### Patent Document

Non-Patent Document 1: Y. Minagawa et al., On-Chip Enrichment System for Digital Bioassay Based on Aqueous Two-Phase System. ACS Nano 2023, 17, 1, 212-220.

### SUMMARY OF INVENTION

### Technical Problem

In the liquid-liquid phase-separated droplet technology of the related art, the droplets are unstable, and thus it is difficult to control the droplets. Therefore, it is difficult to use the droplets as a site for a chemical reaction or a biological phenomenon. In addition, for droplets formed within a microspace such as the femtoliter reactor array, it is difficult to selectively recover only desired droplets after the assay. In addition, it may be difficult to selectively collect a target substance from a test composition containing a plurality of substances.

Therefore, an object of the present invention is to provide a droplet control device and a droplet production method, capable of controlling liquid-liquid phase-separated droplets. Another object of the present invention is to provide a composition capable of controlling initiation of a reaction of a plurality of components. Still another object of the present invention is to provide a device for separating a target substance from a sample and a method for separating a target substance from a sample.

### Solution to Problem

The present invention includes the following aspects.
[1] A droplet control device for controlling droplets produced from a polysaccharide and a polyether, the droplet control device including: a light irradiation unit configured to irradiate with light; and a monitoring unit configured to monitor a state of the droplets, in which an aqueous medium containing the droplets further contains a photoisomerizable compound, and the light irradiation unit is configured to irradiate the aqueous medium with light having a wavelength that causes the photoisomerizable compound to undergo photoisomerization.
[2] The droplet control device according to [1], further including a processor configured to control an operation of the droplet control device.
[3] The droplet control device according to [1] or [2], further including a container configured to store the aqueous medium.
[4] The droplet control device according to [2] or [3], in which the processor is configured to control an operation of the light irradiation unit, and the operation of the light irradiation unit controlled by the processor is at least one selected from the group consisting of a timing of starting light irradiation, a light irradiation time, a wavelength of irradiation light, and a light irradiation position.
[5] The droplet control device according to any one of [2] to [4], in which the processor is configured to control an operation of the monitoring unit, and the operation of the monitoring unit controlled by the processor is at least one selected from the group consisting of a timing of starting monitoring, a monitoring time, and a monitoring position.
[6] The droplet control device according to any one of [1] to [5], further including: an output unit configured to output a monitoring result obtained by the monitoring unit.
[7] The droplet control device according to any one of [1] to [6], in which the monitoring unit includes a microscope unit configured to magnify and observe the droplets.
[8] The droplet control device according to any one of [1] to [7], in which the droplets include a first droplet containing a first component and a second droplet containing a second component, and the first component is a component that interacts with the second component.
[9] The droplet control device according to [8], further including: a first component storage section configured to store the first component; a second component storage section configured to store the second component; a polysaccharide storage section configured to store a polysaccharide; a polyether storage section configured to store a polyether; and a photoisomerizable compound storage section configured to store the photoisomerizable compound.
[10] The droplet control device according to [9], further including a droplet production unit, in which the droplet production unit is controlled to mix aqueous solutions containing the first component supplied from the first component storage section, the polysaccharide supplied from the polysaccharide storage section, the polyether supplied from the polyether storage section, and the photoisomerizable compound supplied from the photoisomerizable compound storage section to produce the first droplet, and mix aqueous solutions containing the second component supplied from the second component storage section, the polysaccharide supplied from the polysaccharide storage section, the polyether supplied from the polyether storage section, and the photoisomerizable compound supplied from the photoisomerizable compound storage section to produce the second droplet.
[11] The droplet control device according to [10], further including a detection reagent storage section configured to supply a detection reagent, in which the detection reagent storage section is controlled to supply the detection reagent to the droplet production unit at at least one of a timing of producing the first droplet and a timing of producing the second droplet.
[12] The droplet control device according to [10] or [11], in which the droplet production unit includes a stirring mechanism.
[13] The droplet control device according to any one of [8] to [12], in which the monitoring unit is controlled to monitor an interaction between the first component and the second component in a third droplet generated by fusing the first droplet and the second droplet.
[14] The droplet control device according to any one of [8] to [13], in which the monitoring unit is controlled to perform the monitoring with reference to a time point at which the light irradiation unit starts irradiation of the aqueous medium with light having a wavelength that causes the photoisomerizable compound to undergo photoisomerization.
[15] The droplet control device according to any one of [8] to [14], in which the first component is a cell, the second component is a virus, and the interaction is an infection of the cell by the virus.
[16] The droplet control device according to [3], in which the container includes a plurality of wells and a flow path communicating with the plurality of wells.
[17] The droplet control device according to [16], in which the volume of the well is 1 picoliter or less.
[18] The droplet control device according to [16] or [17], further including a liquid feeding unit configured to feed a liquid to the plurality of wells through the flow path.
[19] The droplet control device according to [18], in which the liquid feeding unit includes a polysaccharide storage section configured to supply a polysaccharide, a polyether storage section configured to supply a polyether, and a photoisomerizable compound storage section configured to supply the photoisomerizable compound.
[20] The droplet control device according to [19], in which the liquid feeding unit is controlled to perform, in the following order, (i) feeding an aqueous solution containing the polysaccharide to the plurality of wells, (ii) feeding an aqueous solution containing the polyether to the plurality of wells, and (iii) feeding an aqueous solution containing the polyether and the photoisomerizable compound to the plurality of wells.
[21] The droplet control device according to [20], in which the monitoring unit is controlled to start the monitoring after the (iii).
[22] The droplet control device according to [21], in which the light irradiation unit is controlled to irradiate wells other than a specific well with light having a wavelength that causes the photoisomerizable compound to undergo photoisomerization, based on a monitoring result of the monitoring unit.
[23] The droplet control device according to [22], further including a droplet collection unit configured to collect the droplets from the specific well.
[24] The droplet control device according to any one of [1] to [23], in which the photoisomerizable compound is a compound including a peptide structure represented by General Formula (p1) or (p2),

   XaXb(XaXc)ₙXaXdXa (p1)

   XaXd(XaXc)ₐXaXbXa (p2)

   [in the formulae, Xa represents an amino acid residue having a hydrophilic side chain, Xb represents an amino acid residue having a side chain represented by General Formula (b1), Xc and Xd each independently represent an amino acid residue having a hydrophobic side chain, or a glycine residue, and n represents an integer of 1 to 3, provided that a plurality of Xa's may be the same or different from each other, and when n is 2 or 3, a plurality of Xc's may be the same or different from each other]
   [Chemical Formula 1]

   *-Ar²-N=N-Ar¹ (b1)

   [in the formula, Ar¹ represents an aryl group which may have a substituent or a heteroaryl group which may have a substituent, Ar² represents an arylene group which may have a substituent or a heteroarylene group which may have a substituent, and * represents a bonding site].
[25] A device for separating a target substance from a sample prepared in an aqueous medium containing a photoisomerizable compound, the device including: a container having a plurality of compartments for storing the sample; a light irradiation unit configured to irradiate with light to the sample stored in the compartments; and a monitoring unit configured to monitor the sample stored in the compartments, in which the light irradiation unit is configured to irradiate the sample with light having a wavelength that causes the photoisomerizable compound to undergo photoisomerization, and the sample containing the photoisomerizable compound undergoes a sol-gel transition due to the photoisomerization of the photoisomerizable compound.
[26] A method for producing droplets using a droplet container including a plurality of wells and a flow path communicating with the plurality of wells, the method including the following (a), (b), and (c1) in this order, or the following (b), (a), and (c2) in this order:
   (a) a step of feeding an aqueous solution containing a polysaccharide to the plurality of wells through the flow path;
   (b) a step of feeding an aqueous solution containing a polyether to the plurality of wells through the flow path;
   (c1) a step of feeding an aqueous solution containing the polyether and a photoisomerizable compound to the plurality of wells through the flow path; and
   (c2) a step of feeding an aqueous solution containing the polysaccharide and a photoisomerizable compound to the plurality of wells through the flow path.
[27] The method according to [26], further including, after the step (c1) or (c2):
   (d) a step of irradiating wells other than a specific well with light having a wavelength that causes the photoisomerizable compound to undergo photoisomerization.
[28] The method according to [27], further including, after the step (d):
   (e1) a step of feeding an aqueous solution containing a polyether to the plurality of wells through the flow path, and collecting droplets from the specific well; or
   (e2) a step of feeding an aqueous solution containing a polysaccharide to the plurality of wells through the flow path, and collecting the droplet from the specific well.
[29] A method for separating a target substance from a sample prepared in an aqueous medium containing a photoisomerizable compound, the method including:
   (A) a step of distributing the sample prepared in the aqueous medium in a sol state, containing the photoisomerizable compound, into a plurality of compartments;
   (B) a step of irradiating either (i) a compartment containing the target substance or (ii) a compartment not containing the target substance with light having a wavelength that causes the photoisomerizable compound to undergo photoisomerization, to gel the sample stored in the compartments irradiated with the light; and
   (C) a step of taking out the sample from the compartments not irradiated with the light.
[30] The method according to any one of [26] to [29], in which the photoisomerizable compound is a compound including a peptide structure represented by General Formula (p1) or (p2),

   XaXb(XaXc)ₙXaXdXa (p1)

   XaXd(XaXc)ₙXaXbXa (p2)

   [in the formulae, Xa represents an amino acid residue having a hydrophilic side chain, Xb represents an amino acid residue having a side chain represented by General Formula (b1), Xc and Xd each independently represent an amino acid residue having a hydrophobic side chain, or a glycine residue, and n represents an integer of 1 to 3, provided that a plurality of Xa's may be the same or different from each other, and when n is 2 or 3, a plurality of Xc's may be the same or different from each other]
   [Chemical Formula 2]

   * -Ar²-N=N-Ar¹ (b1)

   [in the formula, Ar¹ represents an aryl group which may have a substituent or a heteroaryl group which may have a substituent, Ar² represents an arylene group which may have a substituent or a heteroarylene group which may have a substituent, and * represents a bonding site].
[29] A composition stored in a light-shielding container, the composition containing: a polysaccharide; a polyether; a photoisomerizable compound; a first droplet in which a first component is encapsulated; and a second droplet in which a second component is encapsulated.
[30] The composition according to [29], in which the photoisomerizable compound is a compound including a peptide structure represented by General Formula (p1) or (p2),

   XaXb(XaXc)ₙXaXdXa (p1)

   XaXd(XaXc)ₐXaXbXa (p2)

   [in the formulae, Xa represents an amino acid residue having a hydrophilic side chain, Xb represents an amino acid residue having a side chain represented by General Formula (b1), Xc and Xd each independently represent an amino acid residue having a hydrophobic side chain, or a glycine residue, and n represents an integer of 1 to 3, provided that a plurality of Xa's may be the same or different from each other, and when n is 2 or 3, a plurality of Xc's may be the same or different from each other]
   [Chemical Formula 3]

   * -Ar²-N=N-Ar¹ (b1)

   [in the formula, Ar¹ represents an aryl group which may have a substituent or a heteroaryl group which may have a substituent, Ar² represents an arylene group which may have a substituent or a heteroarylene group which may have a substituent, and * represents a bonding site].
[31] A multi-component formulation including: an openable and closable light-shielding container; and the composition according to [29 or 30], which is stored in the light-shielding container.

### Advantageous Effects of Invention

According to the present invention, there are provided a droplet control device and a droplet production method, capable of controlling liquid-liquid phase-separated droplets. In addition, there is provided a composition capable of controlling initiation of a reaction of a plurality of components. In addition, according to the present invention, there is provided a device for separating a target substance from a sample and a method for separating a target substance from a sample.

### BRIEF DESCRIPTION OF DRAWINGS

[FIG. 1] A schematic view showing an example configuration of a droplet control device according to a first embodiment.
[FIG. 2] A schematic view showing an example configuration of a microscope unit as an example of a monitoring unit.
[FIG. 3] A flowchart showing an operation example of the droplet control device according to the first embodiment.
[FIG. 4A] A schematic view showing a state of a photoisomerizable compound and droplets before light irradiation in the droplet control device according to the first embodiment.
[FIG. 4B] A schematic view showing a state of the photoisomerizable compound and the droplets during the light irradiation in the droplet control device according to the first embodiment.
[FIG. 4C] A schematic view showing a state of the photoisomerizable compound and the droplets after the light irradiation in the droplet control device according to the first embodiment.
[FIG. 5] A schematic view showing an example configuration of a droplet control device according to a second embodiment.
[FIG. 6A] A schematic view showing an example configuration of a container in the droplet control device according to the second embodiment.
[FIG. 6B] A schematic view showing a modification example of the container in the droplet control device according to the second embodiment.
[FIG. 7] A flowchart showing an operation example of the droplet control device according to the second embodiment.
[FIG. 8A] A schematic view for explaining a procedure for producing droplets in the droplet control device according to the second embodiment, showing a state in which a polysaccharide aqueous solution is fed.
[FIG. 8B] A schematic view for explaining the procedure for producing droplets in the droplet control device according to the second embodiment, showing a state in which a polyether aqueous solution is fed.
[FIG. 8C] A schematic view for explaining the procedure for producing droplets in the droplet control device according to the second embodiment, showing a state in which a polyether aqueous solution containing a photoisomerizable compound is fed.
[FIG. 9A] A schematic view for explaining a procedure for collecting droplets in the droplet control device according to the second embodiment, showing a state in which wells other than a specific well are irradiated with light.
[FIG. 9B] A schematic view for explaining the procedure for collecting droplets in the droplet control device according to the second embodiment, showing a state after wells other than the specific well are irradiated with light.
[FIG. 9C] A schematic view for explaining the procedure for collecting droplets in the droplet control device according to the second embodiment, showing a state in which a polyether aqueous solution is fed.
[FIG. 10] A flowchart showing another operation example of the droplet control device according to the second embodiment.
[FIG. 11] A flowchart showing an operation example when collecting target cells using the droplet control device according to the second embodiment.
[FIG. 12A] A schematic view for explaining a procedure for producing droplets containing cells in the droplet control device according to the second embodiment, showing a state in which a polysaccharide aqueous solution is fed.
[FIG. 12B] A schematic view for explaining the procedure for producing droplets containing cells in the droplet control device according to the second embodiment, showing a state in which a polyether aqueous solution containing the cells is fed.
[FIG. 12C] A schematic view for explaining the procedure for producing droplets containing cells in the droplet control device according to the second embodiment, showing a state in which a polyether aqueous solution containing a photoisomerizable compound is fed.
[FIG. 13A] A schematic view for explaining a procedure for collecting droplets containing target cells in the droplet control device according to the second embodiment, showing a state in which wells other than a well containing the target cells are irradiated with light.
[FIG. 13B] A schematic view for explaining the procedure for collecting droplets containing the target cells in the droplet control device according to the second embodiment, showing a state after wells other than the well containing the target cells are irradiated with light.
[FIG. 13C] A schematic view for explaining the procedure for collecting droplets containing the target cells in the droplet control device according to the second embodiment, showing a state in which a polyether aqueous solution is fed.
[FIG. 14] A flowchart showing an operation example when collecting a second component that reacts with a first component using the droplet control device according to the second embodiment.
[FIG. 15A] A schematic view for explaining a procedure for producing droplets containing the first component and the second component in the droplet control device according to the second embodiment, showing a state in which a polysaccharide aqueous solution containing the first component is fed.
[FIG. 15B] A schematic view for explaining the procedure for producing droplets containing the first component and the second component in the droplet control device according to the second embodiment, showing a state in which a polyether aqueous solution containing the second component is fed.
[FIG. 15C] A schematic view for explaining the procedure for producing droplets containing the first component and the second component in the droplet control device according to the second embodiment, showing a state in which a polyether aqueous solution containing a photoisomerizable compound is fed.
[FIG. 16A] A schematic view for explaining a procedure for collecting droplets containing the first component and the second component in the droplet control device according to the second embodiment, showing a state in which wells other than a well containing the first component and the second component are irradiated with light.
[FIG. 16B] A schematic view for explaining the procedure for collecting droplets containing the first component and the second component in the droplet control device according to the second embodiment, showing a state after wells other than the well containing the first component and the second component are irradiated with light.
[FIG. 16C] A schematic view for explaining the procedure for collecting droplets containing the first component and the second component in the droplet control device according to the second embodiment, showing a state in which a polyether aqueous solution is fed.
[FIG. 17] A schematic view showing the coexisting state of a droplet A and a droplet B in Example 1; the droplet A contains Escherichia coli and fluorescein-di-β-D-galactopyranoside (FDG); and the droplet B contains an M13 phage.
[FIG. 18] An image showing results of detecting green fluorescence from fluorescein when light irradiation was performed and when light irradiation was not performed, in a solution containing the droplet A and the droplet B in Example 1.
[FIG. 19] A schematic view showing a procedure of Example 2.
[FIG. 20] Images showing states of wells before feeding of a PEG aqueous solution (before (4) in FIG. 19) and before feeding of a PEG aqueous solution (after (4) in FIG. 19) in Example 2.
[FIG. 21] A schematic view showing a procedure of Example 3.
[FIG. 22] Images showing states of wells when a PEG aqueous solution is fed without light irradiation (after (4) in FIG. 21) and when a PEG aqueous solution is fed with light irradiation (after (6) in FIG. 21) in Example 3.
[FIG. 23] A schematic view showing an example configuration of a separation device for a target substance according to an embodiment.
[FIG. 24] A flowchart showing an operation example when collecting the target substance using the separation device for the target substance according to the embodiment.

### DESCRIPTION OF EMBODIMENTS

Hereinafter, embodiments of the present invention will be described in detail with reference to the drawings as appropriate. In the drawings, identical or corresponding parts are denoted by identical or corresponding reference numerals, and overlapping descriptions are omitted. The dimensional ratios in the respective drawings may be exaggerated for the sake of explanation, and do not necessarily match the actual dimensional ratios.

A numerical range represented by "to" refers to a range including numerical values denoted before and after "to" as the lower limit value and the upper limit value.

Unless otherwise indicated, the articles "a," "an," and "the" encompass both the singular and plural forms and are understood to mean "one or more."

The term "comprise" means that constituent elements other than the target constituent elements may be included. The term "consist of" means that constituent elements other than the target constituent elements are not included. The term "consist essentially of" means that constituent elements other than the target constituent elements are not included in a manner whereby a special function is exhibited (such as a manner in which the effect of the invention is completely lost). In the present specification, the term "comprise" encompasses the meanings of "consist of" and "consist essentially of".

### [Droplet control device]

A first aspect of the present disclosure is a droplet control device for controlling droplets produced from a polysaccharide and a polyether. In one embodiment, the droplet control device includes a light irradiation unit configured to irradiate with light, and a monitoring unit configured to monitor a state of the droplets. An aqueous medium containing the droplets further contains a photoisomerizable compound. The light irradiation unit is configured to irradiate the aqueous medium with light having a wavelength that causes the photoisomerizable compound to undergo photoisomerization.

### <Droplets>

The droplets controlled by the droplet control device according to the embodiment are droplets produced from a polysaccharide and a polyether (hereinafter, referred to as "droplets D"). When an aqueous solution containing a polysaccharide and a polyether is mixed and stirred, micro-sized droplets containing the polysaccharide at a high concentration are generally formed by liquid-liquid phase separation.
Alternatively, as described later, the droplets containing the polysaccharide at a high concentration are formed in wells by feeding a polysaccharide aqueous solution and subsequently feeding a polyether aqueous solution to a flow path device in which the wells are formed in a flow path. Alternatively, the droplets containing the polyether at a high concentration are formed in the wells by feeding a polyether aqueous solution and subsequently feeding a polysaccharide aqueous solution to a flow path device in which the wells are formed in a flow path.

By causing the droplets D to coexist with the photoisomerizable compound, the state of the droplets D can be controlled by changing a photoisomerization state of the photoisomerizable compound.

### (Polysaccharide)

The polysaccharide is not particularly limited. Examples of the polysaccharide include dextran, dextrin, amylose, amylopectin, glycogen, cellulose, agarose, carrageenan, heparin, alginic acid, hyaluronic acid, pectin, glucomannan, tamarind seed gum, guar gum, locust bean gum, gum arabic, karaya gum, xanthan gum, and gellan gum, without being limited thereto. The polysaccharide is preferably one having glucose as a constituent sugar, preferably dextran or dextrin, and more preferably dextran.

The molecular weight of the polysaccharide is not particularly limited, and examples thereof include 10,000 to 10,000,000, 50,000 to 5,000,000, 100,000 to 5,000,000, 300,000 to 1,000,000, and 400,000 to 800,000.

The polysaccharide may be labeled with a fluorescent dye or the like. The fluorescent dye is not particularly limited and it is possible to use any known fluorescent dye. To avoid droplet fusion, it is preferable to use a fluorescent dye having an excitation light wavelength and a fluorescent wavelength different from the wavelength at which the compound (P) undergoes cis-conversion. For example, when the compound (P) having a side chain represented by Formula (b1-1-1) described later is used, it is possible to use a fluorescent dye having an excitation light wavelength and a fluorescent wavelength of 490 nm or more. Examples of such fluorescent dyes include rhodamine, 5-FAM, 5-FITC, ATTO520, AlexaFlour555, Cy3Cy3, Cy5, Alexa568, Alexa647, and the like, without being limited thereto.

### (Polyether)

Polyethers are not particularly limited. Polyethers are compounds having a plurality of ether bonds in the molecule. Polyethers may be aliphatic polyethers or aromatic polyethers, and aliphatic polyethers are preferable. An example of an aliphatic polyether is polyalkylene glycol. Examples of polyalkylene glycols include polyethylene glycol, polypropylene glycol, polytetramethylene glycol, polyoxyethylene polyoxypropylene glycol, and the like. The polyether is preferably polyethylene glycol.

The molecular weight of the polyether is not particularly limited, and examples thereof include 300 to 10,000,000, 500 to 5,000,000, 1,000 to 3,000,000, 5,000 to 1,000,000, and 10,000 to 500,000.

### (Photoisomerizable compound)

The "photoisomerizable compound" refers to a compound that undergoes isomerization upon light irradiation. The photoisomerizable compound reversibly changes from a trans form to a cis form, or from a cis form to a trans form, upon irradiation with light of a specific wavelength.

The photoisomerizable compound is preferably a compound in which the aqueous medium containing the photoisomerizable compound undergoes a sol-gel transition due to the photoisomerization. In one embodiment, the photoisomerizable compound forms a gel in the trans form and becomes a sol in the cis form. In one embodiment, the photoisomerizable compound forms a sol in the trans form and becomes a gel in the cis form.

The photoisomerizable compound is preferably a compound having an affinity for any of the polyether and the polysaccharide. When the photoisomerizable compound has a higher affinity for the polyether than for the polysaccharide, the photoisomerizable compound is localized in a polyether phase in a dispersion liquid of the droplets D. When the photoisomerizable compound has a higher affinity for the polysaccharide than for the polyether, the photoisomerizable compound is localized in a polysaccharide phase in a dispersion liquid of the droplets D. It is preferable to use a photoisomerizable compound having a high affinity for a compound localized outside the droplets.

Examples of the photoisomerizable compound having a high affinity for the polyether include a compound (P) described later.

### (Aqueous medium)

The "aqueous medium" is an aqueous liquid used for dissolving the polysaccharide, the polyether, and the photoisomerizable compound during the production of the droplets D. Examples of the aqueous medium include water, various buffers (phosphate-buffered saline, acetate buffer, phosphate buffer, citrate buffer, citratephosphate buffer, borate buffer, tartrate buffer, Tris buffer, HEPES buffer, and the like), and various liquid media (Dulbecco's Modified Eagle's (D-MEM) medium, MEMα medium, and the like).

The aqueous medium preferably has a pH of 4 to 10, more preferably a pH of 5 to 9, still more preferably a pH of 6 to 9, and particularly preferably a pH of 7 to 9.

The droplets D are formed by mixing the polysaccharide and the polyether in the aqueous medium. The formed droplets D are maintained in the aqueous medium.

### (First component and second component)

When producing the droplets D by mixing the polysaccharide aqueous solution and the polyether aqueous solution, droplets D in which a desired component is encapsulated can be formed by containing the desired component in these aqueous solutions.

Components to be encapsulated in the droplets D are not particularly limited, and examples thereof include organic synthetic compounds such as low-molecular-weight compounds, fluorescent dyes, radical generators, and synthetic polymer compounds; biomolecules such as amino acids, nucleotides, sugars, vitamins, peptides, proteins, and nucleic acids; inorganic compounds such as inorganic nanoparticles, inorganic anions, inorganic cations, inorganic salts, and aggregates thereof; cells such as animal cells, plant cells, microorganisms and fungi, algal cells, and protozoan cells; microstructures such as phages, viruses, and molecular assemblies; and cell aggregates such as organoids and spheroids.

For example, a third droplet D3 containing the first component and the second component can be formed by separately producing a first droplet D1 containing the first component and a second droplet D2 containing the second component, and mixing and fusing them. When the second component is a component that interacts with the first component, the interaction between the first component and the second component can be monitored over time with the time at which the third droplet D3 is formed, serving as a start point of the interaction. Examples of combinations of the first component and the second component include cells and viruses that infect the cells, compounds that react with each other, antibodies and antigens, and active agents and inhibitors.

In the following description, the droplet containing the first component is referred to as the first droplet D1, the droplet containing the second component is referred to as the second droplet D2, and the droplet formed by fusion of the first droplet D1 and the second droplet D2 is referred to as the third droplet D3. In addition, these may be collectively referred to as the droplets D.

### <First embodiment>

### (Configuration example of droplet control device)

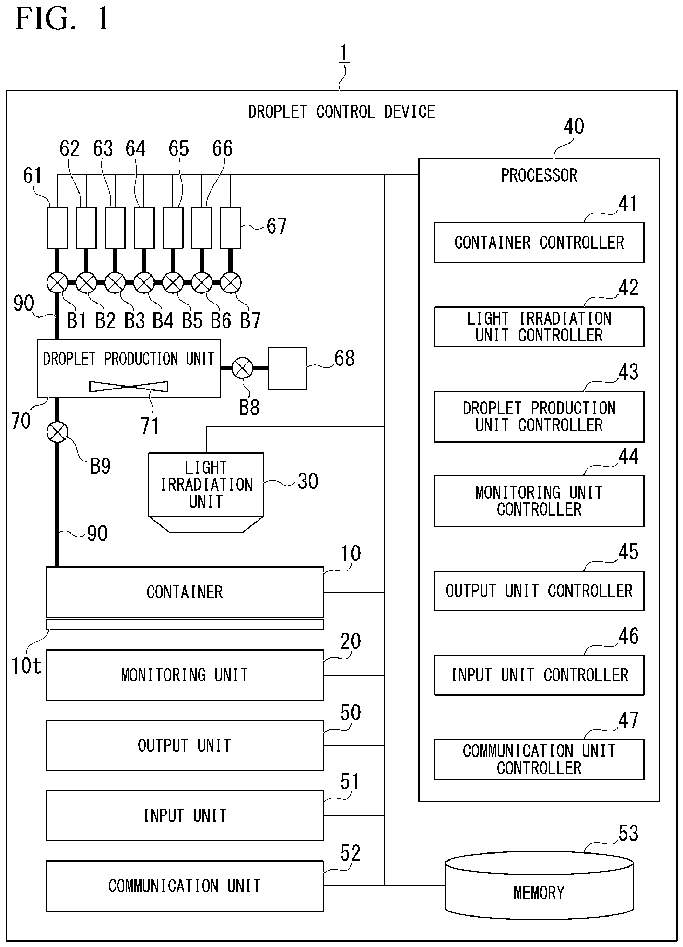

FIG. 1 is a schematic view showing an example configuration of the droplet control device according to the first embodiment. A droplet control device 1 includes a container 10, a monitoring unit 20, a light irradiation unit 30, an output unit 50, an input unit 51, a communication unit 52, a memory 53, a waste liquid storage section 68, a droplet production unit 70, and a processor 40 configured to control operations of these components. In addition, the droplet control device 1 includes, as storage sections for various components, a polysaccharide storage section 61, a polyether storage section 62, a photoisomerizable compound storage section 63, a first component storage section 64, a second component storage section 65, a detection reagent storage section 66, and an aqueous medium storage section 67.

In the droplet control device 1, components for producing the first droplet D1 are supplied to the droplet production unit 70 from the polysaccharide storage section 61, the polyether storage section 62, the photoisomerizable compound storage section 63, and the first component storage section 64. The droplet production unit 70 mixes and stirs these components to produce the first droplet D1. The produced first droplet D1 is transferred to the container 10 together with the aqueous medium.

In addition, components for producing the second droplet D2 are supplied to the droplet production unit 70 from the polysaccharide storage section 61, the polyether storage section 62, the photoisomerizable compound storage section 63, and the second component storage section 65. The droplet production unit 70 mixes and stirs these components to produce the second droplet D2. The produced second droplet D2 is transferred to the container 10 together with the aqueous medium.

The light irradiation unit 30 irradiates the aqueous medium containing the first droplet D1 and the second droplet D2 stored in the container 10 with light. As a result, the photoisomerizable compound in the aqueous medium undergoes photoisomerization, the first droplet D1 and the second droplet D2 fuse, and the third droplet D3 is formed. In the third droplet D3, the first component and the second component coexist, and the interaction between these components starts along with the formation of the third droplet D3. The monitoring unit 20 monitors states of the first droplet D1, the second droplet D2, and the third droplet D3 in the container 10. The interaction between the first component and the second component in the third droplet D3 is observed over time by the monitoring unit 20.

### <<Container>>

The container 10 is configured to store the aqueous medium containing the droplets D. The container 10 is not particularly limited, but preferably has an internal space surrounded by a bottom surface, side wall surfaces, and a top surface. The container 10 may be configured with an openable and closable lid for the top surface, or may be a closed type in which a top surface plate is fixed to the side walls. Alternatively, the container 10 may be an open type without the top surface.

A material for the container 10 is not particularly limited, but when it has the top surface, the top surface is composed of a material capable of transmitting the light irradiated by the light irradiation unit 30. In addition, when the monitoring unit 20 includes a microscope unit described later, the entire container 10 may be composed of a transparent material with low autofluorescence.

Examples of the transparent material with low autofluorescence include glass, polyethylene terephthalate (PET), polycarbonate, cycloolefin polymer, polydimethylsiloxane, polystyrene, and polyacrylate (acrylic resin).

The size of the container 10 is not particularly limited, but the depth of a droplet storage space is preferably 1 mm or less. When the depth of the droplet storage space is 1 cm or less, overlapping of the droplets D in a vertical direction is suppressed, and observation of the droplets D by the monitoring unit 20 is facilitated. Examples of the depth of the droplet storage space include 10 nm or more and 0.5 mm or less, 0.1 mm or less, 50 µm or less, and 10 µm or less. The lower limit value of the depth of the droplet storage space is not particularly limited, and examples thereof include 10 nm or more, 50 nm or more, 100 nm or more, 500 nm or more, and 1 µm or more. Examples of the range of the depth of the droplet storage space include 10 nm or more and 1 mm or less, 10 nm or more and 0.5 mm or less, 10 nm or more and 0.1 mm or less, 10 nm or more and 50 µm or less, and 10 nm or more and 10 µm or less.

The "droplet storage space" is an internal space of the container 10 defined by the bottom surface, the side wall surfaces, and the top surface. When the container 10 does not have the top surface, the "droplet storage space" is a space defined by the bottom surface and the side wall surfaces.

The container 10 includes a temperature controller 10t configured to control a temperature of the droplet storage space. The temperature controller 10t controls the temperature of the droplet storage space to be a temperature suitable for the interaction between the first component and the second component. A heater, a cooler, a thermostat, or the like can be used as the temperature controller 10t.

The container 10 may be removably attachable. For example, the droplet control device 1 may include a container holding part, and a container prepared by a user may be installed as the container 10 in the container holding part.

### <<Monitoring unit>>

The monitoring unit 20 is configured to monitor the state of the droplets D stored in the container 10. The monitoring unit 20 is not particularly limited as long as it can observe the state of the droplets D.

Examples of the monitoring unit 20 include a unit including a microscope unit configured to magnify and observe the droplets D.

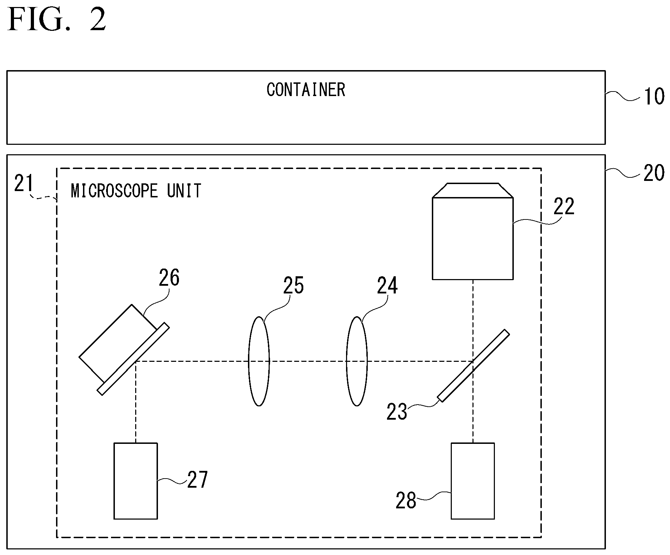

FIG. 2 shows an example of the microscope unit. The microscope unit 21 is disposed to face, for example, a lower surface of the container 10. The microscope unit 21 may be any microscope such as a bright-field microscope, a phase-contrast microscope, a differential interference contrast microscope, and a fluorescence microscope. The microscope unit 21 may be a digital microscope.

The microscope unit 21 includes an objective lens 22. The objective lens 22 is disposed to face the lower surface of the container 10. The objective lens 22 forms a magnified image of the droplets D in the container 10 using light that enters the objective lens 22 from the container 10. Any known objective lens can be used as the objective lens 22.

An image sensor 28 acquires the magnified image inside the container 10 formed by the microscope unit 21 as image data, and outputs the acquired image data. The image sensor 28 may be provided inside the microscope unit 21, or may be disposed outside the microscope unit 21 so as to receive the output of the magnified image from the microscope unit 21. For example, the microscope unit 21 may be a digital microscope incorporating the image sensor 28. Any image sensor such as a CMOS camera or a CCD camera can be used as the image sensor 28.

A light source 27 is disposed, for example, to emit light parallel to an optical axis of the objective lens 22 toward a control mirror 26. Any light source such as a laser device can be used as the light source 27. The light emitted from the light source 27 is used for observing the droplets D in the container 10 through the objective lens 22. A wavelength of the light emitted by the light source 27 can be appropriately selected depending on the state of the droplets D to be observed. For example, when a fluorescence intensity changes as the interaction within the third droplet D3 progresses, the light source 27 may emit excitation light to detect the fluorescence.

The wavelength of the light emitted by the light source 27 may be the same as or different from the wavelength that causes the photoisomerizable compound to undergo photoisomerization. From the viewpoint of strictly controlling the photoisomerization of the photoisomerizable compound, the wavelength of the light emitted by the light source 27 is preferably different from the wavelength that causes the photoisomerizable compound to undergo photoisomerization.

The microscope unit 21 may include a control mirror 26, a pupil projection lens 25, an imaging lens 24, a dichroic mirror 23, and the like, in order to cause the light from the light source 27 to enter the objective lens 22.

The control mirror 26 has one or more mirrors and a driving portion configured to drive the mirrors. The control mirror 26 can adjust an optical path of reflected light by controlling the position and tilt of the mirrors at high speed. Examples of the control mirror 26 include, but are not limited to, electromagnetic drive mirrors such as galvano mirrors, digital mirror devices (DMD), and MEMS mirrors.

The pupil projection lens 25 is disposed on the optical path of the reflected light of the control mirror 26. The pupil projection lens 25 converts a light pattern diverging from the control mirror 26 into parallel rays. Any lens such as a general convex lens can be used as the pupil projection lens 25 as long as it satisfies the above-described function.

The imaging lens 24 is disposed downstream of the pupil projection lens 25 on the optical path of the reflected light of the control mirror 26. The imaging lens 24 focuses the light pattern converted into parallel rays by the pupil projection lens 25 on a rear focal position of the objective lens 22. Any lens such as a general convex lens can be used as the imaging lens 24 as long as it satisfies the above-described function.

The dichroic mirror 23 is provided below the objective lens 22, and is disposed downstream of the imaging lens 24 on the optical path of the reflected light of the control mirror 26. The dichroic mirror 23 reflects only light of a specific wavelength (for example, a wavelength in the near-infrared region), and transmits light of other wavelengths. The dichroic mirror 23 reflects the light of the wavelength output by the light source 27. The dichroic mirror 23 reflects the light pattern formed by the light source 27 and the control mirror 26 toward the objective lens 22.

The monitoring unit 20 is not limited to one including the microscope unit 21, and may be configured to observe the droplets in the container 10 by other methods. The monitoring unit 20 may include, for example, an analyzer unit. An analyzer similar to an analyzer of a flow cytometer can be used as the analyzer unit. The analyzer unit may include a channel through which droplets pass one by one, a light source configured to irradiate with light to the droplets passing through the channel, and a photodetector configured to detect scattered light from the droplets.

### <<Light irradiation unit>>

The light irradiation unit 30 is configured to irradiate with light to the aqueous medium containing the droplets D in the container 10. The light irradiation unit 30 includes a mechanism configured to irradiate the aqueous medium with light having a wavelength that causes the photoisomerizable compound to undergo photoisomerization. The wavelength that causes the photoisomerizable compound to undergo photoisomerization differs depending on a structure of the photoisomerizable compound. Therefore, the wavelength of the light irradiated by the light irradiation unit 30 is controlled depending on the type of the photoisomerizable compound.

When the photoisomerizable compound is in a gel state in the trans form and in a sol state in the cis form, the light irradiated by the light irradiation unit 30 preferably has a wavelength that causes the photoisomerizable compound to undergo photoisomerization from the trans form to the cis form. For example, when the compound (P) described later has, as a side chain (b1), a side chain represented by Formula (b1-1-1) described later, examples of the wavelength of the irradiation light that causes cis-conversion of the compound (P) include 200 to 400 nm, 250 to 390 nm, 300 to 380 nm, 320 to 370 nm, or 330 to 360 nm; and for example, irradiation light of 350 nm can be used. When the compound (P) has, as the side chain (b1), a side chain represented by Formula (b1-1-7) described later, examples of the wavelength of the irradiation light that causes cis-conversion of the compound (P) include 400 to 700 nm, 450 to 650 nm, 500 to 600 nm, 510 to 580 nm, 520 to 570 nm, or 530 to 560 nm; and for example, irradiation light of 540 nm can be used.

As the light irradiation unit 30, a unit including a light source that generates light can be used. Examples of the light source include lasers, continuous-spectrum lamps (for example, mercury vapor lamps, halogen lamps, tungsten lamps), and light-emitting diodes (LEDs). Examples of the lasers include solid-state lasers such as YAG lasers, ruby lasers, glass lasers, YVO4 lasers, LD lasers, and fiber lasers; and semiconductor lasers.

The light irradiation unit 30 may include a plurality of light sources generating light of different wavelengths, and may be configured to switch the light sources according to the type of the photoisomerizable compound. Alternatively, the light irradiation unit 30 may include a tunable laser.

The light irradiation unit may include an optical member configured to adjust the wavelength and direction of the light generated from the light source. Examples of the optical member include a control mirror, a dichroic mirror, a bandpass filter, a mirror, a collimating lens, and a spectroscope.

### <<Droplet production unit>>

The droplet production unit 70 is configured to produce the droplets D. The droplet production unit 70 mixes and stirs the polysaccharide aqueous solution, the polyether aqueous solution, the photoisomerizable compound aqueous solution, and the first component, supplied from the polysaccharide storage section 61, the polyether storage section 62, the photoisomerizable compound storage section 63, and the first component storage section 64, to produce the first droplet D1.

In addition, the droplet production unit 70 mixes and stirs the polysaccharide aqueous solution, the polyether aqueous solution, the photoisomerizable compound aqueous solution, and the second component, supplied from the polysaccharide storage section 61, the polyether storage section 62, the photoisomerizable compound storage section 63, and the second component storage section 65, to produce the second droplet D2.

The droplet production unit 70 includes a mixing tank configured to mix and stir the respective components, and a stirring mechanism 71 configured to stir the liquid in the mixing tank. The stirring mechanism 71 is not particularly limited, and examples thereof include a mechanism for rotating a stirrer having a rod shape, a plate shape, a propeller shape, or the like, and a mechanism for rotating stirring blades fixed inside the mixing tank, a mechanism for rotating the mixing tank itself.

The droplet production unit 70 may include a temperature controller configured to control a temperature in the mixing tank. Examples of the temperature controller include a heater, a cooler, and a thermostat.

The waste liquid storage section 68 is connected to the droplet production unit 70. The waste liquid storage section 68 stores, for example, waste liquid used to clean the inside of the mixing tank after the droplets D produced in the mixing tank are transferred to the container 10. The cleaning of the inside of the mixing tank is performed using, for example, the aqueous medium supplied from the aqueous medium storage section 67. Connection and disconnection between the droplet production unit 70 and the waste liquid storage section 68 are switched by a valve B8.

### <<Component storage sections>>

The polysaccharide storage section 61 is configured to store the polysaccharide aqueous solution. The concentration of the polysaccharide aqueous solution stored in the polysaccharide storage section 61 is preferably 0.3% by mass or more. In a mixed solution containing various components in the production of the droplets D, the concentration of the polysaccharide is, for example, 0.1% to 40% by mass with respect to the total mass of the mixed solution. The concentration of the polysaccharide aqueous solution stored in the polysaccharide storage section 61 may be any concentration as long as the concentration of the polysaccharide can be adjusted to fall within the above-described concentration range when the mixed solution is prepared. The polysaccharide aqueous solution stored in the polysaccharide storage section 61 may be a concentrated solution, and in this case, the aqueous medium may be supplied from the aqueous medium storage section 67 for dilution.

The polysaccharide storage section 61 is connected to the droplet production unit 70 by a conduit 90. Connection and disconnection between the polysaccharide storage section 61 and the droplet production unit 70 are switched by a valve B1.

The polyether storage section 62 is configured to store the polyether aqueous solution. The concentration of the polyether aqueous solution stored in the polyether storage section 62 is preferably 0.3% by mass or more. In the mixed solution containing various components in the production of the droplets D, the concentration of the polyether is, for example, 0.1% to 40% by mass with respect to the total mass of the mixed solution. The concentration of the polyether aqueous solution stored in the polyether storage section 62 may be any concentration as long as the concentration of the polyether can be adjusted to fall within the above-described concentration range when the mixed solution is prepared. The polyether aqueous solution stored in the polyether storage section 62 may be a concentrated solution, and in this case, the aqueous medium may be supplied from the aqueous medium storage section 67 for dilution.

The polyether storage section 62 is connected to the droplet production unit 70 by a conduit 90. Connection and disconnection between the polyether storage section 62 and the droplet production unit 70 are switched by valves B1 and B2.

The photoisomerizable compound storage section 63 is configured to store the photoisomerizable compound aqueous solution. The concentration of the photoisomerizable compound aqueous solution stored in the photoisomerizable compound storage section 63 is preferably 0.3% by mass or more. In the mixed solution containing various components in the production of the droplets D, the concentration of the photoisomerizable compound is, for example, 0.1% to 10% by mass with respect to the total mass of the mixed solution. The concentration of the photoisomerizable compound aqueous solution stored in the photoisomerizable compound storage section 63 may be any concentration as long as the concentration of the photoisomerizable compound can be adjusted to fall within the above-described concentration range when the mixed solution is prepared. The photoisomerizable compound aqueous solution stored in the photoisomerizable compound storage section 63 may be a concentrated solution, and in this case, the aqueous medium may be supplied from the aqueous medium storage section 67 for dilution.

The photoisomerizable compound storage section 63 is connected to the droplet production unit 70 by a conduit 90. Connection and disconnection between the photoisomerizable compound storage section 63 and the droplet production unit 70 are switched by valves B1, B2, and B3.

The photoisomerizable compound stored in the photoisomerizable compound storage section 63 is preferably stored as an isomer that forms a gel. For example, for a photoisomerizable compound in which the trans form is in a gel state and the cis form is in a sol state, the trans form is preferably stored in the photoisomerizable compound storage section 63. In the case of the compound (P) described later, it is preferable that the trans form of the compound (P) is stored in the photoisomerizable compound storage section 63.

The first component storage section 64 is configured to store a liquid (solution or dispersion liquid) containing the first component. The above-described liquid can be selected depending on the type of the first component. Examples of a medium of the liquid include the same media as those listed as the aqueous medium described above. The concentration of the first component in the liquid stored in the first component storage section 64 is not particularly limited, and can be appropriately set depending on the type of the first component. The concentration of the first component in the liquid stored in the first component storage section 64 may be any concentration as long as the concentration of the first component can be adjusted to fall within a desired concentration range when the mixed solution is prepared. The liquid containing the first component, stored in the first component storage section 64, may be a concentrated solution, and in this case, the aqueous medium may be supplied from the aqueous medium storage section 67 for dilution.

The first component storage section 64 is connected to the droplet production unit 70 by a conduit 90. Connection and disconnection between the first component storage section 64 and the droplet production unit 70 are switched by valves B1, B2, B3, and B4.

The second component storage section 65 is configured to store a liquid (solution or dispersion liquid) containing the second component. The above-described liquid can be selected depending on the type of the second component. Examples of a medium of the liquid include the same media as those listed as the aqueous medium described above. The concentration of the second component in the liquid stored in the second component storage section 65 is not particularly limited, and can be appropriately set depending on the type of the second component. The concentration of the second component in the liquid stored in the second component storage section 65 may be any concentration as long as the concentration of the second component can be adjusted to fall within a desired concentration range when the mixed solution is prepared. The liquid containing the second component, stored in the second component storage section 65, may be a concentrated solution, and in this case, the aqueous medium may be supplied from the aqueous medium storage section 67 for dilution.

The second component storage section 65 is connected to the droplet production unit 70 by a conduit 90. Connection and disconnection between the second component storage section 65 and the droplet production unit 70 are switched by valves B1, B2, B3, B4, and B5.

The detection reagent storage section 66 is configured to store a detection reagent aqueous solution. The detection reagent is a reagent capable of detecting the interaction between the first component and the second component. The detection reagent may be supplied to the droplet production unit 70 during the production of the first droplet D1, or may be supplied to the droplet production unit 70 during the production of the second droplet D2. Alternatively, the detection reagent may be supplied to the droplet production unit 70 at both timings of producing the first droplet D1 and producing the second droplet D2.

The detection reagent aqueous solution stored in the detection reagent storage section 66 may be a concentrated solution, and in this case, the aqueous medium may be supplied from the aqueous medium storage section 67 for dilution.

The detection reagent storage section 66 is connected to the droplet production unit 70 by a conduit 90. Connection and disconnection between the detection reagent storage section 66 and the droplet production unit 70 are switched by valves B1, B2, B3, B4, B5, and B6.

The aqueous medium storage section 67 is configured to store the aqueous medium. The aqueous medium is used for diluting the mixed solution during the production of the droplets D, cleaning the mixing tank of the droplet production unit 70 after the production of the droplets D, and the like.

The aqueous medium storage section 67 is connected to the droplet production unit 70 by a conduit 90. Connection and disconnection between the aqueous medium storage section 67 and the droplet production unit 70 are switched by valves B1, B2, B3, B4, B5, B6, and B7.

### <<Output unit, input unit, communication unit, and memory>>

The droplet control device 1 includes the output unit 50, the input unit 51, and the communication unit 52.

The output unit 50 outputs various types of information such as a monitoring result of the monitoring unit 20. The output unit 50 includes, for example, a display, a speaker, and the like. The display displays the information. For example, the display displays various types of information output from the processor 40 as an image, or displays a Graphical User Interface (GUI) for receiving various input operations from a user. For example, the display is a Liquid Crystal Display (LCD), an organic Electro Luminescence (EL) display, or the like. The speaker outputs the information output by the processor 40 as sound.

The input unit 51 receives various input operations from the user, converts the received input operations into electrical signals, and outputs them to the processor 40. The input unit 51 includes, for example, physical operating components such as a mouse, a keyboard, a trackball, a switch, a button, a joystick, and a touch panel. The input unit 51 may be, for example, a user interface that receives voice input such as a microphone. When the input unit 51 is a touch panel, the input unit 51 may also have a display function such as the display.

The input unit 51 is not limited to a unit including physical operating components such as a mouse and a keyboard. For example, an electrical signal processing circuit that receives, from an external input device provided separately from the device, an electrical signal corresponding to an input operation and outputs the received electrical signal to a control circuit is also included in examples of the input unit 51.

The communication unit 52 includes, for example, a Network Interface Card (NIC), an antenna for wireless communication, and the like. The communication unit 52 communicates with an external device through a communication network NW, for example.

The communication network NW may mean any information communication network utilizing telecommunication technology. For example, the communication network NW includes a Local Area Network (LAN), a Wide Area Network (WAN), the Internet, a telephone communication line network, an optical fiber communication network, a cable communication network, a satellite communication network, and the like.

The external device may be, for example, a computer connected to the LAN. Such a computer is also referred to as a workstation. In addition, the external device may be a cloud server connected to the WAN or the Internet.

The memory 53 may be implemented by, for example, semiconductor memory elements such as Random Access Memory (RAM) and flash memory, a hard disk, or an optical disk. These non-transitory storage media may be implemented by one or more other storage devices connected through the communication network NW, such as a Network Attached Storage (NAS) and an external storage server device. In addition, the memory 53 may include a non-transitory storage medium such as a ROM (Read Only Memory) and a register.

### <<Processor>>

The processor 40 controls an operation of the droplet control device 1. The operations of the units of the droplet control device 1 described above are controlled by the processor 40. The processor 40 implements such control by executing, for example, a program stored in the memory 53.

The processor 40 may be implemented by, for example, circuitry such as a Central Processing Unit (CPU), a Graphics Processing Unit (GPU), an applicationspecific integrated circuit (ASIC), a programmable logic device (for example, a simple programmable logic device (SPLD) or a complex programmable logic device (CPLD)), and a field-programmable gate array (FPGA). Instead of storing the program in the memory 53, the program may be directly incorporated into the circuitry of the processor 40. In this case, the processor 40 implements its functions by reading and executing the program embedded in the circuitry. The above-described program may be stored in the memory 53 in advance, or may be stored in a non-transitory storage medium such as a DVD and a CD-ROM and installed into the memory 53 from the non-transitory storage medium by the non-transitory storage medium being mounted in a drive device (not shown) of the droplet control device 1. The processor 40 is not limited to being configured as a single circuit, and may be configured as a single hardware processor by combining a plurality of independent circuits to implement each function. In addition, a plurality of components may be integrated into one hardware processor to implement each function.

The processor 40 includes a container controller 41, a light irradiation unit controller 42, a droplet production unit controller 43, a monitoring unit controller 44, an output unit controller 45, an input unit controller 46, and a communication unit controller 47, and has a function of controlling the operations of the units described above.

The container controller 41 has a function of controlling the container 10. The container controller 41 may have a function of controlling the temperature controller 10t to control the temperature of the droplet storage space.

The light irradiation unit controller 42 has a function of controlling the light irradiation unit 30. The light irradiation unit controller 42 may have a function of controlling, for example, at least one operation selected from the group consisting of a timing of starting light irradiation, a light irradiation time, a wavelength of irradiation light, and a light irradiation position in the light irradiation unit 30.

The droplet production unit controller 43 has a function of controlling the droplet production unit 70. The droplet production unit controller 43 may have a function of controlling the supply of the components from the polysaccharide storage section 61, the polyether storage section 62, the photoisomerizable compound storage section 63, the first component storage section 64, the second component storage section 65, the detection reagent storage section 66, and the aqueous medium storage section 67 to the droplet production unit 70. For example, the droplet production unit controller 43 may control the supply of these components to the droplet production unit 70 by controlling the valves B1 to B7.

The droplet production unit controller 43 may have a function of controlling the stirring mechanism 71 of the droplet production unit 70. For example, the droplet production unit controller 43 may have a function of controlling a timing of operation, a stirring time, a stirring intensity, and the like of the stirring mechanism 71.

The droplet production unit controller 43 may have a function of controlling transfer of the droplets D from the droplet production unit 70 to the container 10. For example, the droplet production unit controller 43 may control the transfer of the droplets D from the droplet production unit 70 to the container 10 by controlling a valve B9.

The droplet production unit controller 43 may have a function of controlling the cleaning of the mixing tank of the droplet production unit 70. For example, the droplet production unit controller 43 may have a function of controlling a timing of supply of the aqueous medium from the aqueous medium storage section 67 to the droplet production unit 70, and discharge of the waste liquid to the waste liquid storage section 68. For example, the droplet production unit controller 43 may control the cleaning of the droplet production unit 70 by controlling the valves B1 to B8.

The droplet production unit controller 43 may have a function of controlling the temperature in the mixing tank of the droplet production unit 70.

The monitoring unit controller 44 has a function of controlling the monitoring unit 20. The monitoring unit controller 44 may have a function of controlling, for example, at least one operation selected from the group consisting of a timing of starting monitoring, a monitoring time, and a monitoring position in the monitoring unit 20.

The output unit controller 45 has a function of outputting various types of information such as the monitoring result by the monitoring unit 20 through the output unit 50.

The input unit controller 46 has a function of processing information input from the input unit 51.

The communication unit controller 47 has a function of controlling communication through the communication unit 52.

### (Operation example of droplet control device)

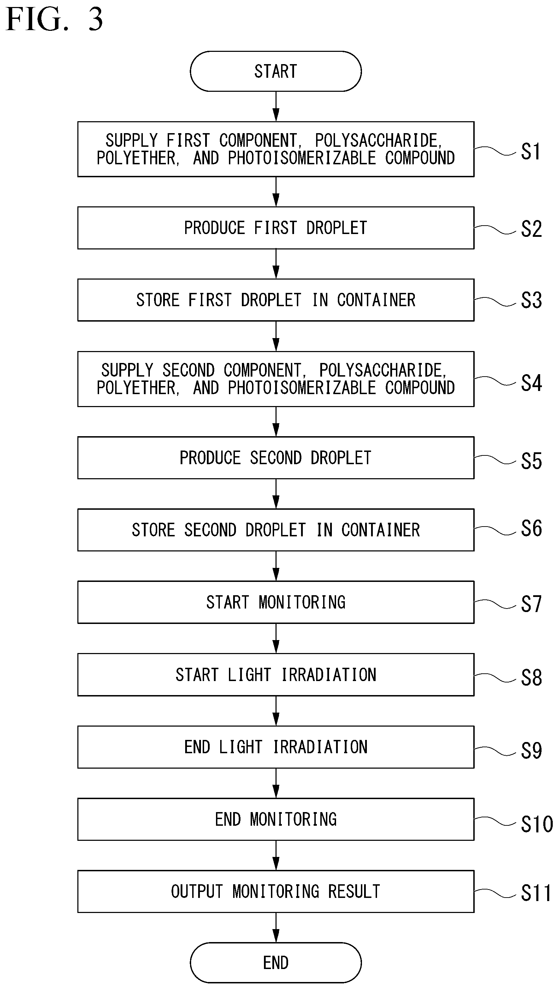

FIG. 3 is a flowchart showing an operation example of the droplet control device 1.

In a step S1, a polysaccharide aqueous solution, a polyether aqueous solution, a photoisomerizable compound aqueous solution, and a liquid containing the first component are co-supplied to the droplet production unit 70 from the polysaccharide storage section 61, the polyether storage section 62, the photoisomerizable compound storage section 63, and the first component storage section 64. At this time, each component is supplied in an amount necessary for producing the first droplet D1. As necessary, the detection reagent may be supplied from the detection reagent storage section 66 to the droplet production unit 70. Alternatively, as necessary, the aqueous medium may be supplied from the aqueous medium storage section 67 to the droplet production unit 70.

The supply amount of each component may be set in advance, or may be set by the user through the input unit 51.

The supply of each component to the droplet production unit 70 may be performed by a pump (not shown) or the like.

In a step S2, the mixed liquid of the components supplied to the mixing tank of the droplet production unit 70 is mixed and stirred to produce the first droplet D1. A stirring time may be set in advance, or may be set by the user through the input unit 51. Alternatively, the user may be allowed to manually stop the stirring through the input unit 51.

In a step S3, the first droplet D1 produced in the step S2 is stored in the container 10. The transfer of the first droplet D1 from the droplet production unit 70 to the container 10 is performed by controlling the valve B9. The entire amount of the aqueous medium containing the first droplet D1 may be transferred to the container 10, or a part thereof may be transferred to the container 10 and the rest may be stored in the waste liquid storage section 68.

The transfer of the aqueous medium containing the first droplet D1 to the container 10 or the waste liquid storage section 68 may be performed by a pump (not shown) or the like.

In a step S4, a polysaccharide aqueous solution, a polyether aqueous solution, a photoisomerizable compound aqueous solution, and a liquid containing the second component are co-supplied to the droplet production unit 70 from the polysaccharide storage section 61, the polyether storage section 62, the photoisomerizable compound storage section 63, and the second component storage section 65. At this time, each component is supplied in an amount necessary for producing the second droplet D2. As necessary, the detection reagent may be supplied from the detection reagent storage section 66 to the droplet production unit 70. Alternatively, as necessary, the aqueous medium may be supplied from the aqueous medium storage section 67 to the droplet production unit 70.

The supply amount of each component may be set in advance, or may be set by the user through the input unit 51.

The supply of each component to the droplet production unit 70 may be performed by a pump (not shown) or the like.

After the step S3 and before the step S4, the aqueous medium may be supplied from the aqueous medium storage section 67 to clean the mixing tank of the droplet production unit 70.

In a step S5, the mixed liquid of the components supplied to the mixing tank of the droplet production unit 70 is mixed and stirred to produce the second droplet D2. A stirring time may be set in advance, or may be set by the user through the input unit 51. Alternatively, the user may be allowed to manually stop the stirring through the input unit 51.

In a step S6, the second droplet D2 produced in the step S5 is stored in the container 10. The transfer of the second droplet D2 from the droplet production unit 70 to the container 10 is performed by controlling the valve B9. The entire amount of the aqueous medium containing the second droplet D2 may be transferred to the container 10, or a part thereof may be transferred to the container 10 and the rest may be stored in the waste liquid storage section 68.

Through the step S6, the aqueous medium containing the first droplet D1 and the second droplet D2 is held in the container 10.

In a step S7, the monitoring unit 20 starts monitoring the droplets D in the container 10. The state of monitoring may be displayed on a display or the like, which is the output unit 50. In addition, monitoring images or videos may be recorded in the memory 53 or an external storage device from time to time.

In a step S8, the light irradiation unit 30 starts light irradiation to the aqueous medium containing the droplets D in the container 10. A wavelength of the light irradiated at this time is a wavelength that causes the photoisomerizable compound to undergo the photoisomerization. The wavelength of light irradiation and the light irradiation time may be set in advance according to the type of the photoisomerizable compound, or may be set by the user through the input unit 51. The light irradiation time may be any time sufficient for the photoisomerizable compound to undergo the photoisomerization. Examples of the light irradiation time include approximately 0.1 seconds to 15 minutes. The light irradiation time may be 0.1 seconds or longer and 15 minutes or shorter, 0.5 seconds or longer and 15 minutes or shorter, 1 second or longer and 15 minutes or shorter, 5 seconds or longer and 15 minutes or shorter, or 10 seconds or longer and 15 minutes or shorter.

In a step S9, the light irradiation unit 30 ends the light irradiation.

In a step S10, the monitoring unit 20 ends the monitoring.

In a step S11, the monitoring result is output through the output unit 50.

As a result, a series of operation flows is ended.

In the above-described operation example, the order of the step S7 and the step S8 may be interchanged. The order of the step S9 and the step S10 may be interchanged.

A start time point of the monitoring in the step 7 may be set to a predetermined time before the start of the light irradiation, simultaneously with the start of the light irradiation, or after a lapse of a predetermined time from the start of the light irradiation, with reference to the time point at which the light irradiation unit starts irradiation of light to the aqueous medium.

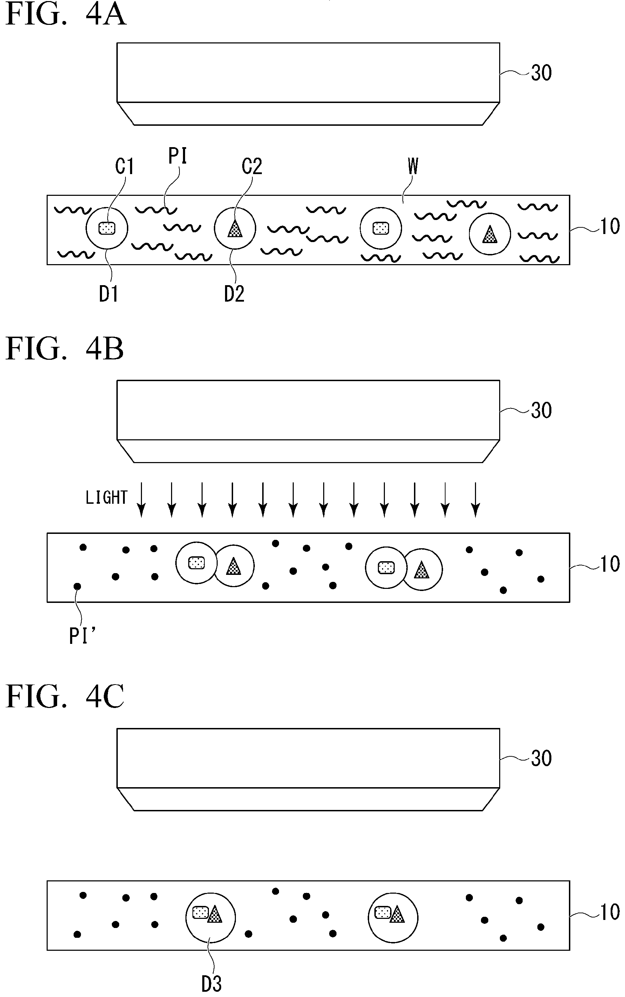

FIGS. 4A to 4C are schematic views showing behaviors of the photoisomerizable compound and the droplets D in the step S8.

FIG. 4A shows a state before the light irradiation. Before the light irradiation, the photoisomerizable compound PI forms a fiber-like structure. When the photoisomerizable compound PI is the compound (P), the compound (P) before the light irradiation is in a trans form.

Since the photoisomerizable compound PI forms the fiber-like structure, the aqueous medium W is in a gel state. Accordingly, the first droplet D1 containing the first component C1 and the second droplet D2 containing the second component C2 are maintained in the aqueous medium W without being fused with each other.

FIG. 4B shows a state in which light is irradiated by the monitoring unit 20. By the light from the monitoring unit 20, the photoisomerizable compound PI is isomerized to become a photoisomerizable compound PI' which is an isomer of the photoisomerizable compound PI. When the photoisomerizable compound PI is the compound (P), the photoisomerizable compound PI' is in a cis form of the compound (P).

The photoisomerizable compound PI' loses its fiber-like structure and is dispersed in the aqueous medium W. As a result, the aqueous medium W becomes a sol state. This makes fusion of the first droplet D1 and the second droplet D2 more likely to occur in the aqueous medium W. Therefore, the fusion of the first droplet D1 and the second droplet D2 is started simultaneously with the start of the light irradiation. Accordingly, the interaction between the first component C1 and the second component C2 is also started.

FIG. 4C shows a state after the light irradiation. After the light irradiation, the third droplet D3 generated by the fusion of the first droplet D1 and the second droplet D2 is present. The first component C1 and the second component C2 are encapsulated in the third droplet D3. Inside the third droplet D3, the interaction between the first component C1 and the second component C2 proceeds.

Since the droplet control device according to the first embodiment has the above-described configuration, the start time point of the interaction between the first component and the second component can be controlled by light irradiation. Therefore, the interaction of the plurality of components can be monitored over time from the start of the interaction.

### (Modification example)

The droplet control device may include two or more droplet production units. In this case, the first droplet D1 and the second droplet D2 may be produced in separate droplet production units.

The droplet control device may be configured such that a container holding the aqueous medium containing the first droplet D1 and the second droplet D2 is installed in the device. In this case, the storage section for each component, the droplet production unit, and the container need not be provided in the droplet control device. The droplet control device may include a holder for holding the container installed by the user.

### <Second embodiment>

### (Configuration example of droplet control device)

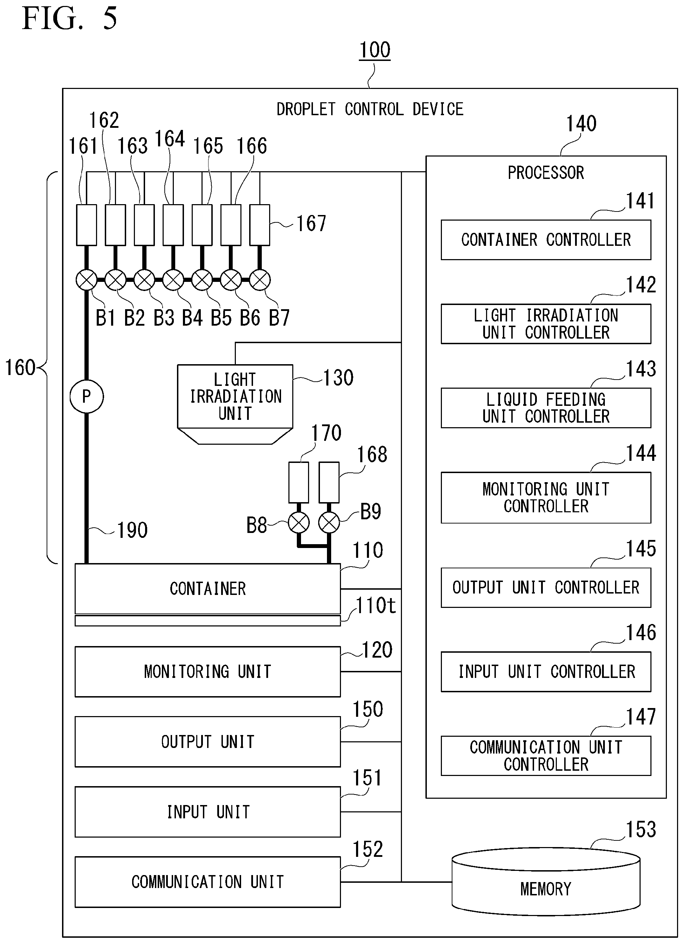

FIG. 5 is a schematic view showing an example configuration of a droplet control device according to a second embodiment. A droplet control device 100 includes a container 110, a monitoring unit 120, a light irradiation unit 130, an output unit 150, an input unit 151, a communication unit 152, a memory 153, a waste liquid storage section 168, and a droplet collection unit 170, and a processor 140 configured to control operations of these components. In addition, the droplet control device 100 includes, as storage sections for various components, a polysaccharide storage section 161, a polyether storage section 162, a photoisomerizable compound storage section 163, a first component storage section 164, a second component storage section 165, a detection reagent storage section 166, and an aqueous medium storage section 167.

The droplet control device 100 has a structure in which the components are fed from the component storage sections 161 to 167 to the container 110, without including the droplet production unit. That is, the container 110 also serves as the droplet production unit. The waste liquid storage section 168 and the droplet collection unit 170 are connected to the container 110 through a conduit 190.

Except for the above-described configuration, it is configured in the same manner as the droplet control device 1.

### <<Container>>

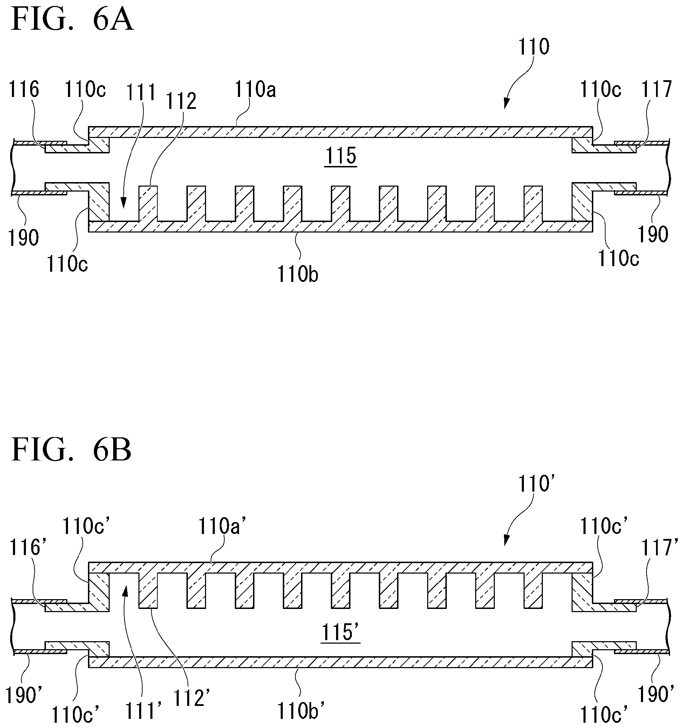

FIG. 6A is a schematic view showing an example of the container 110. The container 110 includes a plurality of wells 111 and a flow path 115 communicating with the plurality of wells 111. The container 110 is composed of a top substrate 110a, a bottom substrate 110b, and a side wall 110c. The side wall 110c is disposed between the top substrate 110a and the bottom substrate 110b, and defines a height in an internal space of the container 110. The side wall 110c is fixed to edge portions of the top substrate 110a and the bottom substrate 110b. In this manner, the inside of the container 110 is a closed space in which only a supply port 116 and a discharge port 117 are opened. Examples of the height of the internal space of the container 110 defined by the side wall 110c include 1 nm or more, 10 nm or more, 100 nm or more, 1 µm or more, and 10 µm or more. Examples of the upper limit value of the height include 5 mm or less, 1 mm or less, 500 µm or less, 100 µm or less, and 50 µm or less. Examples of the range of the height include 1 nm or more and 5 mm or less, 10 nm or more and 1 mm or less, 100 nm or more and 500 µm or less, 1 µm or more and 100 µm or less, and 10 µm or more and 50 µm or less.

On an inner bottom surface of the bottom substrate 110b, the plurality of wells 111 partitioned by partition walls 112 are provided. The flow path 115 is formed in an upper part of the wells 111, and the plurality of wells 111 communicate with each other through the flow path 115. The well 111 is a recessed portion having one open end, and is a space portion defined by the partition walls 112 and the inner surface of the bottom substrate 110b. A shape of the well 111 is not particularly limited, and examples thereof include a columnar shape, a polygonal columnar shape (quadrangular prism, hexagonal prism, and the like), and the like.

The volume of the well 111 is not particularly limited as long as it is the size capable of forming the droplets D. Examples of the volume of the well 111 include 10 nanoliters or less, 1 nanoliter or less, 100 picoliters or less, 10 picoliters or less, 1 picoliter or less, 100 femtoliters or less, and 50 femtoliters or less. The lower limit value of the volume of the well 111 is not particularly limited, and examples thereof include 1 attoliter or more, 10 attoliters or more, 100 attoliters or more, and 1 femtoliter or more. Examples of the range of the volume of the well 111 include 1 attoliter or more and 10 nanoliters or less, 10 attoliters or more and 100 picoliters or less, 100 attoliters or more and 10 picoliters or less, and 1 femtoliter or more and 1 picoliter or less.

The supply port 116 and the discharge port 117 are provided in the side wall 110c, and the conduit 190 is connected to each of the supply port 116 and the discharge port 117.

The liquid supplied from the supply port 116 into the container 110 through the conduit 190 passes through the flow path 115 and is discharged from the discharge port 117. In this process, the liquid is filled into the plurality of wells 111 through the flow path 115.

As described later, by changing the type of the liquid to be fed into the container 110 in a predetermined order, the droplets D are formed in the wells 111.

FIG. 6B shows a modification example of the container 110. A container 110' has the same configuration as the container 110 shown in FIG. 6A, except that a plurality of wells 111' are provided on an inner surface of a top substrate 110a' rather than on an inner surface of a bottom substrate 110b'.

A material for the container 110 is not particularly limited, but a member that transmits light irradiated by the light irradiation unit 130 is preferable. In addition, when the monitoring unit 120 includes a microscope unit, the entire container 110 may be composed of a transparent material with low autofluorescence. Examples of the transparent material with low autofluorescence include the same materials as described above.

The container 110 can be produced by, for example, a microfabrication technology using photolithography, a 3D printer technology, or the like.

The container 110 includes a temperature controller 110t for controlling a temperature of the internal space. The temperature controller 110t controls the temperature of the internal space according to the component to be encapsulated in the droplets D. A heater, a cooler, a thermostat, or the like can be used as the temperature controller 110t.

The container 110 may be removably attachable. For example, the droplet control device 100 may include a container holding part, and a container prepared by a user may be installed as the container 110 in the container holding part.

### <<Liquid feeding unit>>

The droplet control device 100 includes a liquid feeding unit 160 configured to feed a liquid to the internal space of the container 110. The liquid feeding unit 160 feeds the liquid to the plurality of wells 111 through the flow path 115. The liquid feeding unit 160 is composed of the polysaccharide storage section 161, the polyether storage section 162, the photoisomerizable compound storage section 163, the first component storage section 164, the second component storage section 165, the detection reagent storage section 166, the aqueous medium storage section 167, the waste liquid storage section 168, the droplet collection unit 170, and a pump P.

Configurations of the polysaccharide storage section 161, the polyether storage section 162, the photoisomerizable compound storage section 163, the first component storage section 164, the second component storage section 165, the detection reagent storage section 166, and the detection reagent storage section 166 are the same as those in the first embodiment. The photoisomerizable compound storage section 163 may store, instead of a photoisomerizable compound aqueous solution, an aqueous solution containing the photoisomerizable compound and the polyether (photoisomerizable compound/polyether aqueous solution) or an aqueous solution containing the photoisomerizable compound and the polysaccharide (photoisomerizable compound/polysaccharide aqueous solution). When the photoisomerizable compound has a high affinity for the polyether, it is preferable that the photoisomerizable compound/polyether aqueous solution is stored in the photoisomerizable compound storage section 163. When the photoisomerizable compound has a high affinity for the polysaccharide, it is preferable that the photoisomerizable compound/polysaccharide aqueous solution is stored in the photoisomerizable compound storage section 163.

The polysaccharide aqueous solution stored in the polysaccharide storage section 161 is supplied to the inside of the container 110 through the conduit 190 by switching a valve B1. The polysaccharide aqueous solution is fed into the container 110 by a driving force of the pump P. The polysaccharide aqueous solution that has flowed into the container 110 from the supply port 116 passes through the flow path 115, is discharged from the discharge port 117, and is stored in the waste liquid storage section 168. The connection state between the container 110 and the waste liquid storage section 168 is switched by valves B8 and B9.

When the polysaccharide aqueous solution stored in the polysaccharide storage section 161 is a concentrated solution, the aqueous medium may be supplied from the aqueous medium storage section 167 to dilute the polysaccharide aqueous solution in the conduit 190.

Other components are also fed into the container 110 in the same manner.

The liquid feeding unit 160 may be controlled to perform (i) feeding of the polysaccharide aqueous solution to the plurality of wells 111, (ii) feeding of the polyether solution to the plurality of wells 111, and (iii) feeding of the photoisomerizable compound/polyether aqueous solution to the plurality of wells 111 in this order.

Alternatively, the liquid feeding unit 160 may be controlled to perform (i) feeding of the polyether aqueous solution to the plurality of wells 111, (ii) feeding of the polysaccharide solution to the plurality of wells 111, and (iii) feeding of the photoisomerizable compound/polysaccharide aqueous solution to the plurality of wells 111 in this order.

The droplet collection unit 170 is configured to collect the droplets D held in any well 111. The container 110 and the droplet collection unit 170 are connected through the conduit 190. The connection state between the container 110 and the droplet collection unit 170 is switched by valves B8 and B9.

### <<Monitoring unit>>

The monitoring unit 120 is configured to monitor the state of the droplets in the container 110. As the monitoring unit 120, a unit including the above-described microscope unit can be used. In one embodiment, the monitoring unit 120 is controlled to start monitoring after the (iii) described above.

### <<Light irradiation unit>>

The light irradiation unit 130 is configured to irradiate with light to the aqueous medium containing the droplets D in the container 110. As a light source of the light irradiation unit 130, the same light source as that of the light irradiation unit 30 in the first embodiment can be used.

The light irradiation unit 130 includes a mechanism for irradiating light to wells 111 excluding a specific well 111 among the plurality of wells 111 in the container 110. The light irradiation unit 130 may have, for example, a member that shields light irradiated from the light source with respect to the specific well 111. Examples of the light shielding member include a photomask. Alternatively, the light irradiation unit 130 may include a laser device capable of controlling a light irradiation position.

As the output unit 150, the input unit 151, and the communication unit 152, the same units as those in the first embodiment can be used.

### <<Processor>>

The processor 140 controls an operation of the droplet control device 100. The operations of the units of the droplet control device 1 described above are controlled by the processor 40. The processor 40 implements such control by executing, for example, a program stored in the memory 53. The hardware configuration of the processor 140 may be the same as that in the first embodiment.

The processor 140 includes a container controller 141, a light irradiation unit controller 142, a liquid feeding unit controller 143, a monitoring unit controller 144, an output unit controller 145, an input unit controller 146, and a communication unit controller 147, and has a function of controlling operations of the above-described units.

The container controller 141 has a function of controlling the container 110. The container controller 141 may have a function of controlling the temperature controller 110t to control the temperature of the internal space of the container 110.

The light irradiation unit controller 142 has a function of controlling the light irradiation unit 130. The light irradiation unit controller 142 may have a function of controlling, for example, at least one operation selected from the group consisting of a timing of starting light irradiation, a light irradiation time, a wavelength of irradiation light, and a light irradiation position in the light irradiation unit 130. The light irradiation unit controller 142 may have a function of controlling the photomask. For example, the light irradiation unit controller 142 may have a function of selecting a well 111 for blocking irradiation light based on monitoring information of the monitoring unit 120, and disposing the photomask at the position of the specific well 111. Alternatively, the light irradiation unit controller 142 may have a function of controlling the laser device included in the light irradiation unit 130 to irradiate laser light to wells 111 other than the specific well 111.

The liquid feeding unit controller 143 has a function of controlling the liquid feeding unit 160. The liquid feeding unit controller 143 may have a function of controlling feeding of each component from the polysaccharide storage section 161, the polyether storage section 162, the photoisomerizable compound storage section 163, the first component storage section 164, the second component storage section 165, the detection reagent storage section 166, and the aqueous medium storage section 167 to the container 110. For example, the liquid feeding unit controller 143 may control the feeding of these components to the container 110 by controlling valves B1 to B7 and the pump P. The liquid feeding unit controller 143 may have a function of controlling the order of liquid feeding from each component storage section. The liquid feeding unit controller 143 may have a function of controlling the speed of liquid feeding from each component storage section.

The liquid feeding unit controller 143 may have a function of controlling the valves B8 and B9 to switch a destination for storing the liquid discharged from the container 110 between the droplet collection unit 170 and the waste liquid storage section 168.

The monitoring unit controller 144 has a function of controlling the monitoring unit 20. The monitoring unit controller 44 may have a function of controlling, for example, at least one operation selected from the group consisting of a timing of starting monitoring, a monitoring time, and a monitoring position in the monitoring unit 20.

The output unit controller 145, the input unit controller 146, and the communication unit controller 47 are the same as those in the first embodiment.

### (Operation example of droplet control device)

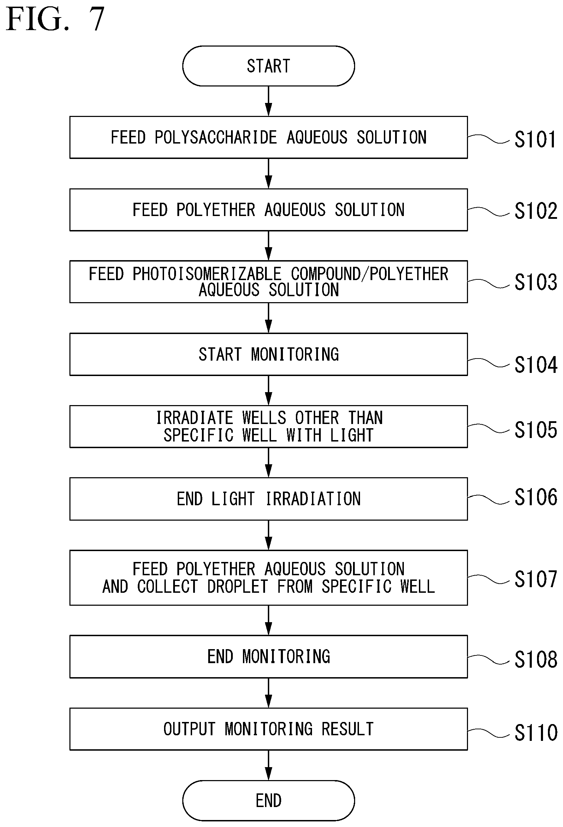

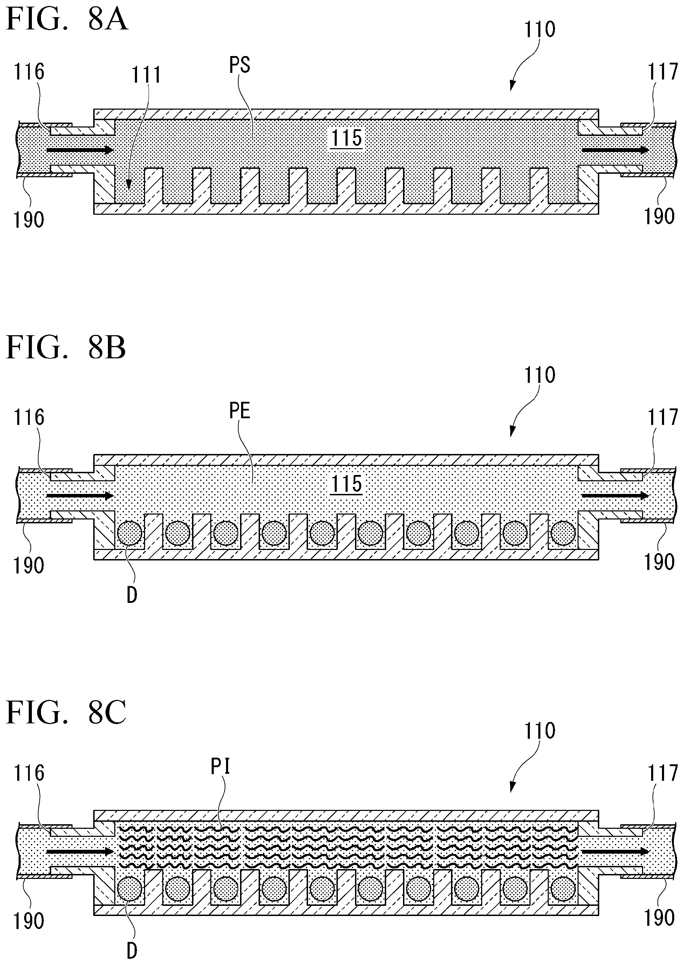

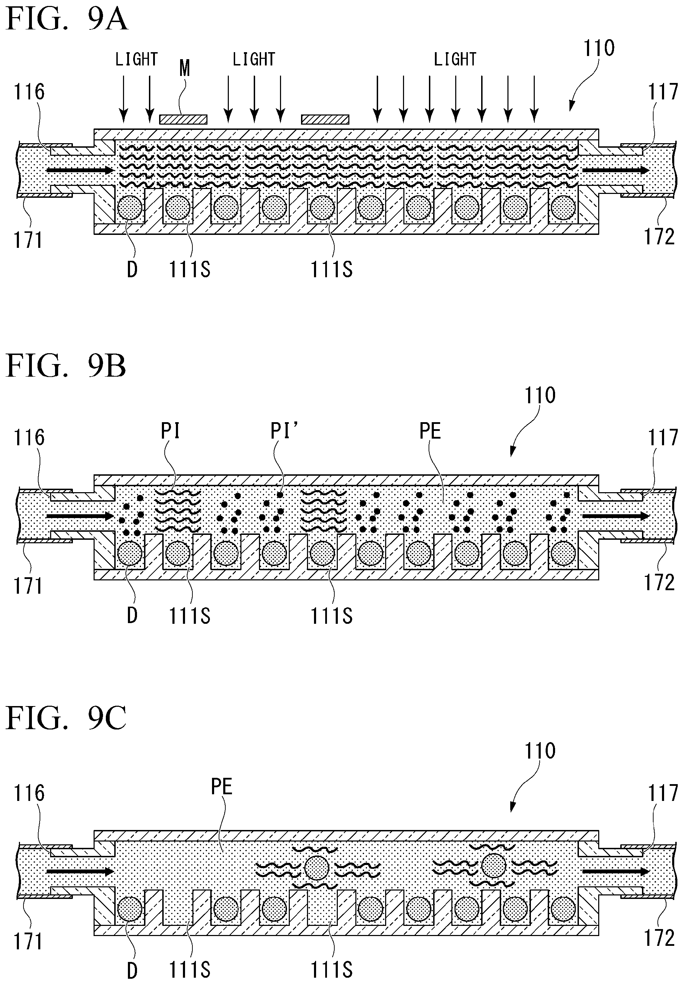

FIG. 7 is a flowchart showing an operation example of the droplet control device 100. FIG. 7 shows an operation example when using a photoisomerizable compound having a higher affinity for the polyether than for the polysaccharide. FIGS. 8A to 8C and 9A to 9C are schematic views for explaining the state inside the container 110 in each step.

In a step S101, the polysaccharide aqueous solution is fed from the polysaccharide storage section 161 to the container 110. The feeding of the polysaccharide aqueous solution is performed by the liquid feeding unit controller 143 controlling the valve B1 and the pump P. The feeding of the polysaccharide aqueous solution is performed until all the wells 111 in the container 110 are filled with the polysaccharide aqueous solution. At this time, the valves B8 and B9 are controlled so that the container 110 is connected to the waste liquid storage section 168. A liquid feeding time may be set in advance, or may be set by the user through the input unit 151. Alternatively, a filling state in the container 110 may be detected by a sensor or the like installed in the container 110, and stopping liquid feeding may be controlled.

The feeding rate of the polysaccharide aqueous solution is not particularly limited as long as it is a rate at which the well 111 is filled with the polysaccharide aqueous solution. The feeding rate of the polysaccharide aqueous solution may be appropriately adjusted according to the sizes of the container 110 and the wells 111. When the size of the well 111 is on the order of femtoliters, the feeding rate of the polysaccharide aqueous solution is, for example, 1 to 1000 µL/min, may be 10 to 800 µL/min, or may be 50 to 500 µL/min.

FIG. 8A shows a state in which the flow path 115 and the wells 111 in the container 110 are filled with the polysaccharide aqueous solution PS by the feeding of the polysaccharide aqueous solution PS. The arrow in the drawing indicates the liquid feeding direction.

In a step S102, the polyether aqueous solution is fed from the polyether storage section 162 to the container 110. The feeding of the polyether aqueous solution is performed by the liquid feeding unit controller 143 controlling the valves B1 and B2 and the pump P. The feeding of the polyether aqueous solution is performed until the flow path 115 in the container 110 is filled with the polyether aqueous solution. A liquid feeding time may be set in advance, or may be set by the user through the input unit 151. Alternatively, a formation state of the droplets D in the plurality of wells 111 may be detected by a sensor or the like installed in the container 110, and stopping liquid feeding may be controlled.

The feeding rate of the polyether aqueous solution is not particularly limited as long as it is a rate at which the polysaccharide aqueous solution in the flow path 115 is replaced with the polyether aqueous solution. The feeding rate of the polyether aqueous solution may be appropriately adjusted according to the sizes of the container 110 and the wells 111. When the size of the well 111 is on the order of femtoliters, the feeding rate of the polyether aqueous solution is, for example, 1 to 1000 µL/min, may be 10 to 800 µL/min, or may be 50 to 500 µL/min.

FIG. 8B shows a state in which the flow path 115 in the container 110 is filled with the polyether aqueous solution PE by the feeding of the polyether aqueous solution PE. At this time, the polysaccharide aqueous solution PS in the wells 111 remains, and the droplets D are formed in the wells 111.

In a step S103, the photoisomerizable compound/polyether aqueous solution is fed from the photoisomerizable compound storage section 163 to the container 110. The feeding of the photoisomerizable compound/polyether aqueous solution from the photoisomerizable compound storage section 163 is performed by the liquid feeding unit controller 143 controlling the valves B1 to B3 and the pump P.

Alternatively, the photoisomerizable compound aqueous solution may be fed from the photoisomerizable compound storage section 163, and the polyether aqueous solution may be fed from the polyether storage section 162, mixed in the conduit 190, and fed into the container 110. The feeding of the polyether aqueous solution from the polyether storage section 162 and the feeding of the photoisomerizable compound from the photoisomerizable compound storage section 163 are performed by the liquid feeding unit controller 143 controlling the valves B1 to B3 and the pump P.

The feeding of the photoisomerizable compound/polyether aqueous solution is performed until the flow path 115 in the container 110 is filled with the photoisomerizable compound/polyether aqueous solution. A liquid feeding time may be set in advance, or may be set by the user through the input unit 151. Alternatively, a viscosity state or the like of the liquid in the flow path 115 may be detected by a sensor or the like installed in the container 110, and stopping liquid feeding may be controlled.

The feeding rate of the photoisomerizable compound/polyether aqueous solution is not particularly limited as long as it is a rate at which the polyether aqueous solution in the flow path 115 is replaced with the photoisomerizable compound/polyether aqueous solution. The feeding rate of the photoisomerizable compound/polyether aqueous solution may be appropriately adjusted according to the sizes of the container 110 and the wells 111. When the size of the well 111 is on the order of femtoliters, the feeding rate of the polyether aqueous solution is, for example, 1 to 1000 µL/min, may be 10 to 800 µL/min, or may be 50 to 500 µL/min.

FIG. 8C shows a state in which the flow path 115 in the container 110 is filled with the polyether aqueous solution PE containing the photoisomerizable compound PI by the feeding of the photoisomerizable compound/polyether aqueous solution. At this time, the droplets D remain in the wells 111.

The photoisomerizable compound PI forms a fiber-like structure. When the photoisomerizable compound PI is the compound (P), the trans form of the compound (P) is fed.

In a step S104, the monitoring unit 120 starts monitoring the droplets D in the container 110. The state of monitoring may be displayed on a display or the like, which is the output unit 150. In addition, monitoring images or videos may be recorded in the memory 153 or an external storage device from time to time.

In a step S105, the light irradiation unit 130 irradiates light to wells 111 other than a specific well 111S. A wavelength of the light irradiated at this time is a wavelength that causes the photoisomerizable compound to undergo photoisomerization. The light irradiation time may be any time sufficient for the photoisomerizable compound to undergo the photoisomerization. Examples of the light irradiation time include approximately 0.1 seconds to 15 minutes. The light irradiation time may be 0.1 seconds or longer and 15 minutes or shorter, 0.5 seconds or longer and 15 minutes or shorter, 1 second or longer and 15 minutes or shorter, 5 seconds or longer and 15 minutes or shorter, or 10 seconds or longer and 15 minutes or shorter.

The specific well 111S to which the light irradiation is not performed is selected based on monitoring information of the monitoring unit 120. For example, the substance encapsulated in the droplets D is detected by the monitoring unit 120, and the well 111 holding the droplets D in which a desired substance is encapsulated is selected as the specific well.

For example, the light irradiation is performed on wells other than the specific well 111S by performing light irradiation while controlling so that a light shielding member such as a mask is disposed in the specific well 111S. Alternatively, the light irradiation may be performed on wells other than the specific well 111S by a laser device or the like capable of controlling a light irradiation position.

FIG. 9A shows a state in which wells 111 other than the specific well 111S are irradiated with light. A mask M is disposed at the position of the specific well 111S between the light irradiation unit 130 and the top substrate 110a of the container 110, and light irradiation to the specific well 111S is blocked.

FIG. 9B shows a change of the photoisomerizable compound PI due to the light irradiation. The photoisomerizable compound PI at a position irradiated with light undergoes photoisomerization and becomes a photoisomerizable compound PI', which is an isomer of the photoisomerizable compound P. The photoisomerizable compound PI' loses its fiber-like structure and is dispersed in the polyether aqueous solution PE. On the other hand, the photoisomerizable compound PI in and around the specific well 111S is not photoisomerized and remains the photoisomerizable compound PI. When the photoisomerizable compound PI is the compound (P), the photoisomerizable compound PI' is in a cis form of the compound (P).

In a step S106, the light irradiation unit 130 ends the light irradiation.

In a step S107, the polyether aqueous solution is fed from the polyether storage section 162 to the container 110. The feeding of the polyether aqueous solution is performed by the liquid feeding unit controller 143 controlling the valves B1 to B3 and the pump P. At this time, the valves B8 and B9 are controlled so that the container 110 is connected to the droplet collection unit 170. The feeding of the polyether aqueous solution is performed until the collection of the droplets D from the specific well 111S is completed.

The feeding rate of the polyether aqueous solution is not particularly limited as long as it is a rate at which the droplets D in the wells 111 irradiated with light in the step S105 remain in the wells 111 and the droplets D in the wells 111 not irradiated with light flow out. The feeding rate of the polyether aqueous solution may be appropriately adjusted according to the sizes of the container 110 and the wells 111. When the size of the well 111 is on the order of femtoliters, the feeding rate of the polyether aqueous solution is, for example, 1 to 1000 µL/min, may be 10 to 800 µL/min, or may be 50 to 500 µL/min.

FIG. 9C shows a state in which the droplets D in the specific well 111S flow out from the specific well 111S by the feeding of the polyether aqueous solution PE. In and around the specific well 111S, the photoisomerizable compound PI remains in a fiber-like form. By feeding the polyether aqueous solution PE, the droplets D in the specific well 111S flow out of the well together with the fiber-like photoisomerizable compound PI. The droplets D flowing out from the specific well 111S are discharged from the discharge port 117 and collected in the droplet collection unit 170 through the conduit 190.

In a step 108, the monitoring unit 120 ends the monitoring.

In a step S109, the monitoring result is output through the output unit 150.

As a result, a series of operation flows is ended.

In the above-described operation example, the order of the step S106 and the step S107 may be interchanged. The step S106 may be performed after the step S108.

### <<Another operation example (1)>>

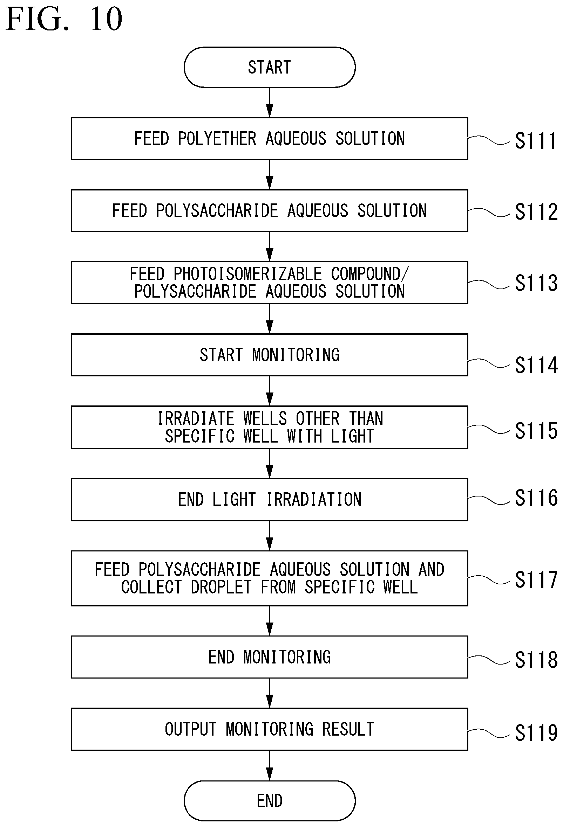

FIG. 10 is a flowchart showing another operation example of the droplet control device 100. FIG. 10 shows an operation example when a photoisomerizable compound having higher affinity for the polysaccharide than for the polyether is used.

In a step S111, the polyether aqueous solution is fed from the polyether storage section 162 to the container 110.

In a step S112, the polysaccharide aqueous solution is fed from the polysaccharide storage section 161 to the container 110.

In a step S113, the photoisomerizable compound/polysaccharide aqueous solution is fed from the photoisomerizable compound storage section 163 to the container 110. Alternatively, the photoisomerizable compound aqueous solution may be fed from the photoisomerizable compound storage section 163, and the polyether aqueous solution may be fed from the polysaccharide storage section 161, mixed in the conduit 190, and fed into the container 110.

In a step S114, the monitoring unit 120 starts monitoring the droplets D in the container 110.

In a step S115, the light irradiation unit 130 irradiates light to wells 111 other than a specific well 111S. A wavelength of the light irradiated at this time is a wavelength that causes the photoisomerizable compound to undergo photoisomerization. Selection of the specific well 111S and avoidance of light irradiation to the specific well 111S are controlled in the same manner as in the above-described step S105. The photoisomerizable compound PI at a position irradiated with light undergoes photoisomerization and becomes a photoisomerizable compound PI', which is an isomer of the photoisomerizable compound P. On the other hand, the photoisomerizable compound PI in and around the specific well 111S is not photoisomerized and remains the photoisomerizable compound PI.

In a step S116, the light irradiation unit 130 ends the light irradiation.

In a step S117, the polysaccharide aqueous solution is fed from the polysaccharide storage section 161 to the container 110. At this time, the valves B8 and B9 are controlled so that the container 110 is connected to the droplet collection unit 170. The feeding of the polysaccharide aqueous solution is performed until the collection of the droplets D from the specific well 111S is completed.

In a step 118, the monitoring unit 120 ends the monitoring.

In a step S119, the monitoring result is output through the output unit 150.

As a result, a series of operation flows is ended.

The operation flow is the same as the operation flow of FIG. 7, except that the polysaccharide phase and the polyether phase are opposite to those in the operation flow of FIG. 7.

### <<Another operation example (2)>>

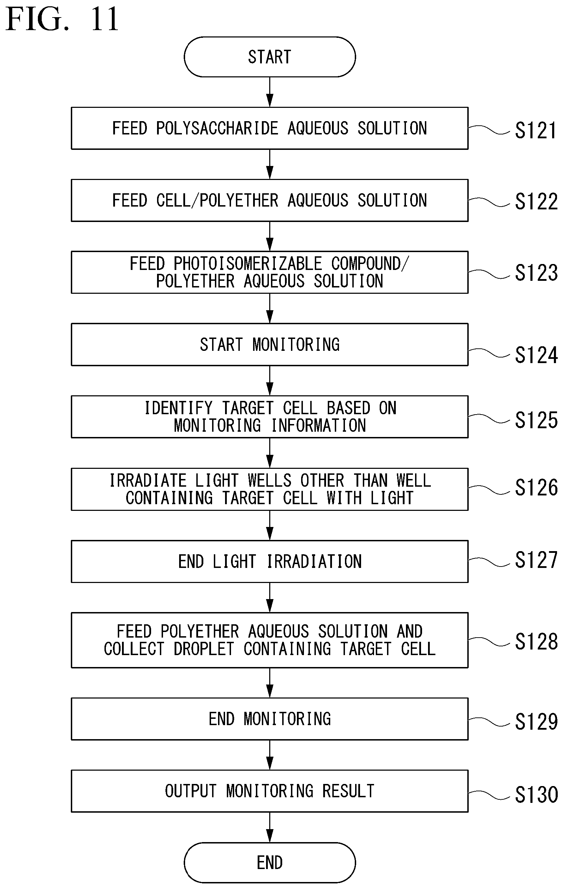

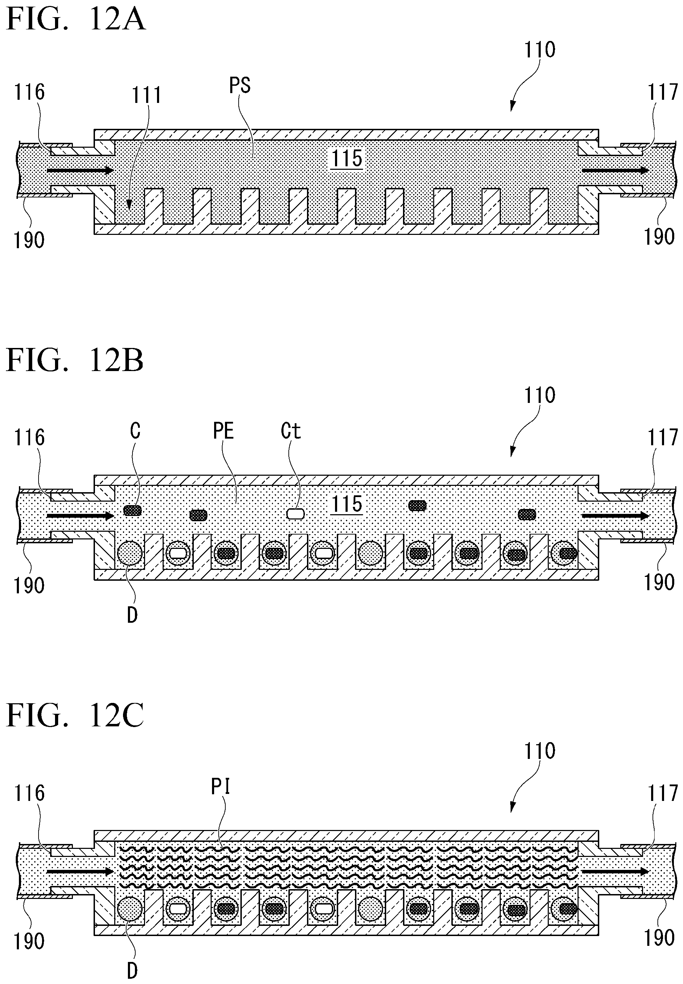

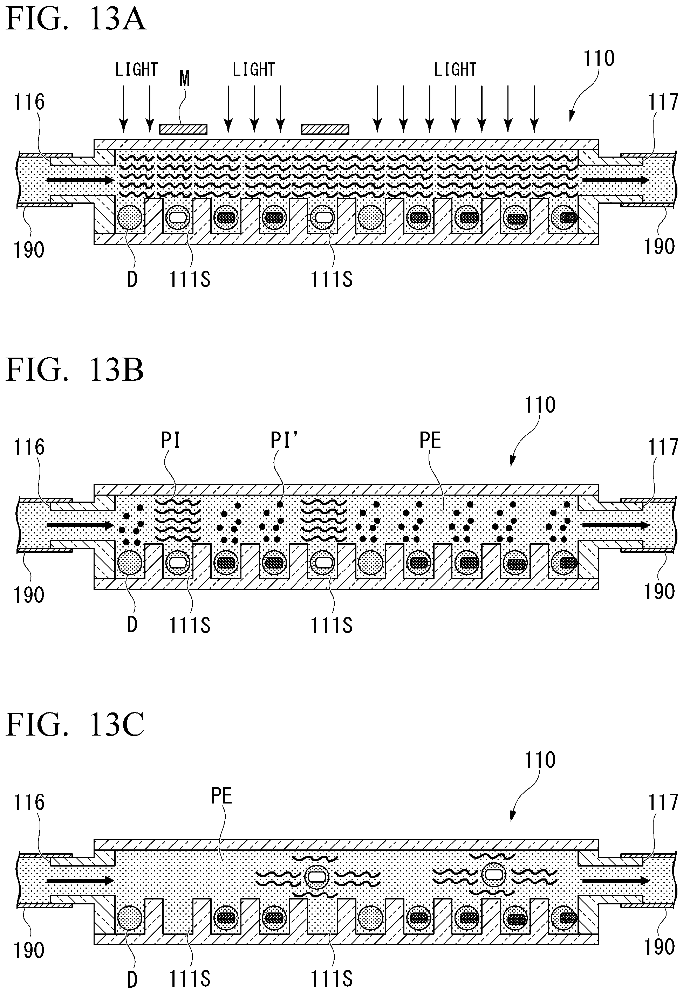

FIG. 11 is a flowchart showing another operation example of the droplet control device 100. FIG. 11 shows an operation example when target cells are collected using a photoisomerizable compound having higher affinity for the polyether than for the polysaccharide. FIGS. 12A to 12C and 13A to 13C are schematic views for explaining the state inside the container 110 in each step.

In a step S121, the polysaccharide aqueous solution is fed from the polysaccharide storage section 161 to the container 110. FIG. 12A shows a state in which the flow path 115 and the wells 111 in the container 110 are filled with the polysaccharide aqueous solution PS by the feeding of the polysaccharide aqueous solution PS. The arrow in the drawing indicates the liquid feeding direction.

In a step S122, a polyether aqueous solution containing cells is fed from the first component storage section 164 to the container 110. Alternatively, the polyether aqueous solution may be fed from the polyether storage section 162, and a cell dispersion liquid may be fed from the first component storage section 164, mixed in the conduit 190, and supplied to the inside of the container 110.

FIG. 12B shows a state in which the flow path 115 in the container 110 is filled with the polyether aqueous solution PE containing cells, by the feeding of the polyether aqueous solution PE. At this time, the polysaccharide aqueous solution PS in the wells 111 remains, and the droplets D are formed in the wells 111. The cells include target cells Ct and non-target cells C. Since the cells have a higher affinity for the polysaccharide than for the polyether, the cells are taken into the droplets D upon contact with the droplets D.

In a step S123, the photoisomerizable compound/polyether aqueous solution is fed from the photoisomerizable compound storage section 163 to the container 110. Alternatively, the photoisomerizable compound aqueous solution may be fed from the photoisomerizable compound storage section 163, and the polyether aqueous solution may be fed from the polyether storage section 162, mixed in the conduit 190, and fed into the container 110.

In the step S123, a detection reagent for detecting the target cells Ct may be fed from the detection reagent storage section 166. Examples of the detection reagent include an antibody that binds to a cell membrane protein or the like of the target cell Ct. A labeling substance such as a fluorescent dye may be bound to the antibody. The detection reagent fed into the container 110 binds to the target cells Ct encapsulated in the droplets D, thereby facilitating identification of the target cells Ct.

FIG. 12C shows a state in which the flow path 115 in the container 110 is filled with the polyether aqueous solution PE containing the photoisomerizable compound PI by the feeding of the photoisomerizable compound/polyether aqueous solution.

In a step S124, the monitoring unit 120 starts monitoring the droplets D in the container 110. The monitoring unit 120 observes the cells taken into the droplets D, and transmits information such as the shape of the cells to the processor 140. When the detection reagent is fed in the step S123, the monitoring unit 120 may detect a labeling substance or the like bound to the detection reagent. As a result, information on the binding state of the detection reagent to the cells may be obtained and transmitted to the processor 140.

In a step S125, the processor 140 identifies the target cells Ct from among the cells encapsulated in the droplets D based on monitoring information transmitted from the monitoring unit 120. In addition, the processor 140 identifies the specific well 111S holding the droplets D encapsulating the target cells Ct.

In a step S126, the light irradiation unit 130 irradiates light to wells 111 other than the specific well 111S containing the target cells Ct. A wavelength of the light irradiated at this time is a wavelength that causes the photoisomerizable compound to undergo photoisomerization.

FIG. 13A shows a state in which wells **111** other than the specific well 111S are irradiated with light. The droplets D encapsulating the target cells Ct are held in the specific well 111S. Droplets encapsulating the non-target cells C or not encapsulating cells are held in the other wells 111.

FIG. 13B shows a change of the photoisomerizable compound PI due to the light irradiation. The photoisomerizable compound PI at a position irradiated with light undergoes photoisomerization and becomes a photoisomerizable compound PI', which is an isomer of the photoisomerizable compound P. The photoisomerizable compound PI in and around the specific well 111S is not photoisomerized and remains the photoisomerizable compound PI.

In a step S127, the light irradiation unit 130 ends the light irradiation.

In a step S128, the polyether aqueous solution is fed from the polyether storage section 162 to the container 110. At this time, the valves B8 and B9 are controlled so that the container 110 is connected to the droplet collection unit 170.

FIG. 13C shows a state in which the droplets D encapsulating the target cells Ct in the specific well 111S flow out from the specific well 111S by the feeding of the polyether aqueous solution PE. The droplets D flowing out from the specific well 111S are discharged from the discharge port 117 and collected in the droplet collection unit 170 through the conduit 190.

In a step S129, the monitoring unit 120 ends the monitoring.

In a step S130, the monitoring result is output through the output unit 150.

As a result, a series of operation flows is ended.

### <<Another operation example (3)>>

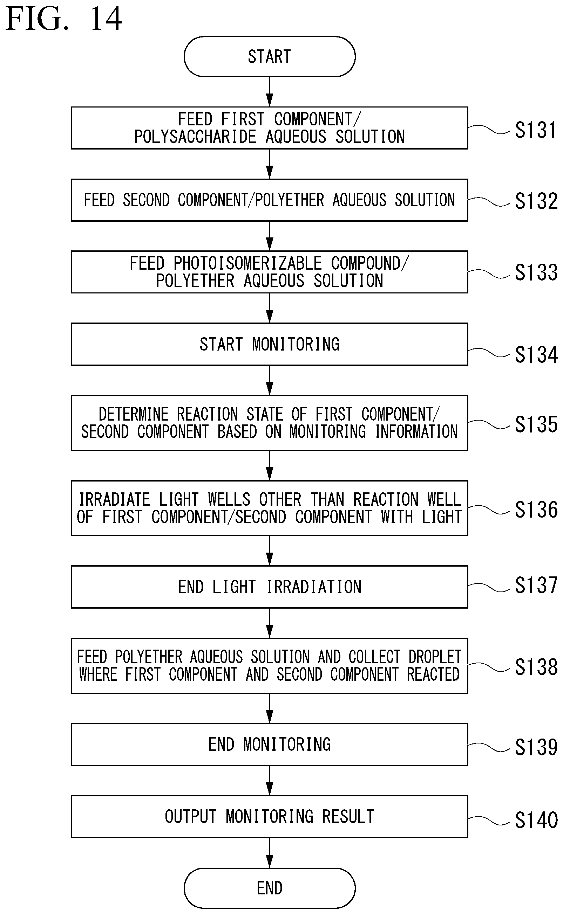

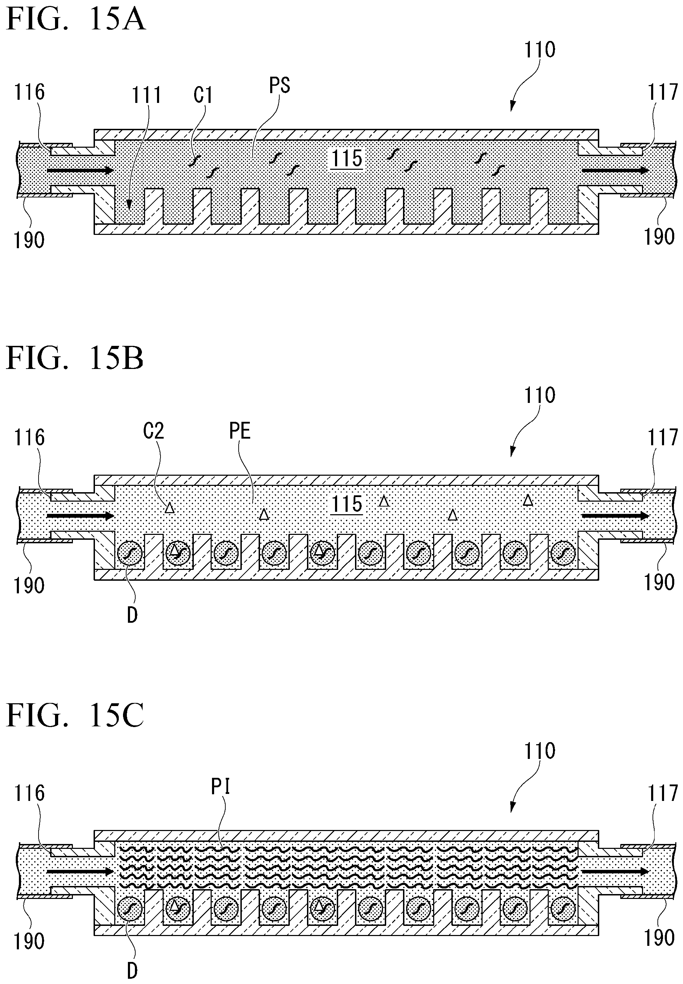

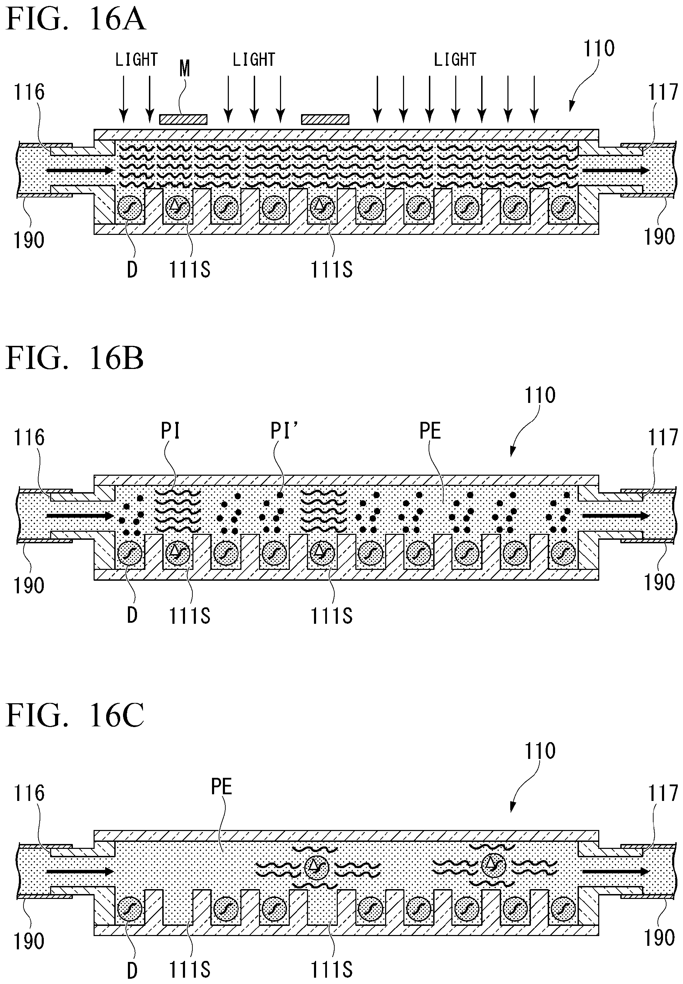

FIG. 14 is a flowchart showing another operation example of the droplet control device 100. FIG. 14 shows an operation example when collecting a component (second component) that reacts with an arbitrary component (first component) using a photoisomerizable compound having a higher affinity for the polyether than for the polysaccharide. It is preferable that the first component and the second component are substances having a higher affinity for the polysaccharide than for the polyether. FIGS. 15A to 15C and 16A to 16C are schematic views for explaining the state inside the container 110 in each step.

In a step S131, a polysaccharide aqueous solution containing the first component is fed from the first component storage section 164 to the container 110. Alternatively, the polysaccharide aqueous solution may be fed from the polysaccharide storage section 161, and a first component aqueous solution may be fed from the first component storage section 164, mixed in the conduit 190, and supplied to the inside of the container 110.

FIG. 15A shows a state in which the flow path 115 and the wells 111 in the container 110 are filled with the polysaccharide aqueous solution PS containing the first component C1, by the feeding of the polysaccharide aqueous solution PS containing the first component C1. The arrow in the drawing indicates the liquid feeding direction.

In a step S132, a polyether aqueous solution containing the second component is fed from the second component storage section 165 to the container 110. Alternatively, the polyether aqueous solution may be fed from the polyether storage section 162, and a second component aqueous solution may be fed from the second component storage section 165, mixed in the conduit 190, and supplied to the inside of the container 110.

FIG. 15B shows a state in which the flow path 115 in the container 110 is filled with the polyether aqueous solution PE containing the second component C2, by the feeding of the polyether aqueous solution PE containing the second component C2. At this time, the polysaccharide aqueous solution PS in the wells 111 remains, and the droplets D are formed in the wells 111. At this time, the first component C1 is encapsulated in the droplets D. The second component C2 having binding property to the first component C1 is taken into the droplets D by binding to the first component C1.

In a step S133, the photoisomerizable compound/polyether aqueous solution is fed from the photoisomerizable compound storage section 163 to the container 110. Alternatively, the photoisomerizable compound aqueous solution may be fed from the photoisomerizable compound storage section 163, and the polyether aqueous solution may be fed from the polyether storage section 162, mixed in the conduit 190, and fed into the container 110.

In the step S123, a detection reagent for detecting the reaction between the first component C1 and the second component C2 may be fed from the detection reagent storage section 166. Examples of the detection reagent include a substance that generates fluorescence, coloration, or the like by a reaction between the first component and the second component. The detection reagent fed into the container 110 generates fluorescence or coloration by the reaction between the first component C1 and the second component C2 encapsulated in the droplets D, thereby facilitating detection of the reaction between the first component C1 and the second component C2.

FIG. 15C shows a state in which the flow path 115 in the container 110 is filled with the polyether aqueous solution PE containing the photoisomerizable compound PI by the feeding of the photoisomerizable compound/polyether aqueous solution.

In a step S134, the monitoring unit 120 starts monitoring the droplets D in the container 110. The monitoring unit 120 monitors the reaction between the first component C1 and the second component C2 taken into the droplets D, and transmits information such as the reaction progress state to the processor 140. When the detection reagent is fed in the step S133, the monitoring unit 120 may detect fluorescence, coloration, or the like caused by the detection reagent. As a result, information on the reaction state of the first component C1 and the second component C2 may be obtained and transmitted to the processor 140.

In a step S135, the processor 140 identifies the droplets D in which the second component C2 that reacts with the first component C1 is contained, based on monitoring information transmitted from the monitoring unit 120. In addition, the processor 140 identifies the specific well 111S holding the droplets D.

In a step S136, the light irradiation unit 130 irradiates light to wells 111 other than a specific well 111S. A wavelength of the light irradiated at this time is a wavelength that causes the photoisomerizable compound to undergo photoisomerization.

FIG. 16A shows a state in which wells 111 other than the specific well 111S are irradiated with light. The droplets D encapsulating the second component C2 reacting with the first component C1 are held in the specific well 111S.

FIG. 16B shows a change of the photoisomerizable compound PI due to the light irradiation. The photoisomerizable compound PI at a position irradiated with light undergoes photoisomerization and becomes a photoisomerizable compound PI', which is an isomer of the photoisomerizable compound P. The photoisomerizable compound PI in and around the specific well 111S is not photoisomerized and remains the photoisomerizable compound PI.

In a step S137, the light irradiation unit 130 ends the light irradiation.

In a step S138, the polyether aqueous solution is fed from the polyether storage section 162 to the container 110. At this time, the valves B8 and B9 are controlled so that the container 110 is connected to the droplet collection unit 170.

FIG. 16C shows a state in which the droplets D encapsulating the second component C2 in the specific well 111S flow out from the specific well 111S by the feeding of the polyether aqueous solution PE. The droplets D flowing out from the specific well 111S are discharged from the discharge port 117 and collected in the droplet collection unit 170 through the conduit 190.

In a step S139, the monitoring unit 120 ends the monitoring.

In a step S140, the monitoring result is output through the output unit 150.

As a result, a series of operation flows is ended.

As the sample containing the second component, a compound library may be used.

Since the droplet control device according to the second embodiment has the above-described configuration, droplets containing a desired component can be collected even from wells having a minute volume on the order of picoliters or on the order of femtoliters. In the related art, it has been difficult to selectively collect specific droplets from such minute-volume wells. By using the photoisomerizable compound, the droplet control device according to the second embodiment can selectively collect specific droplets even from wells having a minute volume.

### [Droplet production method]

A second aspect of the present invention is a droplet production method. In one embodiment, a droplet container including a plurality of wells and a flow path communicating with the plurality of wells is used for the droplet production method. In one embodiment, the droplet production method includes the following (a), (b), and (c1) in this order, or includes the following (b), (a), and (c2) in this order:
(a) a step of feeding an aqueous solution containing a polysaccharide to the plurality of wells through the flow path;
(b) a step of feeding an aqueous solution containing a polyether to the plurality of wells through the flow path;
(c1) a step of feeding an aqueous solution containing the polyether and a photoisomerizable compound to the plurality of wells through the flow path; and
(c2) a step of feeding an aqueous solution containing the polysaccharide and a photoisomerizable compound to the plurality of wells through the flow path.

As the droplet container, the container 110 or the container 110' in the droplet control device 100 according to the second embodiment described above can be used.

In the case of the method including the steps (a), (b), and (c1) in this order, the steps (a), (b), and (c1) can be performed in the same manner as in the steps S101, S102, and S103 described above.

In the case of the method including the steps (b), (a), and (c2) in this order, the steps (b), (a), and (c2) can be performed in the same manner as in the steps S111, S112, and S113 described above.

In one embodiment, the droplet production method may further include the following step (d) after the step (c1) or (c2) described above.
(d) A step of irradiating wells other than a specific well with light having a wavelength that causes the photoisomerizable compound to undergo photoisomerization

The step (d) can be performed in the same manner as in the step S105 or step S115 described above.

In one embodiment, the droplet production method may include the following step (e1) or (e2) after the step (d) described above.
(e1) A step of feeding an aqueous solution containing a polyether to the plurality of wells through the flow path, and collecting droplets from the specific well
(e2) A step of feeding an aqueous solution containing a polysaccharide to the plurality of wells through the flow path, and collecting droplets from the specific well

The step (e1) can be performed in the same manner as in the step S107 described above.

The step (e2) can be performed in the same manner as in the step S117 described above.

With the droplet production method according to the present embodiment, stable droplets can be formed and maintained in wells having a minute volume on the order of picoliters or on the order of femtoliters. In addition, by controlling a light irradiation position, specific droplets can be selectively collected from arbitrary wells.

### [Separation device for target substance]

A third aspect of the present disclosure is a device for separating a target substance from a sample prepared in an aqueous medium containing a photoisomerizable compound. In one embodiment, the device for separating a target substance includes a container having a plurality of compartments for storing the above-described sample, a light irradiation unit configured to irradiate with light to the sample stored in the compartments, and a monitoring unit configured to monitor the sample stored in the compartments. The above-described light irradiation unit is configured to irradiate the sample with light having a wavelength that causes the photoisomerizable compound to undergo photoisomerization. The sample containing the photoisomerizable compound undergoes a sol-gel transition due to photoisomerization of the photoisomerizable compound.

### (Target substance)

The target substance is not particularly limited. Examples of the target substance include organic synthetic compounds such as low molecular weight compounds, fluorescent dyes, radical generators, and synthetic polymer compounds; biomolecules such as amino acids, nucleotides, sugars, vitamins, peptides, proteins, and nucleic acids; inorganic compounds such as inorganic nanoparticles, inorganic anions, inorganic cations, inorganic salts, and aggregates thereof; cells such as animal cells, plant cells, microorganisms and fungi, algae cells, and protozoa cells; extremely small structures such as phages, viruses, and molecular aggregates; and cell aggregates such as organoids and spheroids.

### (Sample)

The sample can be prepared by mixing the photoisomerizable compound with a test composition containing the target substance. Examples of the test composition containing the target substance include body fluids (blood, plasma, serum, saliva, tissue fluid, lymph, urine, pleural effusion, ascites, cerebrospinal fluid, bile, pancreatic juice, amniotic fluid, nasal discharge, tears, sweat, and the like), cell culture media, cell culture supernatants, cell lysates, cell homogenates, and compound libraries, without being limited thereto.

### (Photoisomerizable compound)

Examples of the photoisomerizable compound include the same as those described above. In one embodiment, the photoisomerizable compound is a compound in which the above-described sample undergoes a sol-gel transition due to photoisomerization of the photoisomerizable compound. Specific examples of the photoisomerizable compound include a compound (P) described later.

### (Aqueous medium)

Examples of the aqueous medium include the same as those described above.

### (Configuration example of separation device for target substance)

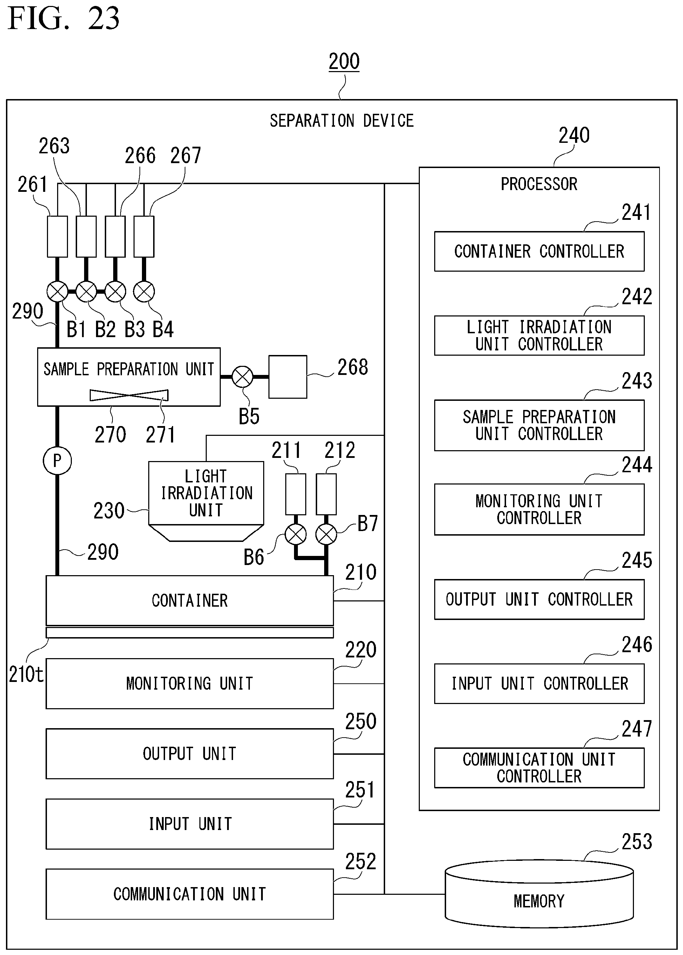

FIG. 23 is a schematic view showing an example configuration of the separation device for the target substance. A separation device 200 includes a container 210, a monitoring unit 220, a light irradiation unit 230, an output unit 250, an input unit 251, a communication unit 252, a memory 253, waste liquid storage sections 268 and 212, a sample preparation unit 270, a target substance collection unit 211, and a processor 240 configured to control operations of these components. In addition, the separation device 200 includes, as storage sections for various components, a test composition storage section 261, a photoisomerizable compound storage section 263, a detection reagent storage section 266, and an aqueous medium storage section 267.

In the separation device 200, components for sample preparation are supplied from the test composition storage section 261 and the photoisomerizable compound storage section 263 to the sample preparation unit 270. The sample preparation unit 270 mixes and stirs these components to produce the sample. The prepared sample is transferred to the container 210. The container 210 has a plurality of compartments for storing the sample. The sample transferred to the container 210 is distributed to each compartment of the container 210. In one embodiment, the sample transferred to the container 210 is in a sol state.

The light irradiation unit 30 irradiates the sample stored in each compartment of the container 210 with light. As a result, the photoisomerizable compound contained in the sample undergoes photoisomerization. The sample containing the photoisomerized photoisomerizable compound forms a gel.

### <<Container>>

The container 210 is not particularly limited as long as it is a container having a plurality of compartments. Examples of the plurality of compartments include a plurality of wells. Examples of the container 210 include the same ones as the container 110 of the droplet control device according to the second embodiment described above. Examples of the container 210 include a microwell plate (for example, a 96-well microplate) and a nanowell plate.

### <<Monitoring unit>>

The monitoring unit 220 is configured to monitor the state of the sample stored in each compartment of the container 210. The monitoring unit 20 is not particularly limited as long as it is capable of observing the state of the sample stored in each compartment. As the monitoring unit 220, a unit including the above-described microscope unit can be used.

### <<Light irradiation unit>>

The light irradiation unit 230 is configured to irradiate with light to the sample stored in each compartment in the container 210. As a light source of the light irradiation unit 230, the same light source as that of the light irradiation unit 130 in the droplet control device according to the second embodiment described above can be used.

### <<Sample preparation unit>>

The sample preparation unit 270 is configured to prepare the sample. The sample preparation unit 270 prepares the sample by mixing and stirring the test composition supplied from the test composition storage section 261 and the photoisomerizable compound aqueous solution supplied from the photoisomerizable compound storage section 263.

The sample preparation unit 270 includes a mixing tank for mixing and stirring each component, and a stirring mechanism 271 configured to stir the liquid in the mixing tank. The stirring mechanism 271 is not particularly limited, and examples thereof include a mechanism for rotating a stirrer having a rod shape, a plate shape, a propeller shape, or the like, and a mechanism for rotating stirring blades fixed inside the mixing tank, a mechanism for rotating the mixing tank itself.

The sample preparation unit 270 may include a temperature controller configured to control a temperature in the mixing tank. Examples of the temperature controller include a heater, a cooler, and a thermostat.

The waste liquid storage section 268 is connected to the sample preparation unit 270. The waste liquid storage section 268 stores, for example, waste liquid generated by cleaning the inside of the mixing tank after the sample produced in the mixing tank is transferred to the container 210. The cleaning of the inside of the mixing tank is performed using, for example, the aqueous medium supplied from the aqueous medium storage section 267. Connection and disconnection between the sample preparation unit 270 and the waste liquid storage section 268 are switched by a valve B5.

### <<Component storage sections>>

The test composition storage section 261 is configured to store the test composition. The test composition stored in the test composition storage section 261 is preferably in a liquid state. The test composition may be a solution or a suspension.

The test composition storage section 261 is connected to the sample preparation unit 270 through a conduit 290. Connection and disconnection between the test composition storage section 261 and the sample preparation unit 270 are switched by a valve B1.

The photoisomerizable compound storage section 263 is configured to store the photoisomerizable compound aqueous solution. The concentration of the photoisomerizable compound aqueous solution stored in the photoisomerizable compound storage section 263 is preferably 0.3% by mass or more. In the sample prepared by the sample preparation unit 270, the concentration of the photoisomerizable compound is, for example, 0.1% to 10% by mass with respect to the total mass of the sample. The concentration of the photoisomerizable compound aqueous solution stored in the photoisomerizable compound storage section 263 may be any concentration as long as the concentration of the photoisomerizable compound can be adjusted to fall within the above-described concentration range when the sample is prepared. The photoisomerizable compound aqueous solution stored in the photoisomerizable compound storage section 263 may be a concentrated solution, and in this case, the aqueous medium may be supplied from the aqueous medium storage section 267 for dilution.

The photoisomerizable compound storage section 263 is connected to the sample preparation unit 270 through the conduit 290. Connection and disconnection between the photoisomerizable compound storage section 263 and the sample preparation unit 270 are switched by valves B1 and B2.

The photoisomerizable compound stored in the photoisomerizable compound storage section 263 is preferably stored in an isomeric form that forms a sol state. For example, for a photoisomerizable compound in which the trans form is in a gel state and the cis form is in a sol state, the cis form is preferably stored in the photoisomerizable compound storage section 263. In the case of the compound (P) described later, it is preferable that the cis form of the compound (P) is stored in the photoisomerizable compound storage section 263.

The detection reagent storage section 266 is configured to store a detection reagent aqueous solution. The detection reagent is a reagent capable of specifically detecting the target substance. The detection reagent is supplied to the sample preparation unit 270 during sample preparation. Examples of the detection reagent include a reagent containing a specific binding substance (an antibody, an aptamer, and the like) that specifically binds to the target substance. The detection reagent may be one in which a labeling substance (fluorescent dye and the like) is bound to the specific binding substance for the target substance. Examples of the fluorescent dye include the same ones as described above.

The detection reagent storage section 266 is connected to the sample preparation unit 270 through the conduit 290. Connection and disconnection between the detection reagent storage section 266 and the sample preparation unit 270 are switched by valves B1, B2, and B3.

The aqueous medium storage section 267 is configured to store the aqueous medium. The aqueous medium is used for dilution during sample preparation, cleaning of the mixing tank of the sample preparation unit 270 after sample preparation, and the like.

The aqueous medium storage section 267 is connected to the sample preparation unit 270 through the conduit 290. Connection and disconnection between the aqueous medium storage section 267 and the sample preparation unit 270 are switched by valves B1, B2, B3, and B4.

As the output unit 250, the input unit 251, and the communication unit 252, the same units as those in the above-described droplet control device can be used.

### <<Processor>>

The processor 240 controls an operation of the separation device 200. The operation of each unit of the separation device 200 described above is controlled by the processor 240. The processor 240 implements such control by executing, for example, a program stored in the memory 253. The hardware configuration of the processor 240 may be the same as that of the droplet control device described above.

The processor 240 includes a container controller 241, a light irradiation unit controller 242, a sample preparation unit controller 243, a monitoring unit controller 244, an output unit controller 245, an input unit controller 246, and a communication unit controller 247, and has a function of controlling operations of the above-described units.

The container controller 241 has a function of controlling the container 210. The container controller 241 may have a function of controlling the temperature controller 210t to control the temperature of the internal space of the container 210.

The light irradiation unit controller 242 has a function of controlling the light irradiation unit 230. The light irradiation unit controller 242 may have a function of controlling, for example, at least one operation selected from the group consisting of a timing of starting light irradiation, a light irradiation time, a wavelength of irradiation light, and a light irradiation position in the light irradiation unit 230. The light irradiation unit controller 242 may have a function of controlling the photomask. For example, the light irradiation unit controller 242 may have a function of selecting a compartment for blocking irradiation light based on monitoring information of the monitoring unit 220, and disposing the photomask at the position of the specific compartment. Alternatively, the light irradiation unit controller 242 may have a function of controlling the laser device provided in the light irradiation unit 230 to irradiate the compartment other than the specific compartment with the laser light.

The sample preparation unit controller 243 has a function of controlling the sample preparation unit 270. The sample preparation unit controller 243 may have a function of controlling supply of each component from the test composition storage section 261, the photoisomerizable compound storage section 263, the detection reagent storage section 266, and the aqueous medium storage section 267 to the sample preparation unit 270. For example, the sample preparation unit controller 243 may control supply of these components to the sample preparation unit 270 by controlling the valves B1 to B4.

The sample preparation unit controller 243 may have a function of controlling the stirring mechanism 271 of the sample preparation unit 270. For example, the sample preparation unit controller 243 may have a function of controlling operation timing, stirring time, stirring intensity, and the like of the stirring mechanism 271.

The sample preparation unit controller 243 may have a function of controlling transfer of the sample from the sample preparation unit 270 to the container 210. For example, the sample preparation unit controller 243 may control transfer of the sample from the sample preparation unit 270 to the container 210 by controlling a pump P.

The sample preparation unit controller 243 may have a function of controlling cleaning of the mixing tank of the sample preparation unit 270. For example, the sample preparation unit controller 243 may have a function of controlling timing of supply of the aqueous medium from the aqueous medium storage section 267 to the sample preparation unit 270, and discharge of waste liquid to the waste liquid storage section 268. For example, the sample preparation unit controller 243 may control cleaning of the sample preparation unit 270 by controlling the valves B1 to B5.

The sample preparation unit controller 243 may have a function of controlling the temperature in the mixing tank of the sample preparation unit 270.

The monitoring unit controller 244 has a function of controlling the monitoring unit 220. The monitoring unit controller 244 may have a function of controlling, for example, at least one operation selected from the group consisting of a timing of starting monitoring, a monitoring time, and a monitoring position in the monitoring unit 220.

The output unit controller 245, the input unit controller 246, and the communication unit controller 247 are the same as those in the droplet control device.

### (Operation example of separation device)

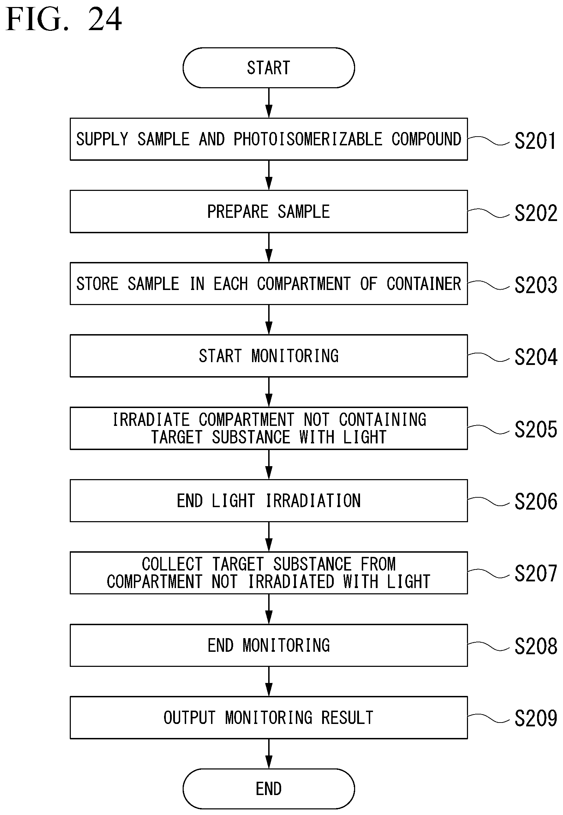

FIG. 24 is a flowchart showing an operation example of the separation device 200.

In a step S201, the test composition and the photoisomerizable compound are each supplied from the test composition storage section 261 and the photoisomerizable compound storage section 263 to the sample preparation unit 270. As necessary, the detection reagent may be supplied from the detection reagent storage section 266 to the sample preparation unit 270. Alternatively, as necessary, the aqueous medium may be supplied from the aqueous medium storage section 267 to the sample preparation unit 270.

The supply amount of each component may be set in advance, or may be set by the user through the input unit 251.

The supply of each component to the sample preparation unit 270 may be performed by a pump (not shown) or the like.

In a step S202, the mixed liquid of the components supplied to the mixing tank of the sample preparation unit 270 is mixed and stirred to produce the sample. A stirring time may be set in advance, or may be set by the user through the input unit 251. Alternatively, the user may be allowed to manually stop the stirring through the input unit 251.

In a step S203, the sample produced in the step S202 is stored in each compartment of the container 210. The transfer of the sample from the sample preparation unit 270 to the container 210 is performed by controlling the pump P. The entire amount of the sample may be transferred to the container 210, or a part thereof may be transferred to the container 210 and the rest may be stored in the waste liquid storage section 268.

In a step S204, the monitoring unit 220 starts monitoring the sample stored in each compartment of the container 210. The state of monitoring may be displayed on a display or the like, which is the output unit 250. In addition, monitoring images or videos may be recorded in the memory 253 or an external storage device from time to time.

In a step S205, the light irradiation unit 230 irradiates light to compartments not containing the target substance in the container 210. A wavelength of the light irradiated at this time is a wavelength that causes the photoisomerizable compound to undergo photoisomerization. The light irradiation time may be any time sufficient for the photoisomerizable compound to undergo the photoisomerization. Examples of the light irradiation time include the same times as those of the droplet control device described above.

The compartments to be irradiated with light are selected based on monitoring information of the monitoring unit 220. For example, the monitoring unit 220 detects a compartment containing the target substance based on a signal of the detection reagent, or the like, and selects a compartment not containing the target substance as a compartment to be irradiated with light. In the compartment irradiated with light, the sample undergoes gelation due to photoisomerization of the photoisomerizable compound.

In a step S206, the light irradiation unit 230 ends the light irradiation.

In a step S207, the aqueous medium is fed from the aqueous medium storage section 267 to the container 210 through the sample preparation unit 270. The feeding of the aqueous medium is performed by the sample preparation unit controller 243 controlling the valves B1 to B4, and the container controller 241 controlling the pump P and the valves B6 and B7. At this time, the valves B6 and B7 are controlled so that the container 210 is connected to the target substance collection unit 211.

Since the compartment not containing the target substance is not irradiated with light in the step S206, the sample is in a sol state. Therefore, the target substance can be flowed out from the compartment by feeding the aqueous medium. On the other hand, since the sample in the compartment irradiated with light in the step 206 is in a gel state, the sample does not flow out from the compartment even when the aqueous medium is fed. Accordingly, the target substance can be selectively collected. The feeding of the aqueous medium is performed until collection of the target substance from the compartment containing the target substance is completed.

In a step S208, the monitoring unit 220 ends the monitoring.

In a step S209, the monitoring result is output through the output unit 250.

As a result, a series of operation flows is ended.

In the step S205, light irradiation may be performed on the compartment containing the target substance. In this case, in the step S207, substances other than the target substance are discharged from the compartment not irradiated with light to the waste liquid storage section 268 by feeding the aqueous medium to the container 210. At this time, the valves B6 and B7 are controlled so that the container 210 is connected to the waste liquid storage section 268. The target substance can be fixed and collected in the container 210 by the gelation of the photoisomerizable compound.

### [Separation method for target substance]

A fourth aspect of the present invention is a method for separating a target substance from a sample prepared in an aqueous medium containing a photoisomerizable compound. In one embodiment, the method for separating a target substance includes the following (A) to (C) in this order:
(A) a step of distributing the sample prepared in the aqueous medium in a sol state, containing the photoisomerizable compound, into a plurality of compartments;
(B) a step of irradiating either (i) a compartment containing the target substance or (ii) a compartment not containing the target substance with light having a wavelength that causes the photoisomerizable compound to undergo photoisomerization, to gel the sample stored in the compartments irradiated with the light; and
(C) a step of taking out the sample from the compartments not irradiated with the light.

As the container including a plurality of compartments, the container 210 in the separation device 200 described above can be used.

The steps (A), (B), and (C) can be performed in the same manner as the step S203, the steps S204 to S206, and the step S207 described above.

### [Composition]

A third aspect of the present invention is a composition stored in a light-shielding container. In one embodiment, the composition contains a saccharide, a polyether, a photoisomerizable compound, a first droplet in which a first component is encapsulated, and a second droplet in which a second component is encapsulated.

In one embodiment, the first component and the second component are components that react with each other. The first component and the second component may be, for example, components that react with each other to cure. For example, the first component is an isocyanate compound having an isocyanate group, and the second component is a compound having a functional group reactive with the isocyanate group (for example, an epoxy group, an amino group, a carboxy group, and the like).

Examples of a method for producing the first droplet and the second droplet include the same method as described in the droplet control device according to the first embodiment above.

In one embodiment, the photoisomerizable compound contained in the above-described composition is an isomer that forms a gel state. For example, when a trans form thereof forms a gel state, the above-described composition contains the trans form of the photoisomerizable compound. When the photoisomerizable compound is the compound (P), the above-described composition contains the trans form of the compound (P).

The light-shielding container is not particularly limited as long as it is a container formed of a material that blocks light. Examples of the light-shielding container include a metallic tube and a resin tube containing a light shielding agent. The light-shielding container preferably includes an openable and closable mechanism such as a lid.

The composition according to the present embodiment can be used for, for example, a two-component adhesive, a two-component coating material, a two-component resin, and the like.

In one embodiment, the above-described composition may be a multicomponent formulation stored in an openable and closable light-shielding container.

### <Compound (P)>

Examples of the photoisomerizable compound include a compound (compound (P)) including a peptide structure represented by General Formula (p1) or (p2).

XaXb(XaXc)ₙXaXdXa (p1)

XaXd(XaXc)ₙXaXbXa (p2)

[in the formulae, Xa represents an amino acid residue having a hydrophilic side chain, Xb represents an amino acid residue having a side chain represented by General Formula (b1), Xc and Xd each independently represent an amino acid residue having a hydrophobic side chain, or a glycine residue, and n represents an integer of 1 to 3, provided that a plurality of Xa's may be the same or different from each other, and when n is 2 or 3, a plurality of Xc's may be the same or different from each other]
[Chemical Formula 4]

*-Ar²-N=N-Ar¹ (b1)

[in the formula, Ar¹ represents an aryl group which may have a substituent or a heteroaryl group which may have a substituent, Ar² represents an arylene group which may have a substituent or a heteroarylene group which may have a substituent, and * represents a bonding site]

The term "peptide structure" refers to a structure in which amino acids are bonded by amide bonds to form a polymer. A compound including a peptide structure may be a peptide, or a compound including a chemical structure other than a peptide. When referring to a specific peptide structure, the specific peptide structure may be a partial structure included in a single peptide molecule.

In Formula (p1) or (p2), Xa represents an amino acid residue having a hydrophilic side chain. The hydrophilic side chain is an amino acid side chain having a hydrophilic group. Xa is preferably an amino acid residue derived from an α-amino acid. Specific examples of Xa include arginine residue, asparagine residue, aspartic acid residue, glutamic acid residue, glutamine residue, lysine residue, serine residue, threonine residue, cysteine residue, histidine residue, ornithine residue, and the like. In Formula (p1) or (p2), the plurality of Xa's may each be identical or different.

In Formula (p1) or (p2), Xb represents an amino acid residue having a side chain represented by Formula (b1) (also referred to below as "side chain (b1)"). In Formula (b1), * is a bond bonded to a carbon atom constituting the main chain of an amino acid polymer. Xb is preferably an amino acid residue derived from an α-amino acid. When Xb is an amino acid residue derived from an α-amino acid, the α-amino acid from which Xb is derived is represented by Formula (Xb-1). 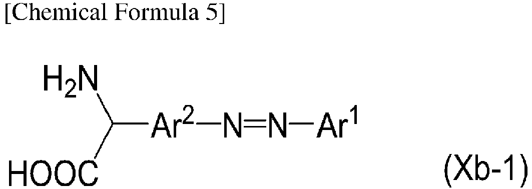 [in the formula, Ar¹ and Ar² are the same as Ar¹ and Ar² in Formula (b1) described above]

In Formula (b1), Ar¹ represents an aryl group which may have a substituent or a heteroaryl group which may have a substituent. The aryl group in Ar¹ preferably has 6 to 18 carbon atoms, more preferably has 6 to 15 carbon atoms, and still more preferably has 6 to 12 carbon atoms. The aryl group may be a monocyclic group or a polycyclic group. The number of aromatic hydrocarbon rings included in the aryl group is preferably 1 to 3, more preferably 1 or 2, and still more preferably 1. Specific examples of the aryl group include a phenyl group, a naphthyl group, an anthrenyl group, a phenanthrenyl group, and the like.

The heteroaryl group in Ar¹ preferably has 1 to 18 carbon atoms, more preferably 2 to 15 carbon atoms, and still more preferably 3 to 12 carbon atoms. For the heteroaryl group, examples of a hetero atom included in an aromatic heterocycle include an oxygen atom, a nitrogen atom, or a sulfur atom. The heteroaryl group may be a monocyclic group or a polycyclic group. The heteroaryl group may be a monocyclic group of an aromatic heterocycle, a condensed cyclic group of an aromatic hydrocarbon ring and an aromatic heterocycle, or a condensed ring of aromatic heterocycles. The total number of aromatic heterocycles and aromatic hydrocarbon rings included in the heteroaryl group is preferably 1 to 3, more preferably 1 or 2, and still more preferably 1.

Examples of aromatic heterocycles included in heteroaryl groups include nitrogen-containing aromatic heterocycles (pyrrole rings, pyridine rings, azepine rings, azocine rings, azonine rings, imidazole rings, pyrazole rings, imidazoline rings, triazole rings, tetrazole rings, pyridazine rings, pyrimidine rings, pyrazine rings, and the like); condensed rings of nitrogen-containing aromatic heterocycles and benzene rings (indole rings, isoindole rings, benzimidazole rings, purine rings, benzotriazole rings, quinoline rings, isoquinoline rings, quinazoline rings, quinoxaline rings, cinnoline rings, and the like); sulfur-containing aromatic heterocycles (thiol rings, thiepine rings, thionine rings, and the like); aromatic heterocycles including nitrogen atoms and sulfur atoms (thiazole rings, thiazine rings, and the like); oxygen-containing aromatic heterocycles (oxole rings, oxepin rings, oxonine rings, and the like); aromatic heterocycles including nitrogen atoms and oxygen atoms (oxazole rings), and the like, without being limited thereto.

The aryl group or heteroaryl group in Ar¹ may or may not have a substituent. The substituent is not particularly limited, but is preferably a hydrophobic group. Examples of the substituent include an alkyl group, a halogenated alkyl group, an alkoxy group, a halogenated alkoxy group, a halogen atom, and the like. As the substituent, the alkyl group and alkoxy group preferably have 1 to 12 carbon atoms, more preferably have 1 to 10 carbon atoms, still more preferably have 1 to 6 carbon atoms, and particularly preferably have 1 to 3 carbon atoms. Examples of the halogen atom in the halogenated alkyl group and halogenated alkoxy group and the halogen atom as the substituent include a fluorine atom, a bromine atom, and an iodine atom, and a fluorine atom is preferable.

Ar¹ is preferably an aryl group which may have a substituent, more preferably a phenyl group or naphthyl group which may have a substituent, still more preferably a phenyl group which may have a substituent, and particularly preferably a perfluorophenyl group (pentafluorophenyl group) or a phenyl group.

In Formula (b1), Ar² represents an arylene group which may have a substituent or a heteroarylene group which may have a substituent. The arylene group in Ar² preferably has 6 to 18 carbon atoms, more preferably has 6 to 15 carbon atoms, and still more preferably has 6 to 12 carbon atoms. The arylene group may be a monocyclic group or a polycyclic group. The number of aromatic hydrocarbon rings included in the arylene group is preferably 1 to 3, more preferably 1 or 2, and still more preferably 1. Specific examples of the arylene group include a phenylene group, a naphthylene group, an anthracenediyl group, a phenanthrenediyl group, and the like.

The heteroarylene group in Ar² preferably has 1 to 18 carbon atoms, more preferably has 2 to 15 carbon atoms, and still more preferably has 3 to 12 carbon atoms. Examples of the heteroarylene group include, as the hetero atom included in the aromatic heterocycle, an oxygen atom, a nitrogen atom, and a sulfur atom. The heteroarylene group may be a monocyclic group or a polycyclic group. The heteroarylene group may be a monocyclic group of an aromatic heterocycle, a condensed cyclic group of an aromatic hydrocarbon ring and an aromatic heterocycle, or a condensed ring of aromatic heterocycles. The total number of aromatic heterocycles and aromatic hydrocarbon rings included in the heteroaryl group is preferably 1 to 3, more preferably 1 or 2, and still more preferably 1.

Examples of the aromatic heterocycle included in the heteroarylene group are the same as for the heteroaryl group in Ar¹ described above.

The arylene group or heteroarylene group in Ar² may or may not have a substituent. The substituent is not particularly limited, but is preferably a hydrophobic group. Examples of the substituent are the same as for the substituent in Ar¹ described above.

Ar² is preferably an arylene group which may have a substituent, more preferably a phenylene group or naphthylene group which may have a substituent, and still more preferably a phenylene group.

The side chain (b1) is preferably a side chain represented by General Formula (b1-1). 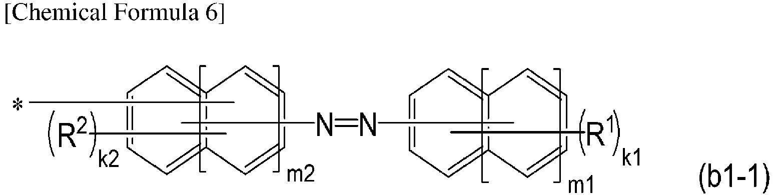 [in the formula, m1 and m2 each independently represent an integer of 0 to 3; R¹ and R² each independently represent a substituent; k1 and k2 each independently represent an integer of 0 or more as allowed by an atomic valence, when k1 is an integer of 2 or more, two or more R¹'s may be the same or different from each other, when k2 is an integer of 2 or more, two or more R²'s may be the same or different from each other; and * represents a bonding site]

In Formula (b1-1), m1 and m2 each independently represent an integer of 0 to 3. m1 and 2 are preferably 0 to 2, more preferably 0 or 1, and still more preferably 0.

In Formula (b1-1), R¹ and R² each independently represent a substituent. The substituent is not particularly limited, but is preferably a hydrophobic group. Specific examples of the substituent include the same examples as for the substituent in Ar¹ in Formula (b1). R¹ and R² are preferably halogen atoms and more preferably fluorine atoms.

When m1 is 0, k1 is an integer of 0 to 5. When m1 is 1, k1 is an integer of 0 to 7. When m1 is 2, k1 is an integer of 0 to 9. When m1 is 3, k1 is an integer of 0 to 11. m1 is preferably 0, and k1 is preferably 0 or 5. When m2 is 0, k2 is an integer of 0 to 4. When m2 is 1, k2 is an integer of 0 to 6. When m2 is 2, k2 is an integer of 0 to 8. When m2 is 3, k2 is an integer of 0 to 10. m2 is preferably 0, and k1 is preferably 0.

Specific examples of the side chain (b1) are shown below, without being limited thereto. In the following formula, * represents a bond bonded to a carbon atom constituting the main chain of the amino acid polymer. The following specific examples show the trans form, but the cis form is also acceptable.

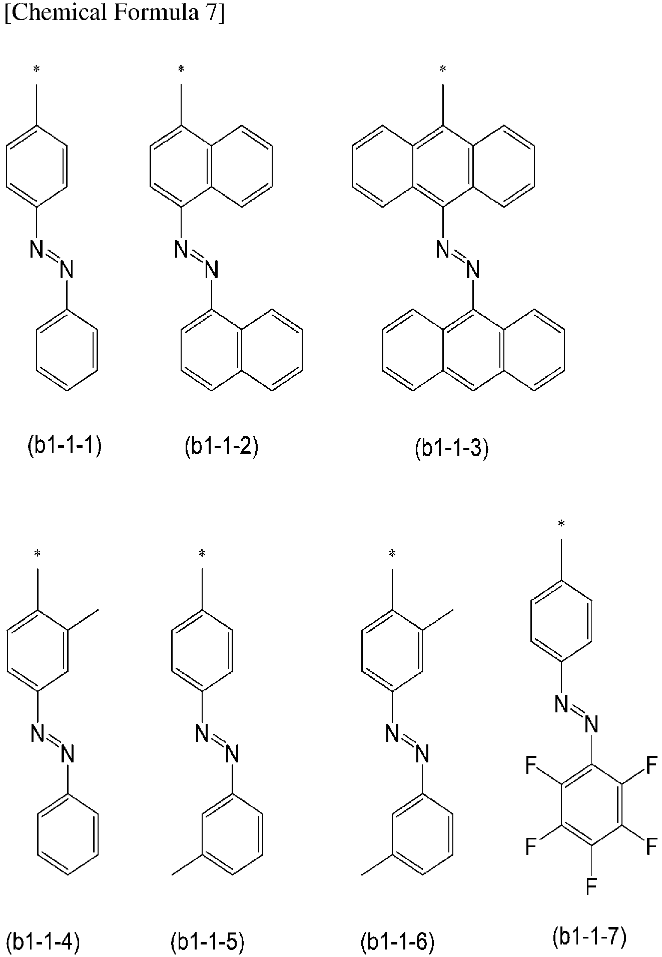

In Formula (p1) or (p2), Xc and Xd represent an amino acid residue having a hydrophobic side chain or a glycine residue. As the amino acid residue having a hydrophobic side chain, an amino acid residue derived from an α-amino acid is preferable. Specific examples of amino acid residues having a hydrophobic side chain include an alanine residue, a valine residue, a leucine residue, an isoleucine residue, a methionine residue, a proline residue, a phenylalanine residue, a tryptophan residue, and a tyrosine residue.

From the viewpoint of the solubility of the compound (P) in water, Xc is preferably an alanine residue or a glycine residue. When n in Formula (p1) or (p2) is 2 or 3, the plurality of Xc's may each be identical or different.

Xd is preferably an amino acid residue having a hydrophobic side chain having 3 or more carbon atoms. The hydrophobic side chain having 3 or more carbon atoms preferably has 3 to 12 carbon atoms, more preferably 3 to 10 carbon atoms, and still more preferably 3 to 8 carbon atoms. Specific examples of Xd include valine residue, leucine residue, isoleucine residue, methionine residue, proline residue, phenylalanine residue, tryptophan residue, tyrosine residue, and the like.

In Formula (p1) or (p2), n represents an integer of 1 to 3, preferably 1 or 2, and more preferably 1.

The peptide structure (p) is preferably a peptide structure represented by General Formula (p1-1) or (p2-1). 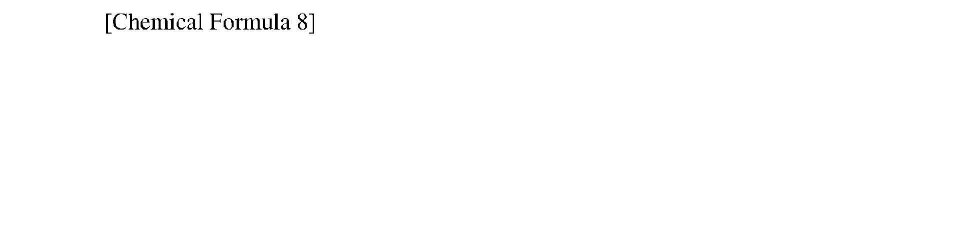 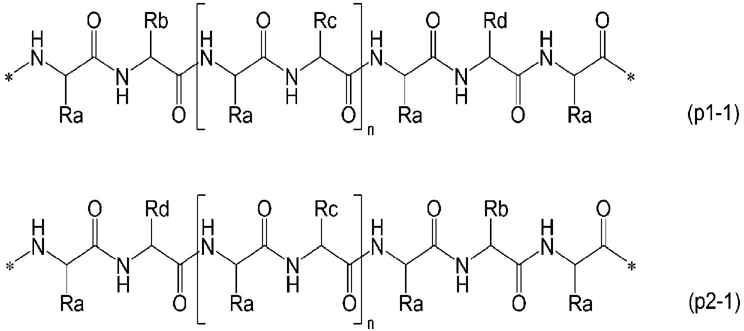 [in the formula, Ra represents a hydrophilic side chain; Rb represents a side chain represented by General Formula (b1); Rc and Rd each independently represent a hydrophobic side chain or a hydrogen atom; n represents an integer of 1 to 3, provided that a plurality of Ra's may be the same or different from each other, and when n is 2 or 3, a plurality of Rc's may be the same or different from each other; and * represents a bonding site]

In Formula (p1-1) or (p2-1), Ra represents a hydrophilic side chain. Examples of the hydrophilic side chain include an aliphatic hydrocarbon group having a hydrophilic group. Examples of the hydrophilic group include an amino group (preferably a primary amino group), a carboxy group, a hydroxyl group (preferably an alcoholic hydroxyl group), a thiol group, and the like. The aliphatic hydrocarbon group may be a saturated aliphatic hydrocarbon group or an unsaturated aliphatic hydrocarbon group, but is preferably a saturated aliphatic hydrocarbon group. The aliphatic hydrocarbon group may be linear, branched, or cyclic, but is preferably linear or branched, and is preferably linear. The aliphatic hydrocarbon group preferably has 1 to 6 carbon atoms, more preferably 1 to 4 carbon atoms, and still more preferably 1 to 3 carbon atoms.

Examples of the hydrophilic side chain in Ra include the side chain of a hydrophilic amino acid. Specific examples of hydrophilic amino acids include arginine, asparagine, aspartic acid, glutamine, glutamic acid, lysine, serine, threonine, cysteine, histidine, ornithine, and the like. Specific examples of Ra are shown below, without being limited thereto. In Formula (p1-1) or (p2-1), a plurality of Ra's may each be identical or different. In the following formula, * represents a bond bonded to a carbon atom constituting the main chain of the amino acid polymer in Formula (p1-1) or (p2-1). The symbol in parentheses represents the derivation source amino acid.

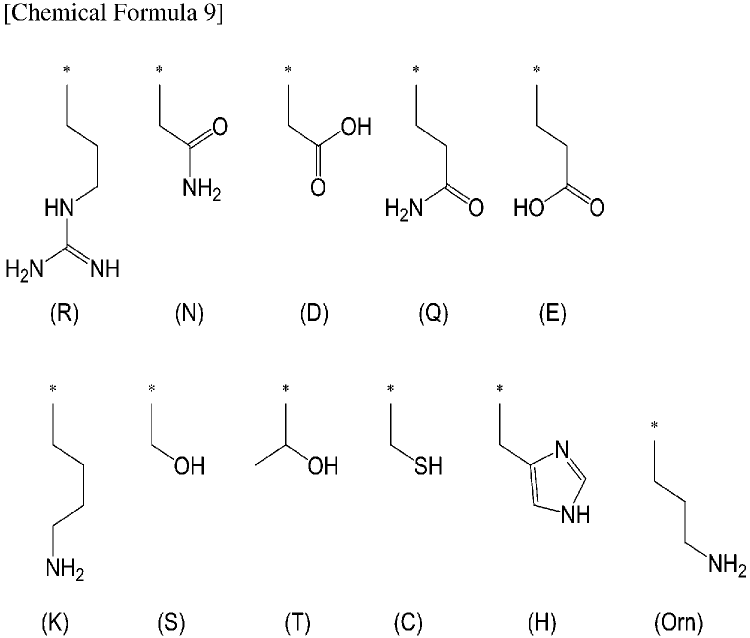

In Formula (p1-1) or (p2-1), Rb represents a side chain represented by Formula (b1). Rb is preferably a side chain represented by Formula (b1-1) and more preferably a side chain represented by any of Formulas (b1-1-1) to (b1-1-7).

In Formula (p1-1) or (p2-1), Rc and Rd represent a hydrophobic side chain or a hydrogen atom. Examples of the hydrophobic side chain include a hydrocarbon group having one or more carbon atoms, which may have a substituent. The hydrocarbon group preferably has 1 to 12 carbon atoms, more preferably 1 to 10 carbon atoms, and still more preferably 1 to 8 carbon atoms. The hydrocarbon group may be an aromatic hydrocarbon group or an aliphatic hydrocarbon group. The aliphatic hydrocarbon group may be a saturated aliphatic hydrocarbon group or an unsaturated aliphatic hydrocarbon group, but is preferably a saturated aliphatic hydrocarbon group. The aliphatic hydrocarbon group may be linear, branched, or cyclic. The hydrocarbon group may have a substituent as long as the substituent is not hydrophilic. The hydrophobic side chain in Rc and Rd may be a side chain of a hydrophobic amino acid. Examples of the hydrophobic amino acid include the same examples as for Xc and Xd.

Rc is preferably a hydrogen atom or a methyl group from the viewpoint of the solubility of the compound (P) in water. When n in Formula (p1-1) or (p2-1) is 2 or 3, the plurality of Rc's may each be identical or different.

Rd is preferably a hydrophobic side chain having 3 or more carbon atoms. Examples of hydrophobic side chains having 3 or more carbon atoms include hydrocarbon groups having 3 or more carbon atoms which may have a substituent. The hydrocarbon group preferably has 3 to 12 carbon atoms, more preferably has 3 to 10 carbon atoms, and still more preferably has 3 to 8 carbon atoms. The hydrocarbon group may be an aromatic hydrocarbon group or an aliphatic hydrocarbon group. The aliphatic hydrocarbon group may be a saturated aliphatic hydrocarbon group or an unsaturated aliphatic hydrocarbon group, but is preferably a saturated aliphatic hydrocarbon group. The aliphatic hydrocarbon group may be linear, branched, or cyclic. The hydrocarbon group may have a substituent as long as the substituent is not hydrophilic. Examples of the substituent that the hydrocarbon group in Rd may have include a secondary amino group, a tertiary amino group, a sulfide group, a phenolic hydroxyl group, and the like.

Examples of the hydrophobic side chain having 3 or more carbon atoms include side chains of hydrophobic amino acids having 3 or more carbon atoms. Specific examples of such hydrophobic amino acids include valine, leucine, isoleucine, methionine, proline, phenylalanine, tryptophan, tyrosine, and the like. Specific examples of Rd are shown below, without being limited thereto. When n in Formula (p1-1) or (p2-1) is 2 or 3, the plurality of Rd's may each be identical or different. In the following formula, * represents a bonding site bonded to a carbon atom constituting the main chain of the amino acid polymer in Formula (p1-1) or (p2-1). The symbol in parentheses represents the derivation source amino acid.

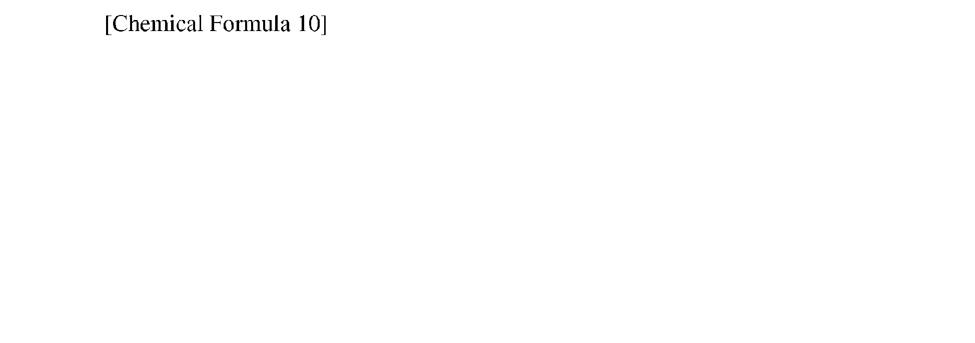

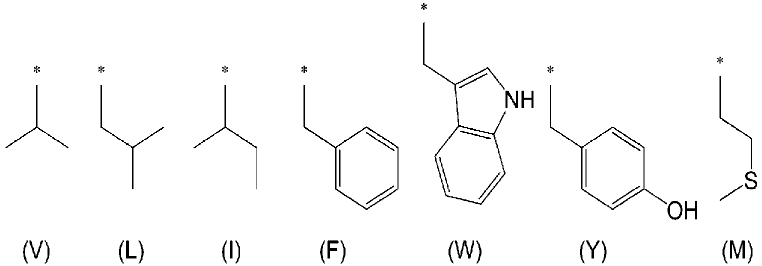

When Rd is a proline side chain, Rd has the following structure together with the carbon atom to which Rd is bonded and the NH group adjacent to this carbon atom in Formula (p1-1) or Formula (p2-1).

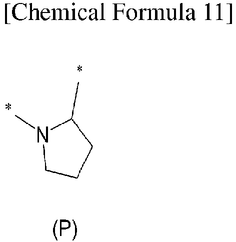

In Formula (p1-1) or (p2-1), n represents an integer of 1 to 3, preferably 1 or 2, and more preferably 1.

In Formula (p1-1) or (p2-1), examples of combinations of Ra, Rb, Rc, and Rd include combinations in which Ra is a side chain of an amino acid selected from the group consisting of arginine, asparagine, aspartic acid, glutamine, glutamic acid, lysine, serine, threonine, cysteine, histidine, and ornithine; Rb is a side chain represented by Formula (b1-1); Rc is a hydrogen atom or a methyl group; and Rd is a side chain of an amino acid selected from the group consisting of valine, leucine, isoleucine, methionine, proline, phenylalanine, tryptophan, and tyrosine. In the above-described combination, n in Formula (p1-1) or (p2-1) is preferably 1 or 2 and more preferably 1.

In Formula (p1-1) or (p2-1), the combination of Ra, Rb, Rc, and Rd is preferably a combination in which Ra is a side chain of arginine or aspartic acid; Rb is a side chain represented by Formula (b1-1); Rc is a hydrogen atom or a methyl group; and Rd is a side chain of methionine. In the above-described combination, n in Formula (p1-1) or (p2-1) is preferably 1 or 2 and more preferably 1.

Specific examples of the peptide structure (p) are shown below, without being limited thereto. In the following specific examples, the side chain (b1) is shown in the trans form, but the side chain (b1) may be in the cis form. * is a bonding site bonded to an adjacent atom.

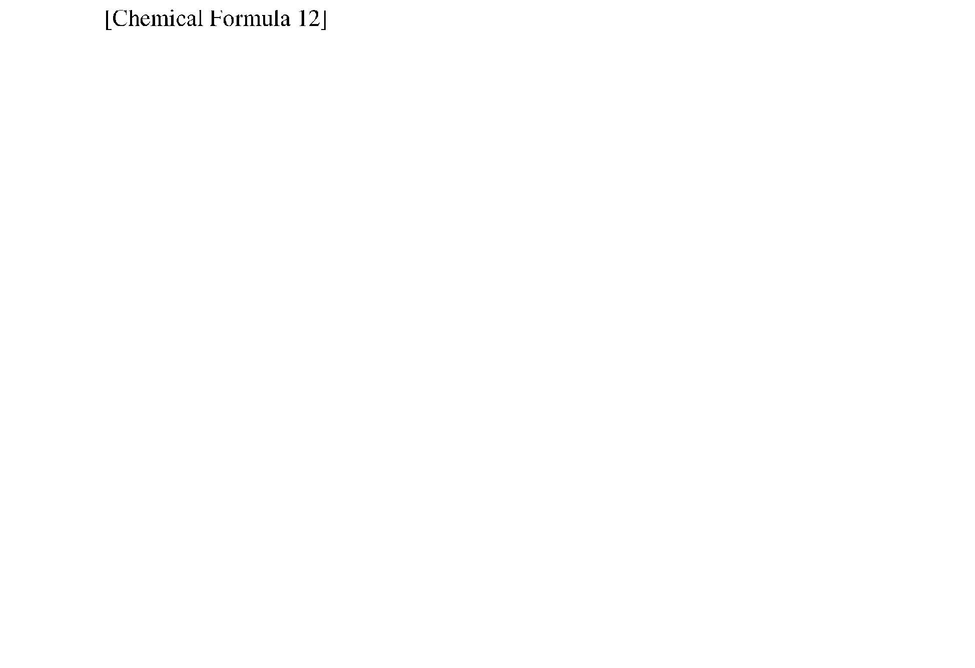

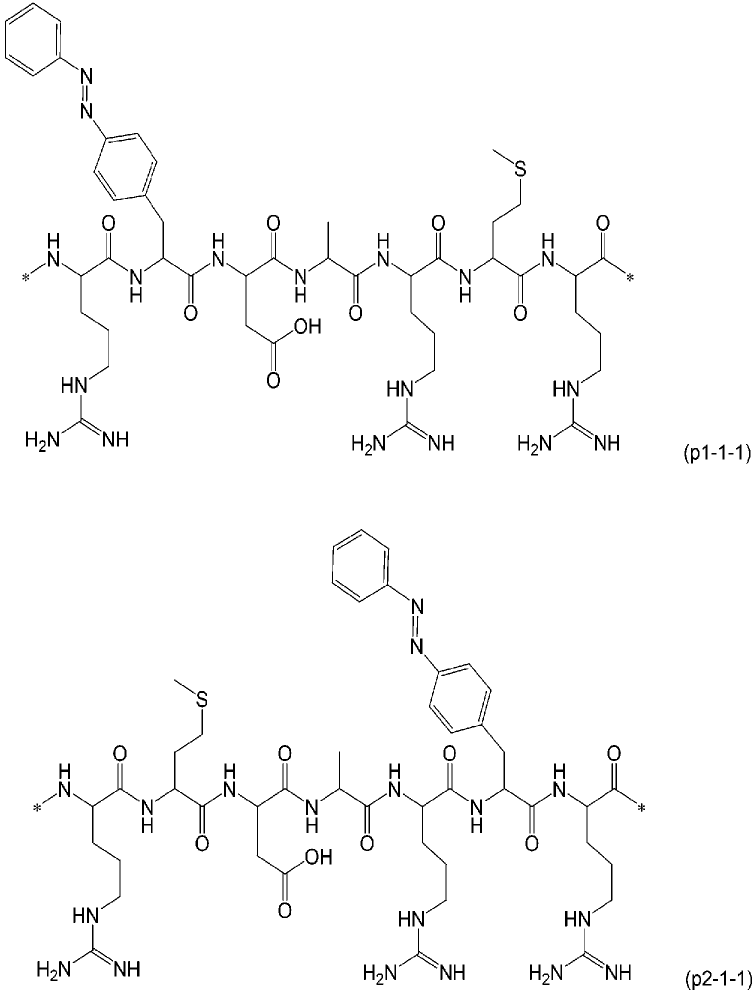

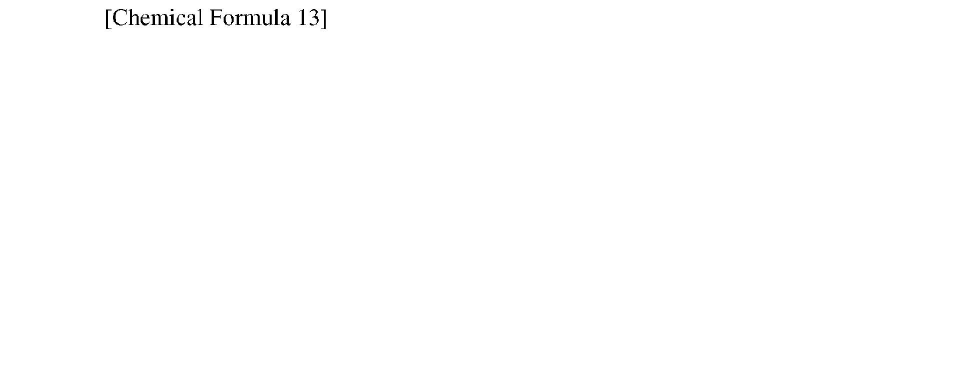

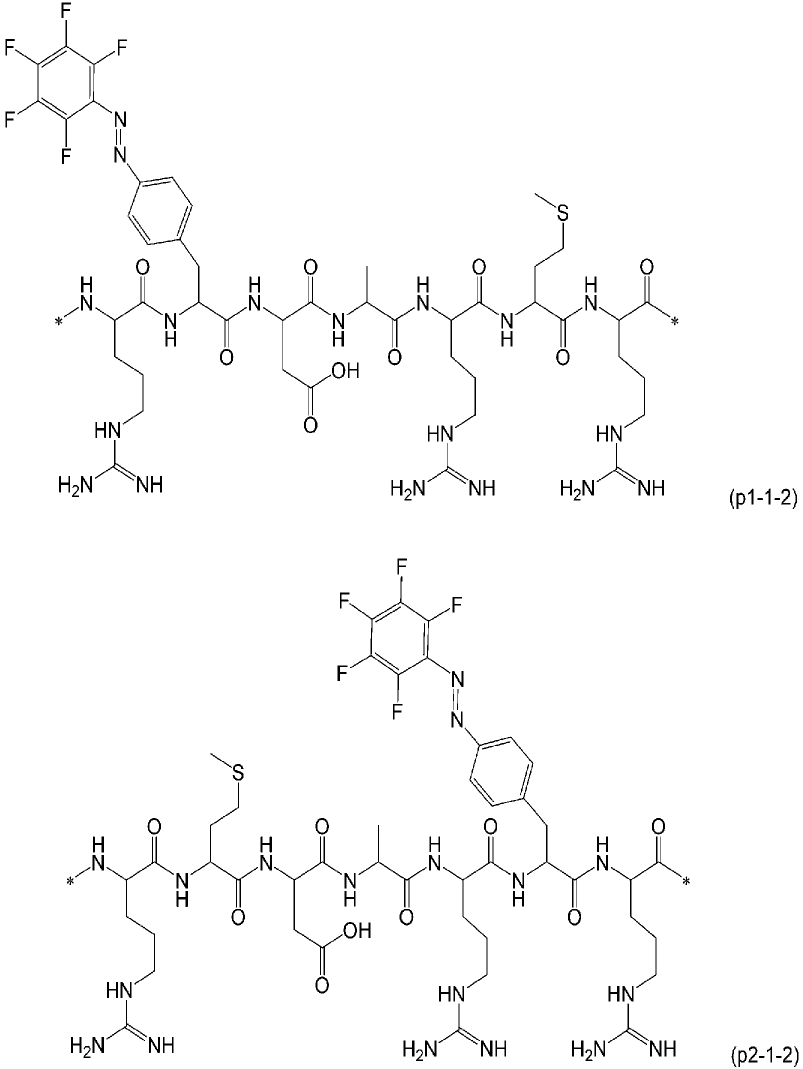

In the compound (P), structures other than the peptide structure (p) are not particularly limited. The compound (P) may be, for example, a peptide including the peptide structure (p). In this case, the number of amino acid residues of the compound (P) is not particularly limited and examples thereof include 40 or less, 30 or less, 25 or less, 20 or less, 15 or less, or 10 or less. The number of amino acid residues includes the number of amino acid residues of the peptide structure (p). When the compound (P) is a peptide including the peptide structure (p), the type of the amino acid residues in the region other than the peptide structure (p) is not particularly limited. The portion of the compound (P) other than the peptide structure (p) does not have to be a peptide. For example, an organic group having 1 to 30 carbon atoms may be bonded to either or both of the two ends of the peptide structure (p). The compound (P) may be a compound represented by General Formula (p1-1') or (p2-1'). 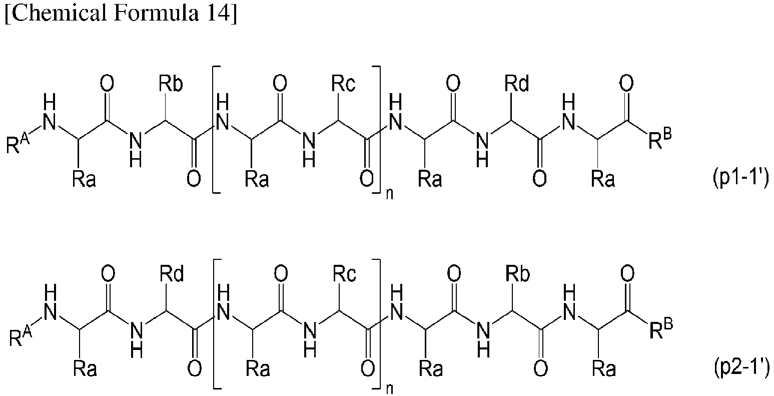 [in the formulae, R^{A} and R^{B} each independently represent a monovalent group, and Ra, Rb, Rc, Rd, and n are the same as Ra, Rb, Rc, Rd, and n in Formula (p1-1) or (p2-1) described above]

The monovalent groups in R^{A} and R^{B} in General Formula (p1-1) or (p2-1') are not particularly limited. Examples of the monovalent groups in R^{A} and R^{B} include an acetyl group, an amino group, a hydroxy group, a hydrogen atom, a halogen atom, a monovalent organic group, and the like. Examples of the monovalent organic group include a peptide consisting of 1 to 20 amino acid residues and monovalent organic groups other than peptides. Examples of the monovalent organic groups other than peptides include a hydrocarbon group which may have a substituent. Specific examples include alkyl groups, polyoxyethylene groups, polyoxypropylene groups, phenyl groups, and the like, without being limited thereto.

Specific examples of the compound (P) are shown below, without being limited thereto. In the following specific examples, the side chain (b1) is shown in the trans form, but the side chain (b1) may be in the cis form.

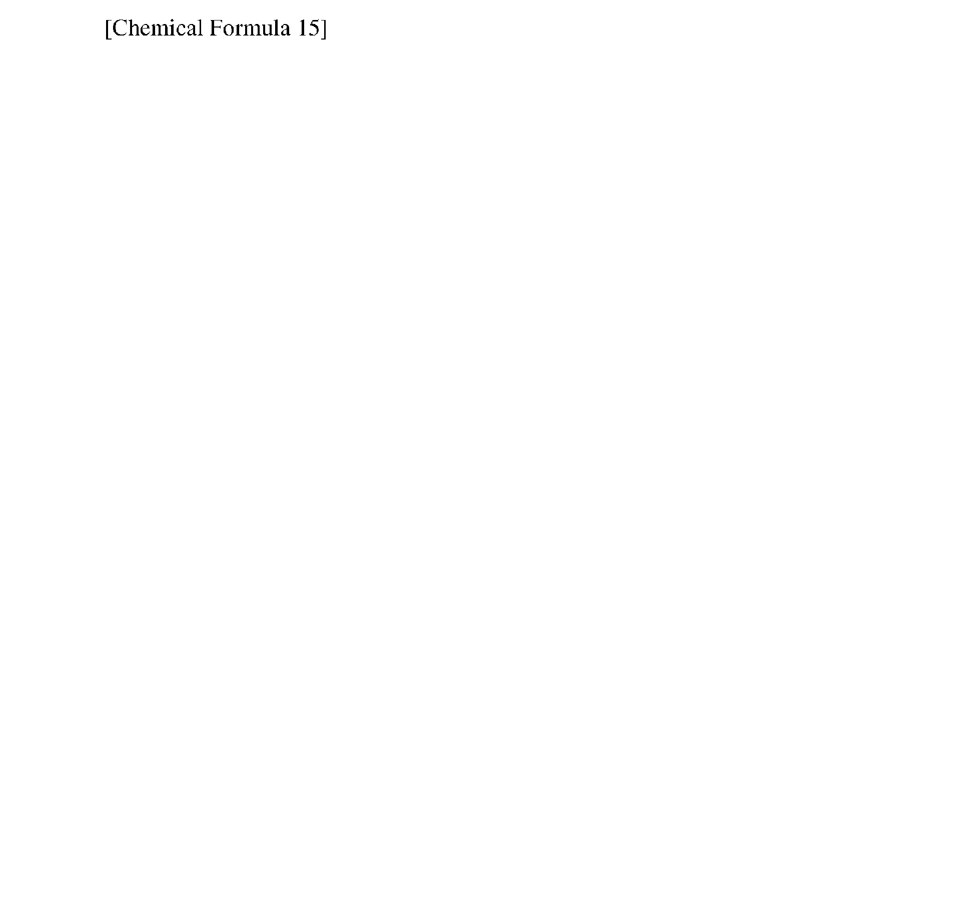

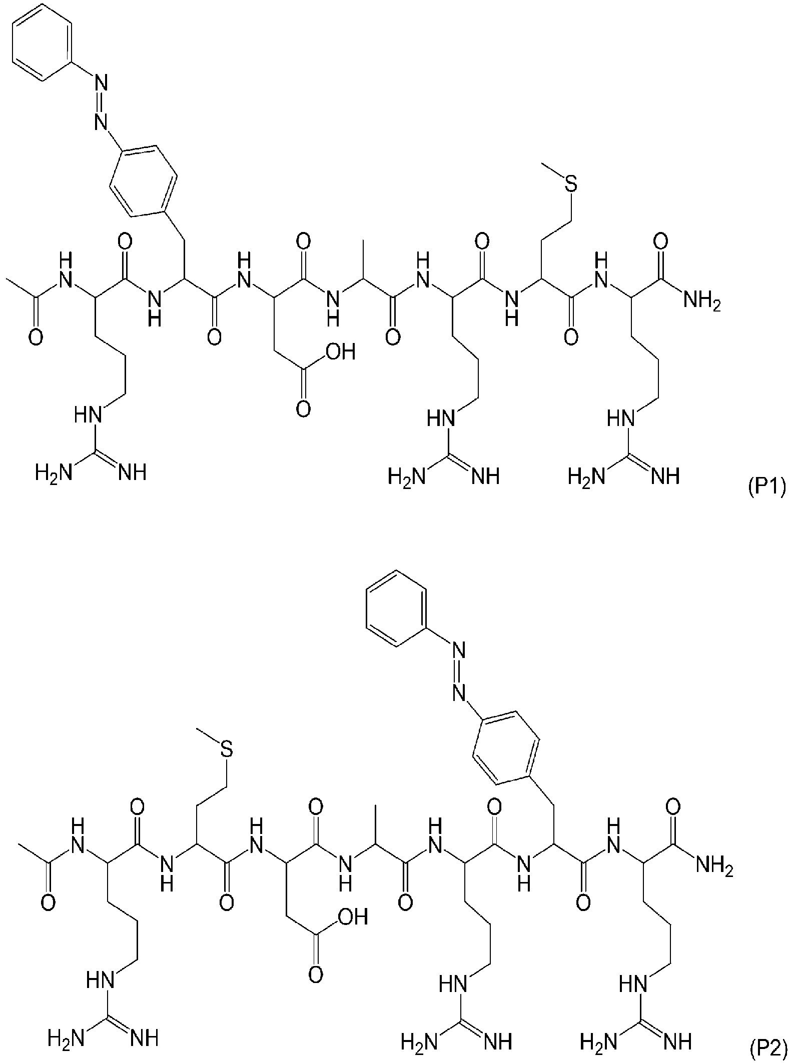

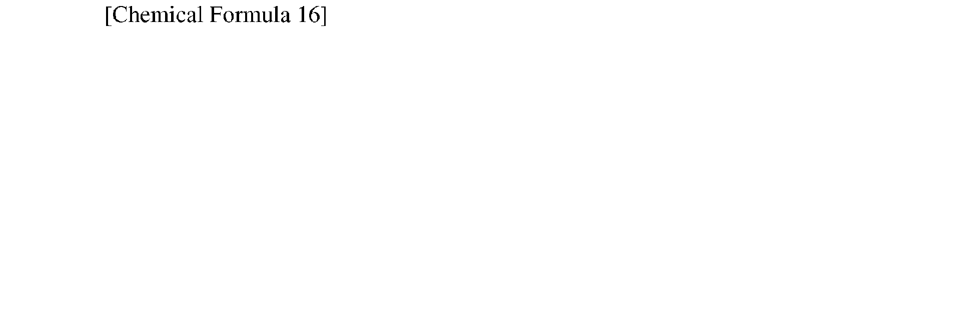

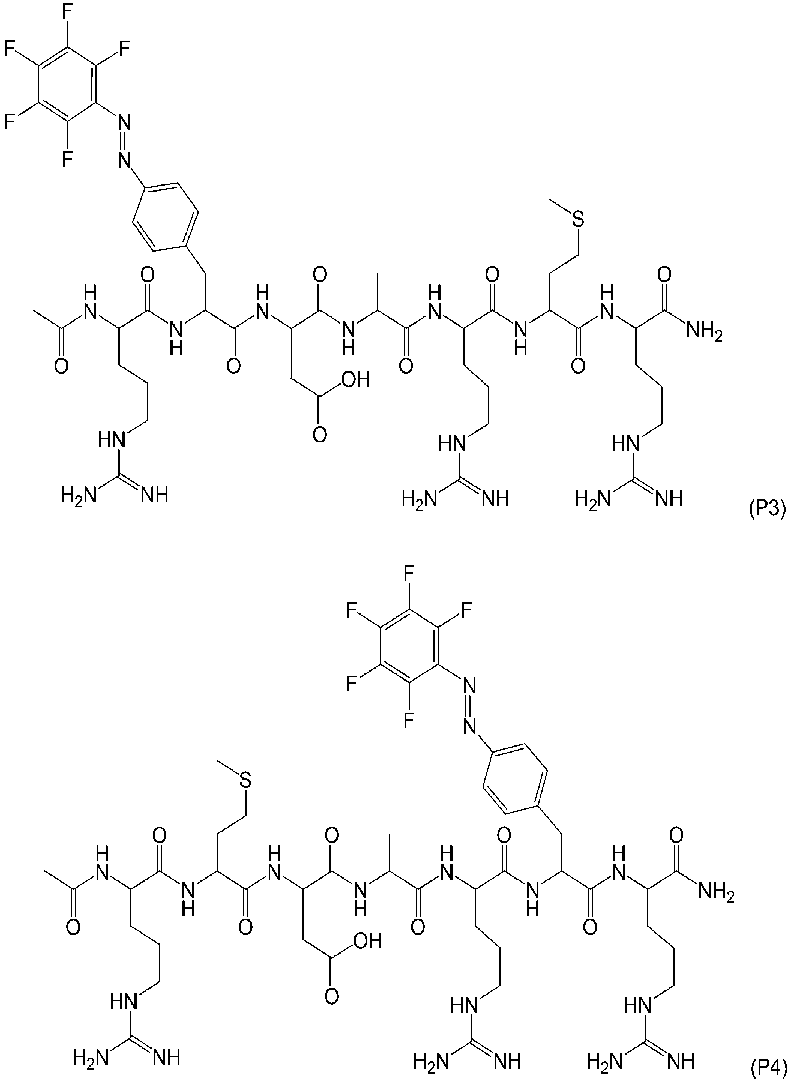

It is possible to produce the compound (P) by a known peptide synthesis method such as solid-phase peptide synthesis. It is possible to use commercially available amino acids as the raw material for the compound (P).

### Examples

Hereinafter, the present invention will be described with reference to Examples, but the present invention is not limited to Examples.

### [Synthesis of photoisomerizable compound (compound (P1))]

A compound (P1) represented by Formula (P1) described above was synthesized as a photoisomerizable compound by solid-phase peptide synthesis. The phenylazophenylalanine used in the peptide synthesis was purchased from Tokyo Chemical Industry Co., Ltd., and the other amino acids (arginine, aspartic acid, alanine, methionine) were purchased from Watanabe Chemical Industry Co., Ltd. The synthesized compound (P1) was analyzed using matrix-assisted laser desorption/ionization (MALDI). A peak (observed value: 1096.65) derived from the molecular weight (theoretical value: 1096.55) of a proton adduct of the compound (P1) was observed. This result confirmed that the compound (P1) was obtained.

The compound (P1) photoisomerized from a trans form to a cis form upon irradiation with 350-nm light, and photoisomerized from a cis form to a trans form upon irradiation with 450-nm light.

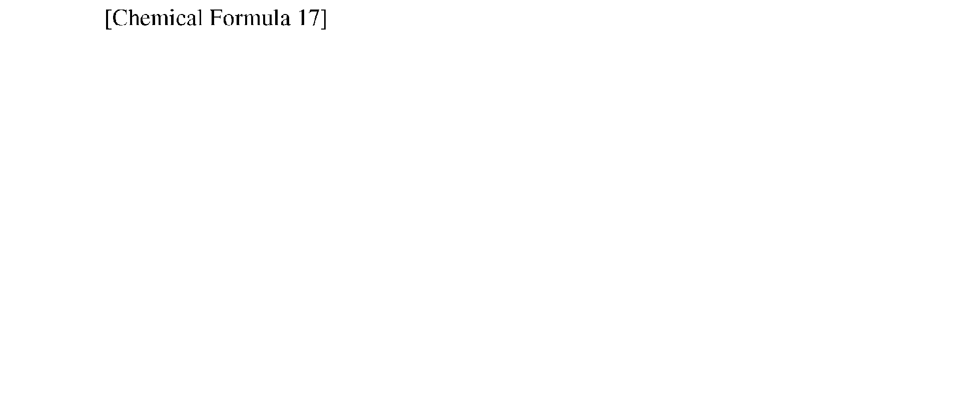

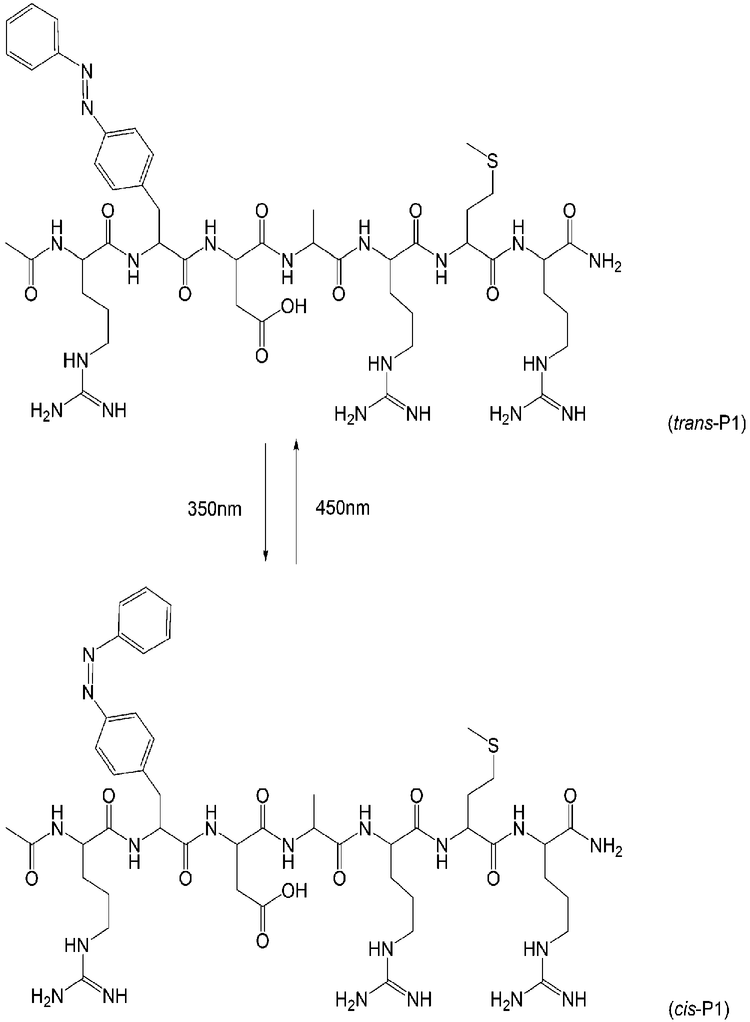

### [Example 1]

### <Preparation of droplets>

The following samples A and B were prepared.

### Sample A:

Polyethylene glycol (35 kDa; PEG35k, 5 wt%), dextran (550 kDa; DEX550k, 5 wt%), the compound (P1) (1 wt%), Escherichia coli, fluorescein-di-β-D-galactopyranoside (FDG, 60 µM), and isopropyl-β-D-thiogalactopyranoside (IPTG, 170 µM) were dispersed in water.

### Sample B:

Polyethylene glycol (35 kDa; PEG35k, 5 wt%), dextran (550 kDa; DEX550k, 5 wt%), the compound (P1) (1 wt%), and M13 phage virus were dispersed in water.

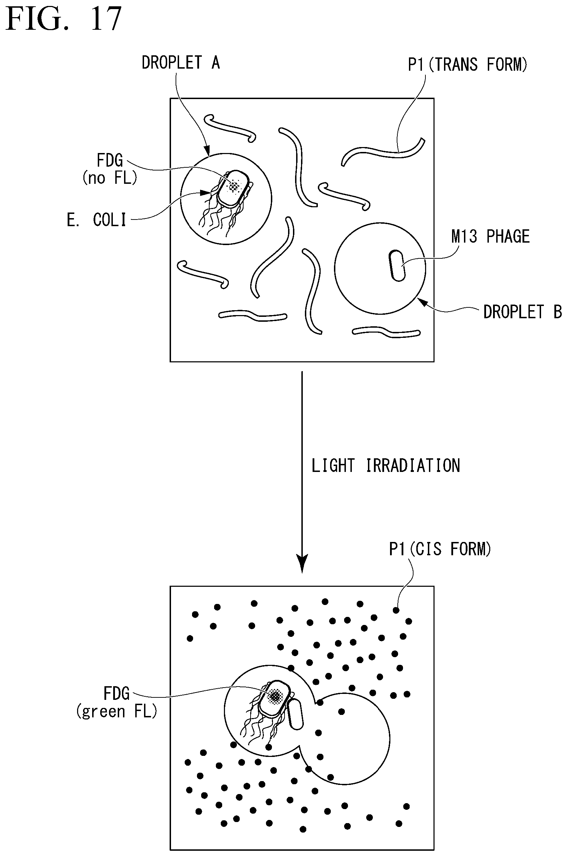

The sample A was stirred by vortexing for 30 seconds to produce droplets (droplets A). It was considered that Escherichia coli, FDG, and IPTG were encapsulated in the droplets A (see FIG. 17).

The sample B was stirred by vortexing for 30 seconds to produce droplets (droplets B). It was considered that M13 phage virus was encapsulated in the droplets B (see FIG. 17).

Thereafter, 2 µL of the dispersion liquid of the droplets A and 2 µL of the dispersion liquid of the droplets B were collected, placed in one microtube, and mixed by pipetting. As a result, a droplet dispersion liquid containing the droplets A and the droplets B was produced (see FIG. 17).

### <Light Irradiation>

4 µL of the droplet dispersion liquid prepared as described above was collected and dropped onto a plate to produce a droplet dispersion liquid plate. Two droplet dispersion liquid plates were produced. One plate was irradiated with light of 360 to 370 nm (UNA) for 5 minutes. The other plate was not irradiated with light.

Thereafter, the two plates were incubated for 1 day under conditions of 37°C and 5% CO₂.

### <Observation of phage infection to Escherichia coli>

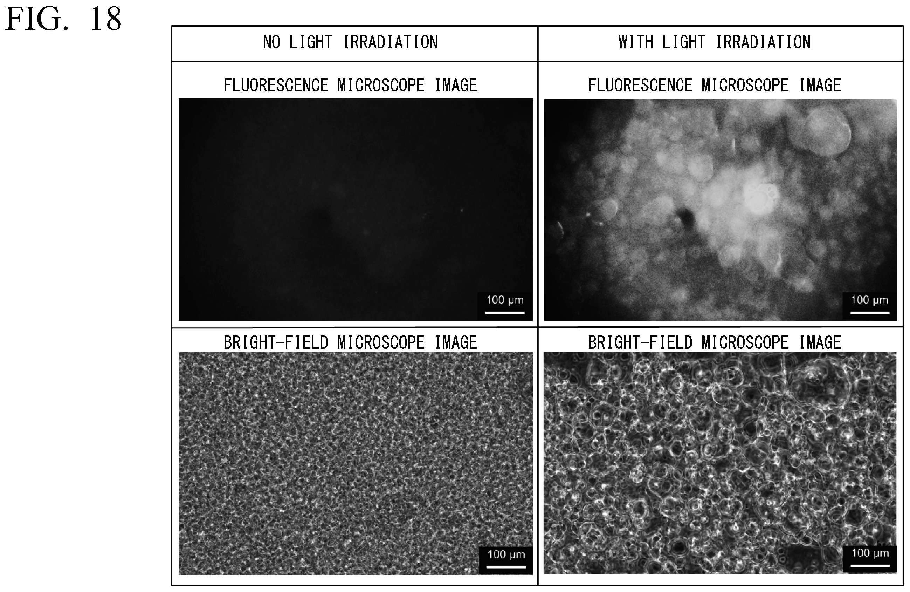

After the incubation, the two plates were observed with a fluorescence microscope (Olympus IX-73). The results are shown in FIG. 18.

In the plate not irradiated with light, little green fluorescence was observed. This was considered to be because infection of Escherichia coli with M13 phage did not occur, and FDG was not degraded by β-galactosidase possessed by M13 phage. It was considered that the slightly observed signal was derived from scattering.

In contrast, strong green fluorescence was observed in the plate irradiated with light. This was considered to be because the droplets A and the droplets B were fused by light irradiation, and thus infection of Escherichia coli with M13 phage occurred. That is, it was considered that β-D-galactopyranoside of FDG was degraded by β-galactosidase possessed by M13 phage to emit fluorescence (see FIG. 17).

The compound (P1) isomerized from a trans form to a cis form upon light irradiation of 360 to 370 nm. The trans form of the compound (P1) was fibrous, and the droplet dispersion liquid was in a gel state. Therefore, in the presence of the trans form compound (P1), the fusion between droplets hardly occurred. On the other hand, since the fibrous structure of the cis form compound (P1) collapsed, the droplet dispersion liquid became a sol state. Therefore, in the presence of the cis form compound (P1), the fusion between droplets occurred.

It was considered that the compound (P1) was isomerized from the trans form to the cis form upon light irradiation of 360 to 370 nm, whereby the fusion of the droplets A and the droplets B occurred.

### [Example 2]

### <Femtoliter chamber array device (FRAD)>

FRAD was produced by microfabrication using photolithography according to a paper by Ueno et al. (Ueno, H. et al., Protein Sci 2021, 30(8), 1628-1639.). A flow cell was assembled using FRAD, a top glass having holes for an inlet and an outlet, and a double-sided tape (to 80 µm) as a spacer. The top glass of the flow-cell chamber was pre-coated with CYTOP 809M to prevent non-specific binding of biomolecules.

The well volume of the produced FRAD is 6.3 fL.

### <Preparation of sample>

Each of the following solutions was prepared.

DEX aqueous solution: DEX550k was dissolved in water to have a concentration of 10 wt%.

PEG aqueous solution: PEG35k was dissolved in water to have a concentration of 5 wt%.

P1/PEG aqueous solution: PEG35k and the compound (P1) were dissolved in water so as to have concentrations of 5 wt% (PEG) and 1 wt% (compound (P1)).

### <Production of droplet>

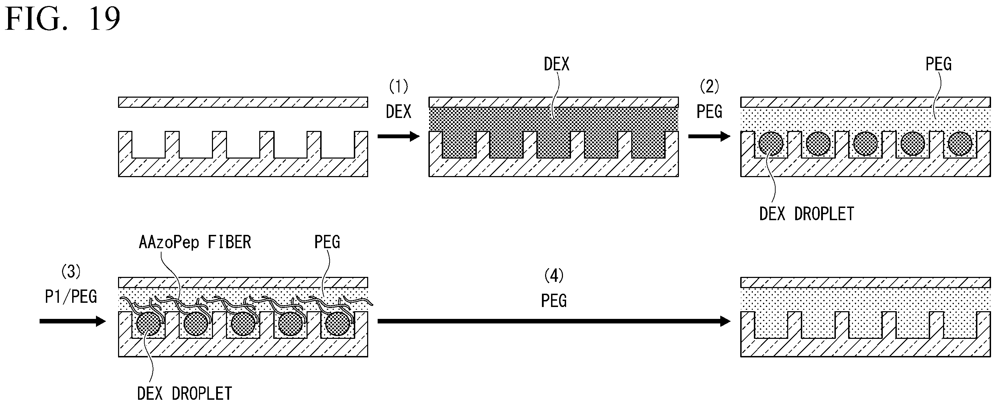

FIG. 19 shows an outline of a procedure in Example 2.

First, the DEX aqueous solution was fed to the flow path in the FRAD ((1) in FIG. 19). The feeding rate was 200 µL/min.

Next, the PEG aqueous solution was fed to the flow path in the FRAD ((2) in FIG. 19). The feeding rate was 200 µL/min. As a result, DEX droplets were formed in the wells of the FRAD.

Next, the P1/PEG aqueous solution was fed to the flow path in the FRAD ((3) in FIG. 19). The feeding rate was 200 µL/min.

### <Collection of droplet>

Next, the PEG aqueous solution was fed to the flow path in the FRAD without performing light irradiation ((4) in FIG. 19). The feeding rate was 200 µL/min.

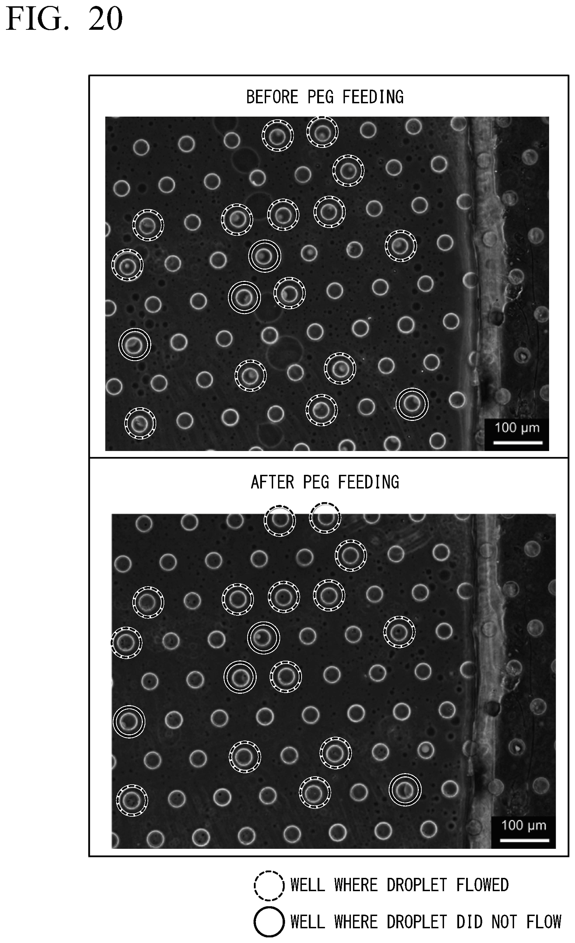

Images of the FRAD before and after the feeding of the PEG aqueous solution are shown in FIG. 20. It was confirmed that most of the droplets existing in the wells before the feeding of the PEG solution flowed out from the wells by feeding of the PEG aqueous solution.

From this result, it was confirmed that droplets could be collected from femtoliter-sized wells by feeding the PEG aqueous solution without irradiating light.

### [Example 3]

### <Preparation of sample>

DEX aqueous solution: DEX550k was dissolved in water to have a concentration of 10 wt%.

PEG aqueous solution: PEG35k was dissolved in water to have a concentration of 5 wt%.

P1/PEG aqueous solution: PEG35k and the compound (P1) were dissolved in water so as to have concentrations of 5 wt% (PEG) and 1 wt% (compound (P1)).

3T3 cell/PEG aqueous solution: 3T3 cells were suspended in an aqueous solution containing 5 wt% PEG35k so as to have a concentration of 1 x 10⁶ cells/mL.

### <Production of droplet>

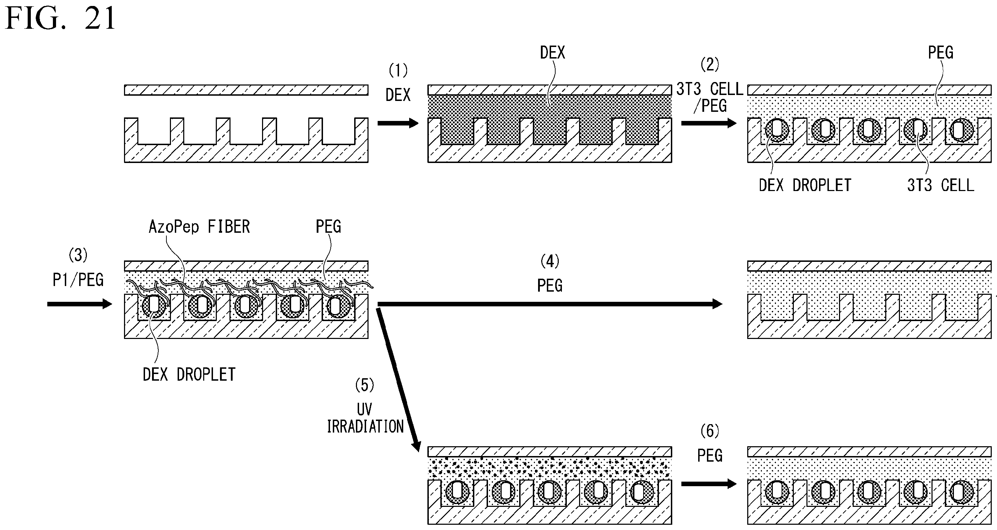

FIG. 21 shows an outline of a procedure in Example 3.

First, the DEX aqueous solution was fed to the flow path in the FRAD ((1) in FIG. 21). The feeding rate was 200 µL/min.

Next, the 3T3 cell/PEG aqueous solution was fed to the flow path in the FRAD ((2) in FIG. 21). The feeding rate was 200 µL/min. As a result, a DEX droplet in which the 3T3 cells were encapsulated was formed in the wells of the FRAD.

Next, the P1/PEG aqueous solution was fed to the flow path in the FRAD ((3) in FIG. 21). The feeding rate was 200 µL/min.

Two FRADs in which droplets were produced by the above-described procedure were provided.

### <Comparison of droplet collection rate depending on presence or absence of light irradiation>

For one FRAD, after (3) in FIG. 21, the PEG aqueous solution was fed to the flow path in the FRAD without performing light irradiation ((4) in FIG. 21). The feeding rate was 200 µL/min.

For the other FRAD, after (3) in FIG. 21, light irradiation (350 nm, 10 minutes) was performed ((5) in FIG. 21). Thereafter, the PEG aqueous solution was fed to the flow path in the FRAD (FIG. 21(6)). The feeding rate was 200 µL/min.

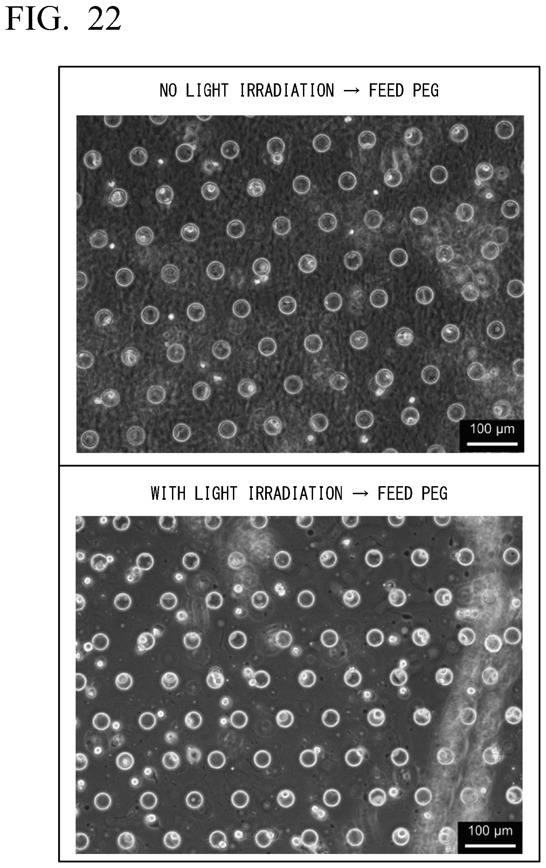

FIG. 22 shows images of the FRAD after feeding the PEG aqueous solution with "no light irradiation" or "no light irradiation".

When the PEG aqueous solution was fed with "no light irradiation", 27 cells remained in the FRAD. On the other hand, when the PEG aqueous solution was fed with "with light irradiation", 46 cells remained in the FRAD.

These results confirmed that irradiation with light at a wavelength that photoisomerized the compound (P1) increased a retention rate of the droplets containing 3T3 cells.

### INDUSTRIAL APPLICABILITY

According to the present invention, there are provided a droplet control device and a droplet production method, capable of controlling liquid-liquid phase-separated droplets. In addition, there is provided a composition capable of controlling initiation of a reaction of a plurality of components.

An explanation was given above of preferable Examples of the present invention, but the present invention is not limited to these Examples. Additions, omissions, substitutions, and other modifications to the configuration are possible in a range not departing from the gist of the present invention. The present invention is not limited by the above-described explanation, and is limited only by the scope of the attached claims.

### REFERENCE SIGNS LIST

1, 100 Droplet control device
10, 110, 210 Container
10t, 110t, 210t Temperature controller
20, 120, 120 Monitoring unit
21 Microscope unit
22 Objective lens
23 Dichroic mirror
24 Imaging lens
25 Pupil projection lens
26 Control mirror
27 Light source
28 Image sensor
30, 130, 230 Light irradiation unit
40, 140, 240 Processor
41, 141, 241 Container controller
42, 142, 242 Light irradiation unit controller
43 Droplet production unit controller
44, 144, 244 Monitoring unit controller
45, 145, 245 Output unit controller
46, 146, 246 Input unit controller
47, 147, 247 Communication unit controller
50, 150, 250 Output unit
51, 151, 251 Input unit
52, 152, 252 Communication unit
53, 153, 253 Memory
61, 161 Polysaccharide storage section
62, 162 Polyether storage section
63, 163, 263 Photoisomerizable compound storage section
64, 164 First component storage section
65, 165 Second component storage section
66, 166, 266 Detection reagent storage section
67, 167, 267 Aqueous medium storage section
68, 168, 212, 268 Waste liquid storage section
70 Droplet production unit
71, 271 Stirring mechanism
160 Liquid feeding unit
170 Droplet collection unit
110a, 110a' Top substrate
110b, 110b' Bottom substrate
110c, 110c' Side wall
111, 111' Well
112, 112' Partition wall
115, 115' Flow path
116, 116' Supply port
117, 117' Discharge port
90, 190, 290 Conduit
211 Target substance collection unit
243 Sample preparation unit controller
261 Test composition storage section
270 Sample preparation unit

## Claims

1. A droplet control device for controlling droplets produced from a polysaccharide and a polyether, the droplet control device comprising:
a light irradiation unit configured to irradiate with light; and
a monitoring unit configured to monitor a state of the droplets,
wherein an aqueous medium containing the droplets further contains a photoisomerizable compound, and
the light irradiation unit is configured to irradiate the aqueous medium with light having a wavelength that causes the photoisomerizable compound to undergo photoisomerization.

2. The droplet control device according to Claim 1, further comprising: a processor configured to control an operation of the droplet control device.

3. The droplet control device according to Claim 1 or 2, further comprising: a container configured to store the aqueous medium.

4. A droplet control device according to Claim 2,
wherein the processor is configured to control an operation of the light irradiation unit, and
the operation of the light irradiation unit controlled by the processor is at least one selected from the group consisting of a timing of starting light irradiation, a light irradiation time, a wavelength of irradiation light, and a light irradiation position.

5. A droplet control device according to Claim 2,
wherein the processor is configured to control an operation of the monitoring unit, and
the operation of the monitoring unit controlled by the processor is at least one selected from the group consisting of a timing of starting monitoring, a monitoring time, and a monitoring position.

6. The droplet control device according to Claim 1 or 2, further comprising: an output unit configured to output a monitoring result obtained by the monitoring unit.

7. The droplet control device according to Claim 1 or 2, wherein the monitoring unit includes a microscope unit configured to magnify and observe the droplets.

8. The droplet control device according to Claim 1 or 2,
wherein the droplets include
a first droplet containing a first component, and
a second droplet containing a second component, and
the first component is a component that interacts with the second component.

9. The droplet control device according to Claim 8, further comprising:
a first component storage section configured to store the first component;
a second component storage section configured to store the second component;
a polysaccharide storage section configured to store a polysaccharide;
a polyether storage section configured to store a polyether; and
a photoisomerizable compound storage section configured to store the photoisomerizable compound.

10. The droplet control device according to Claim 9, further comprising: a droplet production unit,
wherein the droplet production unit is controlled to
mix aqueous solutions containing the first component supplied from the first component storage section, the polysaccharide supplied from the polysaccharide storage section, the polyether supplied from the polyether storage section, and the photoisomerizable compound supplied from the photoisomerizable compound storage section to produce the first droplet, and
mix aqueous solutions containing the second component supplied from the second component storage section, the polysaccharide supplied from the polysaccharide storage section, the polyether supplied from the polyether storage section, and the photoisomerizable compound supplied from the photoisomerizable compound storage section to produce the second droplet.

11. The droplet control device according to Claim 10, further comprising: a detection reagent storage section configured to supply a detection reagent,
wherein the detection reagent storage section is controlled to supply the detection reagent to the droplet production unit at at least one of a timing of producing the first droplet and a timing of producing the second droplet.

12. The droplet control device according to Claim 10, wherein the droplet production unit includes a stirring mechanism.

13. The droplet control device according to Claim 8, wherein the monitoring unit is controlled to monitor an interaction between the first component and the second component in a third droplet generated by fusing the first droplet and the second droplet.

14. The droplet control device according to Claim 8, wherein the monitoring unit is controlled to perform the monitoring with reference to a time point at which the light irradiation unit starts irradiation of the aqueous medium with light having a wavelength that causes the photoisomerizable compound to undergo photoisomerization.

15. The droplet control device according to Claim 8, wherein the first component is a cell, the second component is a virus, and the interaction is an infection of the cell by the virus.

16. The droplet control device according to Claim 3, wherein the container includes a plurality of wells and a flow path communicating with the plurality of wells.

17. The droplet control device according to Claim 16, wherein a volume of the well is 1 picoliter or less.

18. The droplet control device according to Claim 16, further comprising: a liquid feeding unit configured to feed a liquid to the plurality of wells through the flow path.

19. The droplet control device according to Claim 18, wherein the liquid feeding unit includes
a polysaccharide storage section configured to supply a polysaccharide,
a polyether storage section configured to supply a polyether, and
a photoisomerizable compound storage section configured to supply the photoisomerizable compound.

20. The droplet control device according to Claim 19, wherein the liquid feeding unit is controlled to perform, in the following order,
(i) feeding an aqueous solution containing the polysaccharide to the plurality of wells,
(ii) feeding an aqueous solution containing the polyether to the plurality of wells, and
(iii) feeding an aqueous solution containing the polyether and the photoisomerizable compound to the plurality of wells.

21. The droplet control device according to Claim 20, wherein the monitoring unit is controlled to start the monitoring after the (iii).

22. The droplet control device according to Claim 21, wherein the light irradiation unit is controlled to irradiate wells other than a specific well with light having a wavelength that causes the photoisomerizable compound to undergo photoisomerization, based on a monitoring result of the monitoring unit.

23. The droplet control device according to Claim 22, further comprising: a droplet collection unit configured to collect the droplets from the specific well.

24. The droplet control device according to Claim 1 or 2, wherein the photoisomerizable compound is a compound including a peptide structure represented by General Formula (p1) or (p2),
XaXb(XaXc)ₙXaXdXa (p1)
XaXd(XaXc)ₙXaXbXa (p2)
[in the formulae, Xa represents an amino acid residue having a hydrophilic side chain, Xb represents an amino acid residue having a side chain represented by General Formula (b1), Xc and Xd each independently represent an amino acid residue having a hydrophobic side chain, or a glycine residue, and n represents an integer of 1 to 3, provided that a plurality of Xa's may be the same or different from each other, and when n is 2 or 3, a plurality of Xc's may be the same or different from each other]
[Chemical Formula 1]
*-Ar²-N=N-Ar¹ (b1)
[in the formula, Ar¹ represents an aryl group which may have a substituent or a heteroaryl group which may have a substituent, Ar² represents an arylene group which may have a substituent or a heteroarylene group which may have a substituent, and * represents a bonding site].

25. A device for separating a target substance from a sample prepared in an aqueous medium containing a photoisomerizable compound, the device comprising:
a container having a plurality of compartments for storing the sample;
a light irradiation unit configured to irradiate with light to the sample stored in the compartments; and
a monitoring unit configured to monitor the sample stored in the compartments,
wherein the light irradiation unit is configured to irradiate the sample with light having a wavelength that causes the photoisomerizable compound to undergo photoisomerization, and
the sample containing the photoisomerizable compound undergoes a sol-gel transition due to the photoisomerization of the photoisomerizable compound.

26. A method for producing droplets using a droplet container including a plurality of wells and a flow path communicating with the plurality of wells, the method comprising
the following (a), (b), and (c1) in this order, or the following (b), (a), and (c2) in this order:
(a) a step of feeding an aqueous solution containing a polysaccharide to the plurality of wells through the flow path;
(b) a step of feeding an aqueous solution containing a polyether to the plurality of wells through the flow path;
(c1) a step of feeding an aqueous solution containing the polyether and a photoisomerizable compound to the plurality of wells through the flow path; and
(c2) a step of feeding an aqueous solution containing the polysaccharide and a photoisomerizable compound to the plurality of wells through the flow path.

27. The method according to Claim 26, further comprising, after the step (c1) or (c2):
(d) a step of irradiating wells other than a specific well with light having a wavelength that causes the photoisomerizable compound to undergo photoisomerization.

28. The method according to Claim 27, further comprising, after the step (d):
(e1) a step of feeding an aqueous solution containing a polyether to the plurality of wells through the flow path, and collecting droplets from the specific well; or
(e2) a step of feeding an aqueous solution containing a polysaccharide to the plurality of wells through the flow path, and collecting the droplet from the specific well.

29. A method for separating a target substance from a sample prepared in an aqueous medium containing a photoisomerizable compound, the method comprising:
(A) a step of distributing the sample prepared in the aqueous medium in a sol state, containing the photoisomerizable compound, into a plurality of compartments;
(B) a step of irradiating either (i) a compartment containing the target substance or (ii) a compartment not containing the target substance with light having a wavelength that causes the photoisomerizable compound to undergo photoisomerization, to gel the sample stored in the compartments irradiated with the light; and
(C) a step of taking out the sample from the compartments not irradiated with the light.

30. The method according to any one of Claims 26 to 29, wherein the photoisomerizable compound is a compound including a peptide structure represented by General Formula (p1) or (p2),
XaXb(XaXc)ₙXaXdXa (p1)
XaXd(XaXc)ₙXaXbXa (p2)
[in the formulae, Xa represents an amino acid residue having a hydrophilic side chain, Xb represents an amino acid residue having a side chain represented by General Formula (b1), Xc and Xd each independently represent an amino acid residue having a hydrophobic side chain, or a glycine residue, and n represents an integer of 1 to 3, provided that a plurality of Xa's may be the same or different from each other, and when n is 2 or 3, a plurality of Xc's may be the same or different from each other]
[Chemical Formula 2]
*-Ar²-N=N-Ar¹ (b1)
[in the formula, Ar¹ represents an aryl group which may have a substituent or a heteroaryl group which may have a substituent, Ar² represents an arylene group which may have a substituent or a heteroarylene group which may have a substituent, and * represents a bonding site].

31. A composition stored in a light-shielding container, the composition comprising:
a polysaccharide;
a polyether;
a photoisomerizable compound;
a first droplet in which a first component is encapsulated; and
a second droplet in which a second component is encapsulated.

32. The composition according to Claim 31, wherein the photoisomerizable compound is a compound including a peptide structure represented by General Formula (p1) or (p2),
XaXb(XaXc)ₙXaXdXa (p1)
XaXd(XaXc)ₙXaXbXa (p2)
[in the formulae, Xa represents an amino acid residue having a hydrophilic side chain, Xb represents an amino acid residue having a side chain represented by General Formula (b1), Xc and Xd each independently represent an amino acid residue having a hydrophobic side chain, or a glycine residue, and n represents an integer of 1 to 3, provided that a plurality of Xa's may be the same or different from each other, and when n is 2 or 3, a plurality of Xc's may be the same or different from each other]
[Chemical Formula 3]
*-Ar²-N=N-Ar¹ (b1)
[in the formula, Ar¹ represents an aryl group which may have a substituent or a heteroaryl group which may have a substituent, Ar² represents an arylene group which may have a substituent or a heteroarylene group which may have a substituent, and * represents a bonding site].

33. A multi-component formulation comprising:
an openable and closable light-shielding container; and
the composition according to Claim 31 or Claim 32, which is stored in the light-shielding container.
